# EUROPEAN PATENT APPLICATION

(11) **EP 2 386 313 A1**
(43) Date of publication of application: **16.11.2011**
(21) Application number: 10183290.5
(22) Date of filing: 07.05.2004
(51) Int. Cl.: A61K 39/095, A61K 47/48, A61K 47/36

(54) **Multivalent meningococcal derivatized polysaccharide-protein conjugates and vaccine**

(30) Priority: 07.05.2003 US 468581 P
(62) Divisional of application: 04751728.9
(71) Applicant: Sanofi Pasteur, Inc., Swiftwater, PA 18370 (US)
(72) Inventor: Ryall, Robert P., Swiftwater, PA 18370 (US)
(74) Representative: Sahans, Sarah Anne

(57) **Abstract**

The present invention describes derivatized polysaccharide-protein conjugates, a composition comprising one or more of such derivatized polysaccharide-protein conjugates and methods of immunizing human patients with the same. The derivatized polysaccharide-protein conjugates are purified capsular polysaccharides from *Neisseria meningitidis* serogroups A, C, W-135, and Y, derivatized, chemically activated and selectively attached to a carrier protein by means of a covalent chemical bond, forming polysaccharide-protein conjugates capable of eliciting long-lasting immunity to a variety *of N. meningitidis* strains

## Description

The present application claims priority to U.S. provisional application No.: 60/468,581 filed on May 7, 2003, the entire disclosure of which is herein incorporated by reference in its entirety.

### BACKGROUND OF THE INVENTION

### 1. Field of the Invention

The present invention relates to the field of medicine generally, and more specifically to microbiology, immunology, vaccines and the prevention of infection by a bacterial pathogen by immunization.

### 2. Summary of the Related Art

*Neisseria meningitidis* is a leading cause of bacterial meningitis and sepsis throughout the world. The incidence of endemic meningococcal disease during the last thirty years ranges from 1 to 5 per 100,000 in the developed world, and from 10 to 25 per 100,000 in developing countries (Reido, F. X., et al., (1995) Ped.Infect. Dis.J.14, pp.643-6S7). During epidemics the incidence of meningococcal disease approaches 1000 per 1000,000. There are approximately 2,600 cases of bacterial meningitis per year in the United States, and on average 330,000 cases in developing countries. The case fatality rate ranges between 10 and 20%.

Pathogenic meningococci are enveloped by a polysaccharide capsule that is attached to the outer membrane surface of the organism. Thirteen different serogroups of meningococci have been identified on the basis of the immunological specificity of the capsular polysaccharide (Frasch, C. E., et al., (1985) Rev. Infect. Dis. 7, pp. 504-510). Of these thirteen serogroups, five cause the majority of meningococcal disease; these include serogroups A, B, C, W135, and Y. Serogroup A is responsible for most epidemic disease. Serogroups B, C, and Y cause the majority of endemic disease and localized outbreaks. The human naso-orapharyngeal mucosa is the only known natural reservoir of *Neisseria meningitidis.* Colonization takes place both at the exterior surface of the mucosal cell and the subepithelial tissue of the nasopharynx. Carriage of meningococci can last for months. Spreading of meningococci occurs by direct contact or via air droplets. Meningococci become invasive by passing through the mucosal epithelium via phagocytic vacuoles as a result of endocytosis. Host defense of invasive meningococci is dependent upon complement-mediated bacteriolysis. The serum antibodies that are responsible for complement-mediated bacteriolysis are directed in large part against the outer capsular polysaccharide.

Vaccines based on meningococcal polysaccharide have been described which elicit an immune response against the capsular polysaccharide. These antibodies are capable of complement-mediated bacteriolysis of the serogroup specific meningococci. The meningococcal polysaccharide vaccines are shown to be efficacious in children and adults (Peltola, H., et al., (1997) New Engl. J. Med 297, pp. 686-691 and Artenstein, M. S., et al., (1970) New Engl.J. Med. 282, pp. 417-420), but the efficacy is limited in infants and young children (Reingold, A. L., et al., (1985) Lancet 2, pp. 114-118). Subsequent doses of the polysaccharide in younger populations elicited a weak or no booster response (Goldschneider, I., et al., (1973) J. Infect. Diseases 128, pp. 769-776 and Gold, R., et al., (1977) J. Infect. Diseases. 136, S31-S35). The duration of protection elicited by the meningococcal polysaccharide vaccines is not long lasting, and has been estimated to be between 3 to 5 years in adults and children above four years of age (Brandt, B. L. and Artenstein, M. S. (1975) J. Infect. Diseases. 131, pp. S69-572, Kyhty, H., et al., (1980) J. Infect. Diseases. 142, pp. 861-868, and Cessey, S. J., et al., (1993) J. Infect. Diseases. 167, pp 1212-1215). For children from one to four years old the duration of protection is less than three years (Reingold, A. L., et al., (1985) Lancet 2, pp. 114-118).

Polysaccharides are incapable of binding to the major histocompatibility complex molecules, a prerequisite for antigen presentation to and stimulation of T-helper lymphocytes, i.e., they are T-cell independent antigens. Polysaccharides are able to-stimulate B lymphocytes for antibody production without the help of T-helper lymphocytes. As a result of the T-independent stimulation of the B lymphocytes, there is a lack of memory induction following immunization by these antigens. The polysaccharide antigens are capable of eliciting very effective T -independent responses in adults, but these T-independent responses are weak in the immature immune system of infants and young children.

T-independent polysaccharide antigens can be converted to T-dependent antigens by covalent attachment of the polysaccharides to protein molecules ("carriers" or "carrier proteins"). B cells that bind the polysaccharide component of the conjugate vaccine can be activated by helper T cells specific for peptides that are a part of the conjugated carrier protein. The T-helper response to the carrier protein serves to augment the antibody production to the polysaccharide.

The serogroup B polysaccharide has been shown to be poorly to non-immunogenic in the human population (Wyle, F. A., et al., (1972) J. Infect. Diseases. 126, pp. 514-522). Chemical attachment of this serogroup polysaccharide to proteins has not significantly altered the immune response in laboratory animals (Jennings, H. J. and Lugowski, C. (1981) J. Immunol. 127, pp. 1011-1018). The reason for the lack of immune response to this serogroup polysaccharide is thought to arise from structural similarities between the serogroup B polysaccharide and polysialylated host glycoproteins, such as the neural cell adhesion molecules.

A meningococcal conjugate vaccine based on serogroup C polysaccharide has been described. This monovalent vaccine elicits a strong functional antibody response to the capsular polysaccharide present on strains *of N. meningitidis* corresponding to serogroup C. Such a vaccine is only capable of protecting against disease caused by serogroup C bacteria.

Existing vaccines based on meningococcal polysaccharide are of limited use in young children and do not provide long-lasting protection in adults. The only meningococcal vaccine which as been shown to be capable of eliciting long-lasting protection in all groups, including children, at risk for meningococcal infection is based on a polysaccharide from a single serogroup *of N. meningitidis* and provides no protection against infection by other serogroups. Thus, a need exists for a meningococcal conjugate vaccine capable of conferring broad, long-lived protection against meningococcal disease in children and adults at risk for Meningococcal infection. The multivalent Meningococcal polysaccharides of the present invention solve this need by providing vaccine formulations in which immunogenic polysaccharides from the major pathogenic serogroups *of N. meningitidis* have been converted to T-dependent antigens through conjugations to carrier proteins.

FDA licensure of vaccines for meningococcal polysaccharides has been based on bactericidal assays with baby rabbit complement (SBA-BR) performed on blood samples of those immunized with the licensed vaccine. A number of government and expert panels have published current requirements and recommendations for assessing meningococcal polysaccharide vaccines on such assays, for example,

WHO Expert Committee on Biological Standardization for demonstrating the induction of bactericidal antibody production in healthy adult subjects immunized with meningococcal vaccines against *Neisseria meningitides* serogroups A and C (WHO 1976);

CDC SBA-BR in an international comparison study to establish the parameters for standardization of the assay uses the same standard reference serum, CDC donor R21654-3430107, that is one of the Quality Control serum samples in the comparison study (Maslanka SE, et al., 1997. Clin. Diagn. Lab.Immunol. 4: 156-167); and the standardized CDC method, recommended by the WHO Expert Committee of the Department of Vaccines and Biologicals as the optimal methodology (WHO 1999).

Licensure is granted because human immunity to meningococcal disease has been shown to correlate well with the level of complement-mediated bactericidal antibody detected by the Serum Bactericidal Assay (SBA) (Goldschneider, I, et al., 1969, J. Exp. Med.129:1307-1326 and Goldschneider, I, et al., 1969, 1. Exp. Med.129:1327-1348). A surrogate level of a 1:4 SBA titer against serogroup C has been established using a human complement in the assay (SBA-H). However, licensing requirements for meningococcal polysaccharide vaccines are based on the induction of serum bactericidal responses using baby rabbit complement (SBA-BR) as the source of complement in the assay (World Health Organization. 1976. Requirements for meningococcal polysaccharide vaccine. World Health Organization technical report series, no. 594. World Health Organization, Geneva, Switzerland (WHO 1976). According to this recommendation, the antibody titers of the sera from at least 90% of subjects vaccinated with meningococcal polysaccharide vaccine should show a 4-fold or greater rise 2-4 weeks after immunization when tested against the following target strains or equivalent strains: A1 for serogroup A, C11 for serogroup C, S-1975 for serogroup Y, and S-4383 for serogroup W-135 (WHO 1976, WHO 1981, Bureau of Biologics, Food and Drug Administration July 17,1985). The Bureau of Biologics adopted the WHO recommendation and the meningococcal polysaccharide vaccines, groups A and C combined and groups A, C, Y, and W-135 combined, are licensed in the United States based upon this requirement. In order to facilitate interlaboratory comparisons of the bactericidal activity induced by meningococcal vaccines, a standardized SBA using baby rabbit complement (SBA-BR) is established through a multilaboratory study (Maslanka SE, et al., 1997. Clin. Diagn. Lab. Immunol. 4: 156-167.

As data from meningococcal conjugate C vaccines started to become available, concerns began to emerge that the use of rabbit complement in the assay may lead to falsely high SBA titers. Following a March 1999 meeting to clarify and resolve issues relating to the laboratory assay for the analysis of human serum for meningococcal serogroups A and C specific antibodies, the WHO Expert Committee on Biological Standardization recommended that the SBA with baby rabbit complement be used for measuring antibody responses to serogroup C (The World Health Organization.1999. Standardization and validation of serological assays for the evaluation of immune responses to *Neisseria meningitidis* serogroup A/C vaccines. Geneva, WHO/V&B/99.19 (WHO 1999)). In an effort to avoid overestimating protection using baby rabbit complement, the WHO recommended that a study be undertaken to correlate the threshold titers measured by the SBA assay using baby rabbit complement relative to SBA titers measured using human complement. A follow-up meeting is held and results presented to support a general conclusion that a SBA titer of < 1:8 using baby rabbit complement correlates with an absence of protection against serogroup C and that an SBA titer of >=1:128 using baby rabbit complement correlates well to the protective SBA titer of 1:4 using human complement. No information is provided for corresponding correlate SBA-BR titers for other meningococcal serogroups, such as A, Y or W-135 or for polysaccharide conjugates.

SBA titers between 1:8 8 and 1:64 using baby rabbit complement do not necessarily correlate well with the protective SBA titer of 1:4 using human complement (Jodar L, et al., Biological 2002; 30: 323-329). The WHO Expert Committee recommended that post vaccination SBA-BR titers of 1:8,1:16,1:32 and 1:64 be reassessed using human complement. Other measures to resolve the uncertainties of the SBA-BR titers of 1:8,1:16, 1:32, and 1:64 included the assessment of four-fold rise in antibody SBA titers between pre-and post-vaccination. Demonstration of memory as a correlate of protection is also offered, however the Expert Committee recognized that the available data for these surrogates are either inadequate or limited.

An SBA-BR titer higher than 1:8 is a better indicia of human immunity to Meningococcal disease, as is a four-fold rise or higher, of SBA-BR titer from pre-immunization to post-immunization period of about 15 to about 45 days after immunization.

In one embodiment, the present invention provides a method of immunizing a human patient with a multivalent meningococcal polysaccharide conjugate composition, wherein the human patient has a serum SBA-BR titer of 1:16 or higher, preferably, of 1:32 or higher, and more preferably, 1:64 or higher, and even more preferably, 1: 128 or higher. In still further embodiments, the present invention provides a method of immunizing a human patient with a meningococcal polysaccharide conjugate composition, wherein the human patient has four-fold rise, or higher, in antibody SBA titers between pre- and post-vaccination.

In still another embodiment, the present invention provides a method of providing immunity to a human patient against multiple serogroups of N. meningococcal by immunizing the human patient with a multivalent meningococcal polysaccharide conjugate composition, wherein the composition comprises two or more polysaccharides selected from N. meningococcal serogroups A and W-135; Y and W-135; C and Y; C and W-135; A,C and Y; A,C and W-135; C,Y and W-135; A,Y and W-135; and A,C,Y and W-135.

In still further embodiments, the present invention provides a method of providing immunity to a human patient against multiple serogroups of N. meningococcal and by immunizing the human patient with a multivalent meningococcal (purified) polysaccharide conjugate composition, wherein the polysaccharide is derivatized to less than 100,000 daltons. In one embodiment of the invention, the purified polysaccharide is depolymerized to an average polysaccharide size of about 5,000 to about 75,000 daltons; preferably, to an average polysaccharide size of about 7,000 to about 50,000 daltons; more preferably, to an average polysaccharide size of about 8,000 to about 35,000 daltons; even more preferably, to an average polysaccharide size of about 12,000 to about 25,000 daltons. In one embodiment of the invention, the average polysaccharide size in the composition is about 15,000 to about 22,000 daltons.

### SUMMARY OF THE INVENTION

The present invention provides a method of providing human immunity to meningococcal disease caused by pathogenic *Neisseria meningitidis* by administration of immunological compositions of meningococcal polysaccharide-protein conjugates.

In one embodiment of the invention, the immunological composition comprises two or more protein-polysaccharide conjugates, wherein each of the conjugates comprises a capsular polysaccharide from *N. meningitidis* conjugated to a carrier protein. In a preferred embodiment, the immunological composition comprises two or more distinct protein-polysaccharide conjugates, wherein each of the conjugates comprises a, capsular polysaccharide from a different serogroup of *N. meningitidis* conjugated to a carrier protein.

The present invention provides a method of providing human immunity to meningococcal disease caused by pathogenic *Neisseria meningitidis* comprising administration of an immunological composition comprising two or more distinct protein-polysaccharide conjugates, wherein each of the conjugates comprises a capsular polysaccharide from a different serogroup of *N. meningitidis* conjugated to a carrier protein.

The present invention provides a method of providing human immunity to meningococcal disease caused by pathogenic *Neisseria meningitidis* comprising administration of meningococcal polysaccharide-protein conjugates. The present invention provides multivalent meningococcal vaccines comprised of immunologically effective amounts of from two to four distinct protein-polysaccharide conjugates, wherein each of the conjugates contains a different capsular polysaccharide conjugated to a carrier protein, and wherein each capsular polysaccharide is selected from the group consisting of capsular polysaccharide from serogroups A, C, W-135 and Y. The present invention further provides a method of inducing an immunological response to capsular polysaccharide of *N*. *meningitidis* comprising administering an immunologically effective amount of the immunological composition of the invention to a human. In one embodiment, the multivalent meningococcal vaccine comprises immunologically effective amounts of two distinct protein-polysaccharide conjugates, wherein each of the conjugates contains a different capsular polysaccharide conjugated to a carrier protein, and wherein each capsular polysaccharide is selected from the group consisting of capsular polysaccharide from serogroups A, C, W-135 and Y, more preferably, comprises capsular polysaccharides A and W-135, A and Y, C and W-135, C and Y, and W-135 and Y. In one embodiment, the multivalent meningococcal vaccine comprises immunologically effective amounts of three distinct protein-polysaccharide conjugates, wherein each of the conjugates contains a different capsular polysaccharide conjugated to a carrier protein, and wherein each capsular polysaccharide is selected from the group consisting of capsular polysaccharide from serogroups A, C, W-135 and Y, more preferably, comprises capsular polysaccharides A, C and W-135, A, C and Y, C, Y and W-135, C, W-135 and Y, and A, W-135 and Y. In another embodiment, the multivalent meningococcal vaccine comprises immunologically effective amounts of four distinct protein-polysaccharide conjugates, wherein each of the conjugates contains a different capsular polysaccharide conjugated to a carrier protein, and wherein each capsular polysaccharide is selected from the group consisting of capsular polysaccharide from serogroup A, C, W-135 and Y.

The present invention further provides a method of inducing an immunological response to capsular polysaccharide *of N. meningitidis* comprising administering an immunologically effective amount of the immunological composition of the invention to a human or animal.

The present invention provides a multivalent meningococcal vaccine comprised of immunologically effective amounts of from two to four distinct protein-polysaccharide conjugates, wherein each of the conjugates contains a different capsular polysaccharide conjugated to a carrier protein, and wherein each capsular polysaccharide is selected from the group consisting of capsular polysaccharide from serogroups A, C, W-135 and Y.

The present invention provides a method of protecting a human or animal susceptible to infection from *N. meningitidis* comprising administering an immunologically effective dose of the vaccine of the invention to the human or animal.

All patents, patent applications, and other publications recited herein are hereby incorporated by reference in their entirety.

### DETAILED DESCRIPTION OF THE INVENTION

The present invention comprises an immunological composition of two or more distinct protein-polysaccharide conjugates, wherein each of the conjugates comprises a capsular polysaccharide conjugated to a carrier protein. Thus, the present invention includes compositions that comprise two or more different derivatized capsular polysaccharides conjugated to one or more carrier protein(s).

Capsular polysaccharides can be prepared by standard techniques known to those of skill in the art. In the present invention capsular polysaccharides prepared from serogroups A, C, W-135 and Y of *N*. *meningitidis* are preferred.

In a preferred embodiment, these meningococcal serogroup conjugates are prepared by separate processes and formulated into a single dosage formulation. For example, capsular polysaccharides from serogroups A, C, W-135 and Y of *N*. *meningitidis* are separately purified.

In a preferred embodiment of the present invention the purified polysaccharide is depolymerized and activated prior to conjugation to a carrier protein. In a preferred embodiment of the present invention capsular polysaccharides of serogroups A, C, W-135, and Y from *N. meningitidis* are partially depolymerized using mild oxidative conditions.

Native meningococcal polysaccharide is about 500,000 to 1,500,000 daltons. The present invention is directed to meningococcal polysaccharides of a smaller size. When purifying native polysaccharides, a certain percentage of the polysaccharides will be of a smaller size. However, to obtain a better yield, it is generally preferred to depolymerize, or derivatize the native meningococcal polysaccharide to a preferred size range, preferably less than 100,000 daltons. In one embodiment of the invention, the purified polysaccharide is depolymerized to an average polysaccharide size of about 5,000 to about 75,000 daltons; preferably, to an average polysaccharide size of about 7,000 to about 50,000 daltons; more preferably, to an average polysaccharide size of about 8,000 to about 35,000 daltons; even more preferably; to an average polysaccharide size of about 12,000 to about 25,000 daltons. In one embodiment of the invention, the average polysaccharide size in the composition is about 15,000 to about 22,000 daltons.

The depolymerization or partial depolymerization of the polysaccharides may then be followed by an activation step. By "activation" is meant chemical treatment of the polysaccharide to provide chemical groups capable of reacting with the carrier protein. A preferred activation method involves treatment with adipic acid dihyrazide in physiological saline at pH 5.0.+-.0.1 for approximately two hours at 15 to 30 deg. C. One process for activation is described in U.S. Pat. No. 5,965,714.

Once activated, the capsular polysaccharides may then be conjugated to one or more carrier proteins. In a preferred embodiment of the present invention each capsular polysaccharide is separately conjugated to a single carrier protein species. In a preferred embodiment the capsular polysaccharides from serogroups A, C, W-135 and Y of *N. meningitidis* are each separately conjugated to the same carrier protein species.

Carrier proteins may include bacterial toxins such as diphtheria toxin, inactivated bacterial toxins such as diphtheria toxoid, CRM_{197,} tetanus toxoid, pertussis toxoid, E. coli LT, E. coli ST, and exotoxin A from Pseudomonas aeruginosa. Bacterial outer membrane proteins such as, outer membrane complex c (OMPC), porins, transferrin binding proteins, pneumolysis, pneumococcal surface protein A (PspA), or pneumococcal adhesin protein (PsaA), could also be used. Other proteins, such as ovalbumin, keyhole limpit hemocyanin (KLH), bovine serum albumin (BSA) or purified protein derivative of tuberculin (PPD) may also be used as carrier proteins. Carrier proteins are preferably proteins that are non-toxic and non-reactogenic and obtainable in sufficient amount and purity. Carrier proteins should be amenable to standard conjugation procedures. In a preferred embodiment of the present invention diphtheria toxin purified from cultures of *Corynebacteria diphtheriae* and chemically detoxified using formaldehyde is used as the carrier protein. An alternative carrier protein is Protein D which is an outer membrane surface exposed protein of H. influenza.

In one embodiment of the invention, the average ratio of each derivatized polysaccharide to carrier protein is about 1:1 to about 1:20 (w/w). In a preferred embodiment of the invention, the average ratio of total derivatized polysaccharide to carrier protein is about 1:2 to about 1:10 (w/w), and an even more preferred average ratio of each derivatized polysaccharide to carrier protein is about 1:2 to about 1:6 (w/w). In a more preferred embodiment of the invention, the average ratio of total derivatized polysaccharide to carrier protein is about 1 :(4±1); more preferably, 1:(4±0.5), even more preferably, 1:(4+-0.25) (w/w).

After conjugation of the capsular polysaccharide to the carrier protein, the polysaccharide-protein conjugates may be purified (enriched with respect to the amount of polysaccharide-protein conjugate) by a variety of techniques. One goal of the purification step is to remove the unbound polysaccharide from the polysaccharide-protein conjugate. One method for purification, involving ultrafiltration in the presence of ammonium sulfate, is described in U.S. Pat. No. 6,146,902. Alternatively, conjugates can be purified away from unreacted protein and polysaccharide by any number of standard techniques including, inter alia, size exclusion chromatography, density gradient centrifugation, hydrophobic interaction chromatography or ammonium sulfate fractionation. See, e.g., P. W. Anderson, et. al. (1986). J. Immunol. 137: 1181-1186. See also H. J. Jennings and C. Lugowski (1981) J. Immunol. 127: 1011-1018.

After conjugation of the polysaccharide and carrier protein the immunological compositions of the present invention are made by combining the various derivatized polysaccharide-protein conjugates. The immunological compositions of the present invention comprise two or more different capsular polysaccharides conjugated to one or more carrier protein(s). A preferred embodiment of the present invention is a bivalent immunological composition comprising derivatized capsular polysaccharides from serogroups A and C of *N*. *meningitidis* separately conjugated to diptheria toxin or toxoid. More preferably the present invention is a tetravalent immunological composition comprising capsular polysaccharides from serogroups A, C, W-135 and Y *of N. meningitidis* separately conjugated to diptheria toxin or toxoid.

The present invention is directed, in part, to a composition of multicomponent, derivatized polysaccharide conjugates where each derivatized polysaccharide is present in about 0.5 to about 15 µg per dose. Thus, the composition may comprise a total derivatized polysaccharide µg of 1 µg to 60 µg. In a preferred embodiment, the relative amount of each derivatized polysaccharide in the composition is about equal within + 50%; more preferably, within ± 30%; even more preferably, within ± 20%.

Preparation and use of carrier proteins, and a variety of potential conjugation procedures, are well known to those skilled in the art. Conjugates of the present invention can be prepared by such skilled persons using the teachings contained in the present invention as well as information readily available in the general literature. Guidance can also be obtained from any one or all of the following U.S. patents, the teachings of which are hereby incorporated in their entirety by reference: U.S. Pat. Nos. 4,356,170; 4,619,828; 5,153,312; 5,422,427 and 5,445,817.

Alternatively, the immunological compositions may be made by either culturing two or *more N. meningitidis* serogroups together and copurifying, depolymerizing, activating and conjugating the polysaccharides, or by culturing purifying the *N. meningitidis* serogroups separately and combining two or more purified polysaccharides before or after any of the steps of depolymerizing, activating and conjugating the polysaccharides.

The immunological compositions of the present invention are made by separately preparing polysaccharide-protein conjugates from different meningococcal serogroups and then combining the conjugates. The immunological compositions of the present invention can be used as vaccines. Formulation of the vaccines of the present invention can be accomplished using art recognized methods. The vaccine compositions of the present invention may also contain one or more adjuvants. Adjuvants include, by way of example and not limitation, aluminum adjuvants (e.g., aluminum salts such as aluminum hydroxide, aluminum phosphate, aluminium sulfate or combinations thereof, Freund's Adjuvant (Complete or Incomplete), BAY, DC-chol, pcpp, monophoshoryl lipid A, CpG, QS-21, cholera toxin and formyl methionyl peptide. See, e.g., Vaccine Design, the Subunit and Adjuvant Approach, 1995 (M. F. Powell and M. J. Newman, eds., Plenum-Press, N.Y.). The adjuvant is preferably an aluminum adjuvant, such as aluminum hydroxide or aluminum phosphate.

Alternative adjuvants include oil-in-water emulsion formulations for example MF59 as described in PCT Publ. No. WO 90/14837), SAF, containing 10% Squalane, 0.4% Tween 80,5% pluronic-blocked polymer L121, and thr-MDP, RibiTM adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL+CWS (DetoxTM); saponin adjuvants, such as StimulonTM (Cambridge Bioscience, Worcester, Mass.) may be used or particles generated there from such as ISCOMs (immunostimulating complexes); cytokines, such as interleukins (e.g., IL-1, IL-2, IL-4, IL-5, IL-6, IL-7, IL-12, etc.), interferons (e.g., gamma interferon), macrophage colony stimulating factor (M-CSF), tumor necrosis factor (TNF).

In one embodiment of the invention, the protein-polysaccharide conjugates have an average glycosylation ratio (polysaccharide to protein ratio) of about 0.05 to about 2; more preferably, an average ratio of about 0.08 to about 1.25; and even more preferably, an average ratio of about 0.1 to about 0.9. In one preferred embodiment, the protein-polysaccharide conjugates have an average glycosylation ratio polysaccharide to protein ratio of about 0.2 to about 0.8; more preferably, an average ratio of about 0.2 to about 0.6, and even more preferred embodiment, an average ratio of about 0.3 to about 0.5.

As demonstrated below, the vaccines and immunological compositions according to the invention elicit a T-dependent-like immune response in various animal models, whereas the polysaccharide vaccine elicits a T-independent-like immune response. Thus, the compositions of the invention are also useful research tools for studying the biological pathways and processes involved in T-dependent-like immune responses to *N. meningitidis* antigens.

The amount of vaccine of the invention to be administered a human or animal and the regime of administration can be determined in accordance with standard techniques well known to those of ordinary skill in the pharmaceutical and veterinary arts taking into consideration such factors as the particular antigen, the adjuvant (if present), the age, sex, weight, species and condition of the particular animal or patient, and the route of administration. In the present invention, the amount of polysaccharide-protein carrier to provide an efficacious dose for vaccination against *N. meningitidis* can be from between about 0.02 µg to about 5 µg per kg body weight. In a preferred composition and method of the present invention the dosage is between about 0.1 µg to 3 µg per kg of body weight. For example, an efficacious dosage will require less antibody if the post-infection time elapsed is less since there is less time for the bacteria to proliferate. In like manner an efficacious dosage will depend on the bacterial load at the time of diagnosis. Multiple injections administered over a period of days could be considered for therapeutic usage.

The multivalent conjugates of the present invention can be administered as a single dose or in a series (i.e., with a "booster" or "boosters"). For example, a child could receive a single dose early in life, then be administered a booster dose up to ten years later, as is currently recommended for other vaccines to prevent childhood diseases.

The booster dose will generate antibodies from primed B-cells, i.e., an anamnestic response. That is, the multivalent conjugate vaccine elicits a high primary (i.e., following a single administration of vaccine) functional antibody response in younger populations when compared to the licensed polysaccharide vaccine, and is capable of eliciting an anamnestic response (i.e., following a booster administration), demonstrating that the protective immune response elicited by the multivalent conjugate vaccine of the present invention is long-lived.

Compositions of the invention can include liquid preparations for orifice, e.g., oral, nasal, anal, vaginal, peroral, intragastric; mucosal (e.g., perlinqual, alveolar, gingival, olfactory or respiratory mucosa) etc., administration such as suspensions, syrups or elixirs; and, preparations for parenteral, subcutaneous, intradermal, intramuscular, intraperitoneal or intravenous administration (e.g., injectable administration), such as sterile suspensions or emulsions. Intravenous and parenteral administration are preferred. Such compositions may be in admixture with a suitable carrier, diluent, or excipient such as sterile water, physiological saline, glucose or the like. The compositions can also be lyophilized. The compositions can contain auxiliary substances such as wetting or emulsifying agents, pH buffering agents, gelling or viscosity enhancing additives, preservatives, flavoring agents, colors, and the like, depending upon the route of administration and the preparation desired. Standard texts, such as "REMINGTON'S PHARMACEUTICAL SCIENCE", 17.sup.th edition, 1985, incorporated herein by reference, may be consulted to prepare suitable preparations, without undue experimentation.

In one embodiment of the invention, a preferred route of administration is intramuscular or subcutaneous, with intramuscular route preferred. Administration may be by injection or by an alternative delivery device.

Compositions of the invention are conveniently provided as liquid preparations, e.g., isotonic aqueous solutions, suspensions, emulsions or viscous compositions that may be buffered to a selected pH. If digestive tract absorption is preferred, compositions of the invention can be in the "solid" form of pills, tablets, capsules, caplets and the like, including "solid" preparations which are time-released or which have a liquid filling, e.g., gelatin covered liquid; whereby the gelatin is dissolved in the stomach for delivery to the gut. If nasal or respiratory (mucosal) administration is desired, compositions may be in a form and dispensed by a squeeze spray dispenser, pump dispenser or aerosol dispenser. Aerosols are usually under pressure by means of a hydrocarbon. Pump dispensers can preferably dispense a metered dose or a dose having a particular particle size.

Liquid preparations are normally easier to prepare than gels, other viscous compositions, and solid compositions. Additionally, liquid compositions are somewhat more convenient to administer, especially by injection or orally, to animals, children, particularly small children, and others who may have difficulty swallowing a pill, tablet, capsule or the like, or in multi-dose situations. Viscous compositions, on the other hand, can be formulated within the appropriate viscosity range to provide longer contact periods with mucosa, such as the lining of the stomach or nasal mucosa.

In a preferred embodiment of the invention, the vaccine composition is formulated as a sterile liquid, pyrogen-free, phosphate-buffered physiological saline, with or without a preservative. In one preferred embodiment, the formula per dose, comprises about 0.3 to about 1.0 mg sodium phosphate and about 3.5 to about 6.0 mg sodium chloride and up to 1.5 mL water. In one preferred embodiment, the formula per dose, comprises about 0.6 ± 0.2 mg sodium phosphate and 4.4 ± 0.2 mg sodium chloride and up to about 0.5± 0.2 mL water.

Obviously, the choice of suitable carriers and other additives will depend on the exact route of administration and the nature of the particular dosage form, e.g., liquid dosage for (e.g., whether the composition is to be formulated into a solution, a suspension, gel or another liquid form), or solid dosage form (e.g., whether the composition is to be formulated into a pill, tablet, capsule, caplet, time release form or liquid-filled form).

Solutions, suspensions and gels, normally contain a major amount of water (preferably purified water) in addition to the active ingredient. Minor amounts of other ingredients such as pH adjusters (e.g., a base such as NaOH), emulsifiers or dispersing agents, buffering agents, preservatives, wetting agents, jelling agents, (e.g., methylcellulose), colors and/or flavors may also be present. The compositions can be isotonic, i.e., it can have the same osmotic pressure as blood and lacrimal fluid.

The desired isotonicity of the compositions of this invention may be accomplished using sodium tartrate, propylene glycol or other inorganic or organic solutes. In one embodiment, the preferred isotonicity of the composition is obtained from sodium phosphate or sodium chloride, or mixtures thereof. Sodium chloride is preferred particularly for buffers containing sodium ions.

Viscosity of the compositions may be maintained at the selected level using a pharmaceutically acceptable thickening agent. Methylcellulose is preferred because it is readily and economically available and is easy to work with. Other suitable thickening agents include, for example, xanthan gum, carboxymethyl cellulose, hydroxypropyl cellulose, carbomer, and the like. The preferred concentration of the thickener will depend upon the agent selected. The important point is to use an amount that will achieve the selected viscosity. Viscous compositions are normally prepared from solutions by the addition of such thickening agents.

A pharmaceutically acceptable preservative can be employed to increase the shelf life of the compositions. Benzyl alcohol may be suitable, although a variety of preservatives including, for example, parabens, thimerosal, chlorobutanol, or benzalkonium chloride may also be employed. A suitable concentration of the preservative will be from 0.02% to 2% based on the total weight although there may be appreciable variation depending upon the agent selected.

Those skilled in the art will recognize that the components of the compositions must be selected to be chemically inert with respect to the *N. meningitidis* polysaccharide-protein carrier conjugates.

The invention will be further described by reference to the following illustrative, nonlimiting examples setting forth in detail several preferred embodiments of the inventive concept. Other examples of this invention will be apparent to those skilled in the art without departing from the spirit of the invention.

The following abbreviations and Trademarks are: ACIP, Advisory Committee on Immunization Practices; AE, Adverse Event; Cetavalon™, cetyltrimethylammonium bromide, CTAB; CFR, Code of Federal Regulations; CRF, Case Report Form; DTP, Diphtheria Tetanus Pertussis; ELISA, Enzyme Linked Immunosorbent Assay; FDA, Food and Drug Administration; GCP, Good Clinical Practice; GMC, Geometric Mean Concentration; GMT, Geometric Mean Titer; IgG, Immunoglobulin G; IgGl, Immunoglobulin G subclass 1; IgG2, Immunoglobulin G subclass 2; IgM, Immunoglobulin M; ICH, International Conference on Harmonization; IND, Investigational New Drug; IRB, Institutional Review Board; MenA/C-Dt Bivalent (A and C) Meningococcal Polysaccharide Diphtheria Conjugate Vaccine; MenPS, Meningococcal group specific polysaccharide; mL milliliter; Menomune™, licensed Meningococcal A,C Y and W-135 polysaccharide vaccine; OD, Optical Density; PBS, Phosphate Buffered Saline; SAE, Serious Adverse Event; SBA, Serum bactericidal activity; SBA-BR, Serum bactericidal activity assay performed using baby rabbit complement; SBA-HC, Serum bactericidal activity assay performed using human complement; SIDS, Sudden Infant Death Syndrome; TetraMenD, Tetravalent (A, C, Y, and W-135) Meningococcal Polysaccharide Diphtheria Conjugate Vaccine; Td, Tetanus and Diphtheria vaccine; UAE, Unexpected Adverse Experience; URI, Upper Respiratory Infection; µg, Micrograms.

### EXAMPLES

### Example 1 Preparation of Neisseria meningitidis Serogroups A, C, W-135, and Y Purified Capsular Polysaccharides Powders

### Crude Paste Preparation

Separately, *Neisseria meningitidis* serogroup A, C, W-135, and Y wet frozen seed cultures are thawed and recovered with the aid of liquid Watson Scherp medium and planted in Blake bottles containing Mueller Hinton agar medium. The Blake are incubated at 35 to 37 deg. C. in a CO₂ atmosphere for 15 to 19 hours. Following the incubation period, the growth from the Blake bottles are dislodged and added to 4 L flasks containing Watson Scherp medium. The flasks are incubated at 35 to 37 deg. C. for 3 to 7 hours on a platform shaker. The contents of the 4 L flasks are transferred to a fermenter vessel containing Watson Scherp medium. The fermenter vessel is incubated at 35 to 37 deg. C. for 7 to 12 hours controlling dissolved oxygen content and pH with supplement feed and antifoam additions. After the incubation period, the contents of the fermentor vessel are transferred to a 500 L tank, Cetavlon™ is added, and the material mixed for 1 hours. The Cetavlon treated growth is centrifuged at approximately 15,000 to 17,000 x g at a flow rate of approximately 30 to 70 liters per hours. The crude polysaccharide is precipitated from the supernatant with a second Cetavlon™ precipitation. Cetavlon™ is added to the supernatant and the material mixed for at least 1 hour at room temperature. The material is stored at 1 to 5 deg. C. for 8 to 12 hours. The precipitated polysaccharide is collected centrifugation at approximately 45,000 to 50,000 x g at a flow rate of 300 to 400 ml per minute. The collected paste is stored at -60 deg. C. or lower until further processed.

### Purifed Polysaccharide Powder Preparation

The inactivated paste is thawed and transferred to a blender. The paste is blended with 0.9 M calcium chloride to yield a homogeneous suspension. The suspension is centrifuged at approximately 10,000 x g for 15 minutes. The supernatant is decanted through a lint free pad into a container as the first extract. A second volume of 0.9 M calcium chloride is added to the paste, and blended to yield a homogeneous suspension. The suspension is centrifuged as above, and the supernatant combined with the supernatant from the first extractions. A total of four extractions are performed, and the supernatants pooled. The pooled extracts are concentrated by ultrafiltration using 10-30 kDa MWCO spiral would ultrafiltration units.

Magnesium chloride is added to the concentrated, and the pH adjusted to 7.2 to 7.5 using sodium hydroxide. DNase and RNase are added to the concentrate, and incubated at 25 to 28 deg. C. with mixing for 4 hours. Ethanol is added to a concentration of 30 to 50%. Precipitated nucleic acid and protein are removed by centrifugation at 10,000 x g for 2 hours. The supernatant is recovered and the polysaccharide precipitated by adding ethanol to 80% and allowing it to stand overnight at 1 to 5 deg. C. The alcohol is siphoned off, and the precipitated polysaccharide is centrifuged for 5 minutes at 10,000 x g. The precipitated polysaccharide is washed with alcohol. The polysaccharide is washed with acetone, centrifuged at 15 to 20 minutes at 10,000 x g. The polysaccharide is dried under vacuum. The initial polysaccharide powder is dissolved into sodium acetate solution. Magnesium chloride is added and the pH adjusted to 7.2 to 7.5 using sodium hydroxide solution. DNase and RNase are added to the solution and incubated at 25 to 28 deg. C. with mixing for 4 hours to remove residual nucleic acids. After incubation with these enzymes, an equal volume of sodium acetate-phenol solution is added to the polysaccharide-enzyme mixture, and placed on a platform shaker at 1 to 5 deg. C. for approximately 30 minutes. The mixture is centrifuged at 10,000 x g for 15 to 20 minutes. The upper aqueous layer is recovered and saved. An equal volume of sodium acetate-phenol solution is added to the aqueous layer, and extracted as above. A total of four extractions are performed to remove protein and endotoxin from the polysaccharide solution. The combined aqueous extracts are diluted up to ten fold with water for injection, and diafiltered against 10 volumes of water for injection. Calcium chloride is added to the diafiltered polysaccharide. The polysaccharide is precipitated overnight at 1 to 5 deg. C. by adding ethanol to 80%. The alcohol supernatant is withdrawn, and the polysaccharide collected by centrifugation at 10,000 x g for 15 minutes. The purified polysaccharide is washed two times with ethanol, and once with acetone. The washed powder is dried under vacuum in a desiccator. The dried powder is stored at -30 deg. C. or lower until processed onto conjugate.

### Example 2 Depolymerization of Neisseria meningitidis serogroups A,C, W135, and Y Purified Capsular Polysaccharide Powder

Materials used in the preparation include purified capsular polysaccharide powders from *Neisseria meningitidis* serogroups A, C, W-135, and Y (prepared in accordance with Example 1), sterile 50 mM sodium acetate buffer, pH 6.0, sterile 1N hydrocholoric acid, sterile 1N sodium hydroxide, 30% hydrogen peroxide, and sterile physiological saline (0.85% sodium chloride).

Each serogroup polysaccharide is depolymerized in a separate reaction. A stainless steel tank is charged with up to 60 g of purified capsular polysaccharide powder. Sterile 50 mM sodium acetate buffer, pH 6.0 is added to the polysaccharide to yield a concentration of 2.5 g polysaccharide per liter. The polysaccharide solution is allowed to mix at 1 to 5 deg. C. for 12 to 24 hours to effect solution. The reaction tank is connected to a heat exchanger unit. Additional 50 mM sodium acetate buffer, pH 6.0, is added to dilute the polysaccharide to reaction concentration of 1.25 g per liter. The polysaccharide solution is heated to 55 deg. C..+-.0.1. An aliquot of 30% hydrogen peroxide is added to the reaction mixture to yield a reaction concentration of 1% hydrogen peroxide.

The course of the reaction is monitored by following the change in the molecular size of the polysaccharide over time. Every 15 to 20 minutes, aliquots are removed from the reaction mixture and injected onto a HPSEC column to measure the molecular size of the polysaccharide. When the molecular size of the polysaccharide reached the targeted molecular size, the heating unit is turned off and the polysaccharide solution rapidly cooled to 5 deg. C. by circulation through an ice water bath. The depolymerized polysaccharide - solution is concentrated to 15 g per liters by connecting the reaction tank to an ultrafiltration unit equipped with 3000 MWCO regenerated cellulose cartridges. The concentrated depolymerized polysaccharide solution is diafiltered against 10 volumes of sterile physiological saline (0.85% sodium chloride). The depolymerized polysaccharide is stored at 1 to 5 deg. C. until the next process step.

The molecular size of the depolymerized polysaccharide is determined by passage through a gel filtration chromatography column sold under the tradename "Ultahydrogel.TM.250" that is calibrated using Dextran molecular size standards and by multi-angle laser light scattering. The quantity of polysaccharide is determined by phosphorus content for serogroup A using the method of Bartlet, G. R. J. (1959) Journal of Biological Chemistry, 234, pp-466-468, and by the sialic acid content for serogroups C, W135 and Y using the method of Svennerholm, L. (1955) Biochimica Biophysica Acta 24, pp604-611. The O-acetyl content is determined by the method of Hesterin, S. (1949) Journal of Biological Chemistry 180, p249. Reducing activity is determined by the method of Park, J. T. and Johnson, M. J. (1949 Journal of Biological Chemistry 181, pp149-151. The structural integrity of the depolymerized polysaccharide is determined by protein sup. 1H and sup.13C NMR. The purity of the depolymerized polysaccharide is determined by measuring the LAL (endotoxin) content and the residual hydrogen peroxide content.

### Example 3 Derivatization of Neisseria meningitidis Serogroups A, C, W-135, and Y Depolymerized Polysaccharides

Materials used in this preparation include hydrogen peroxide depolymerized capsular polysaccharide serogroups A, C, W-135, and Y from *Neisseria meningitidis* (prepared in accordance with Example 2), adipic acid dihydrazide, 1-ethyl-3-(3-dimethylaminopropyl) carbodiimide (EDAC) for serogroup A only, sodium cyanborohydride, sterile 1N hydrocholoric acid, sterile 1N sodium hydroxide, sterile 1 M sodium chloride, and sterile physiological saline (0.85% sodium chloride).

Each serogroup polysaccharide is derivatized in a separate reaction. A stainless steel tank is charged with the purified depolymerized polysaccharide, and diluted with sterile 0.85% physiological saline to achieve a final reaction concentration of 6 g polysaccharide per liter. To this solution is added a concentrated aliquot of adipic acid dihydrazide dissolved in sterile 0.85% physiological saline, in order to achieve a reaction concentration of 1g per liter. For serogroup A only, EDAC is added as a concentrated aliquot dissolved in sterile 0.85% physiological saline, to achieve a reaction concentration of 1g per liter. The pH is adjusted to 5.0.+-.0.1, and this pH is maintained for 2 hours using sterile 1N hydrochloric acid and sterile 1N sodium hydroxide at room temperature (15 to 30 deg. C.). After two hours, a concentrated aliquot of sodium cyanoborohydride, dissolved in 0.85% physiological saline, is added to the reaction mixture to achieve a reaction concentration of 2 g per liter. The reaction is stirred at room temperature (15 to 30 deg. C.) for 44 hours.+-.4 hours while maintaining the pH at 5.5.+-.0.5. Following this reaction period, the pH is adjusted to 6.0.+-.0.1, and the derivatized polysaccharide is concentrated to 12 g polysaccharide per liter by connecting the reaction tank to a ultrafiltration unit equipped with a 3000 MWCO regenerated cellulose cartridges. The concentrated derivatized polysaccharide is diafiltered against 30 volumes of 1 M sodium chloride, followed by 10 volumes of 0.15 M sodium chloride. The tank is disconnected from the ultrafiltration unit and stored at 1 to 5 deg. C. for 7 days. The tank is reconnected to an ultrafiltration unit equipped with 3000 MWCO regenerated cellulose cartridges, and diafiltered against 30 volumes of 1M sodium chloride, followed by 10 volumes of 0.15 M sodium chloride. The molecular size of the derivatized polysaccharide, the quantity of polysaccharide, and the O-acetyl content are measured by the same methods used on the depolymerized polysaccharide. The hydrazide content is measured by the 2,4,6-trinitrobenzensulfonic acid method of Snyder, S. L. and Sobocinski, P. Z. (1975) Analytical Biochemistry 64, pp282-288. The structural integrity of the derivatized polysaccharide is determined by proton ¹H and ¹³C NMR. The purity of the derivatized polysaccharide is determined by measuring the level of unbound hydrazide, the LAL (endotoxin) content, and the residual cyanoborohydride content.

### Example 4 Preparation of Carrier Protein

### Preparation of Crude Diphtheria Toxoid Protein

Lyophilized seed cultures are reconstituted and incubated for 16 to 18 hours. An aliquot from the culture is transferred to a 0.5-liter flask containing growth medium, and the culture flask is incubated at 34.5 to 36.5 deg. C. on a rotary shaker for 7 to 9 hours. An aliquot from the culture flask is transferred to a 4-liter flask containing growth medium, and the culture flask is incubated at 34.5 to 36.5 deg. C. on a rotary shaker for 14 to 22 hours. The cultures from the 4-liter flask are used to inoculate a fermenter containing growth media. The fermenter is incubated at 34.5 to 36.5 deg. C. for 70 to 144 hours. The contents of the fermenter are filtered through depth filters into a collection vessel. An aliquot of formaldehyde solution, 37% is added to the harvest to achieve a concentration of 0.2%. The pH is adjusted to 7.4 to 7.6. The harvest is filtered through a 0.2 micron filter cartridge into sterile 20 liter bottles. The bottles are incubated at 34.5 to 36.5 deg. C. for 7 days. An aliquot of formaldehyde solution, 37%, is added to each 20 liter bottle to achieve a concentration of 0.4%. The pH of the mixtures is adjusted to 7.4 to 7.6. The bottles are incubated at 34.5 to 36.5 deg. C. for 7 days on a shaker. An aliquot of formaldehyde solution, 37%, is added to each 20 liter bottle to achieve a concentration of 0.5%. The pH of the mixtures is adjusted to 7.4 to 7.6. The bottles are incubated at 34.5 to 36.5 deg. C. for 8 weeks. The crude toxoid is tested for detoxification. The bottles are stored at 1 to 5 deg. C. during the testing period.

### Purification of the Crude Diphtheria Toxoid Protein

The crude toxoid is allowed to warm to room temperature, and the contents of the 20-liter bottles are combined into a purification tank. The pH of the toxoid is adjusted to 7.2 to 7.4, and charcoal is added to the crude toxoid and mixed for 2 minutes. The charcoal toxoid mixture is allowed to stand for 1 hours, and is then filtered through a depth filter cartridge into a second purification tank. Solid ammonium sulfate is added to the filtrate to achieve 70% of saturation. The pH is adjusted to 6.8 to 7.2, and the solution is allowed to stand for 16 hours. The precipitated protein is collected by filtration and washed with 70% of saturation ammonium sulfate solution, pH 7.0. The precipitate is dissolved into sterile distilled water, and the protein solution is filtered into a stainless steel collection vessel. The pH is adjusted to 6.8 to 7.2, and ammonium sulfate is added to 40% of saturation. The pH of the solution is adjusted to 7.0 to 7.2, and the solution is allowed to stand for 16 hours. The precipitate is removed by filtration and discarded. Ammonium sulfate is added to the filtrate to 60% of saturation, and the pH adjusted to 7.0 to 7.2. The mixture is allowed to stand for 16 hours, and the precipitated protein is collected by filtration. The precipitate is dissolved into sterile distilled water, filtered to remove undissolved protein, and diafiltered against 0.85% physiological saline.

### Concentration and Sterile Filtration of the Purified Diphtheria Toxoid Protein

The protein solution is concentrated to 15 g per liter and diafiltered against 10 volumes of 0.85% physiological saline suing a 10,000 MWCO regenerated cellulose filter cartridge. The concentrated protein solution is sterilized by filtration through a 0.2 micron membrane. The protein solution is stored at 1 to 5 deg. C. until processed onto conjugate.

The protein concentration is determined by the method of Lowry, O. H. et. al (1951) Journal of Biological Chemistry 193, p265-275. The purity of the protein is measured by sterility, LAL (endotoxin) content, and residual formaldehyde content.

### Example 5 Preparation of Monovalent Conjugates of Neisseria meningitidis Serogroups A, C, W-135, and Y Polysaccharide to Diphtheria Toxoid Protein

Materials used in this preparation include adipic acid derivatized polysaccharide from *Neisseria meningitidis* serogroups A, C, W-135, and Y (prepared in accordance with Example 3), sterile diphtheria toxoid protein (prepared in accordance with Example 4), EDAC, ammonium sulfate, sterile 1N hydrochloric acid, sterile 1N sodium hydroxide, and sterile physiological saline (0.85%).

Each serogroup polysaccharide conjugate is prepared by a separate reaction. All four conjugates are prepared by the following process. A stainless steel tank is charged with the purified adipic acid derivatized polysaccharide at a reaction concentration of 700 to 1000 .mu.moles of reactive hydrazide per liter and purified diphtheria toxoid protein at a reaction concentration of 3.8 to 4.0 g protein per liter. Physiological saline 0.85%, is used to dilute the starting materials to the target reaction concentrations and the pH is adjusted to 5.0.+-.0.1. An aliquot of EDAC is added to the polysaccharide protein mixture to achieve a reaction concentration of 2.28 to 2.4 g per liter. The pH of the reaction is kept at 5.0.+-.0.1 for 2 hours at 15 to 30 deg. C. After two hours, the pH is adjusted to 7.0.+-.0.1 using sterile 1N sodium hydroxide, and the reaction is stored at 1 to 5 deg. C. for 16 to 20 hours.

The reaction mixture is allowed to warm to 15 to 30 deg. C. and the reaction vessel is connected to an ultrafiltration unit equipped with a 30,000 MWCO regenerated cellulose cartridge. Solid ammonium sulfate is added to 60% of saturation (for serogroups A, W-135 and Y) and 50% of saturation (for serogroup C). The conjugate reaction mixture is diafiltered against 20 volumes of 60% of saturated ammonium sulfate solution (for serogroups A, W-135 and Y) and 50% of saturated ammonium sulfate solution (for serogroup C), followed by 20 volumes of physiological saline, 0.85%. The diafiltered conjugate is first filtered through a filter capsule containing a 1.2 micron and a 0.45 micron filter, and then through a second filter capsule containing a 0.22 micron filter.

The quantity of polysaccharide and 0-acetyl content are measured by the same methods used on the depolymerized and derivatized polysaccharide. The quantity of protein is determined by the Lowry method. The molecular size of the conjugate is determined by passage through a gel filtration chromatography column sold under the tradename "TSK6000PW" that used DNA as the void volume marker, ATP as the total volume marker, and bovine thyroglobulin as a reference marker. In addition, the molecular size of the conjugate eluted from the TKS6000PW column is-measured by multi-angle laser light scattering. The antigenic character of the conjugate is measured by binding to anti-polysaccharide serogroup specific antibody using double-sandwich ELISA method. The purity of the conjugates is determined by measuring the amount of unbound (unconjugated) polysaccharide by elution though a hydrophobic interaction chromatography column, unconjugated protein by capillary electrophoresis, sterility, LAL (endotoxin) content, residual EDAC content, and residual ammonium ion content.

### Example 6 Formulation of a Multivalent Meningococcal A, C, W-135, and Y Polysaccharide Diphtheria Toxoid Conjugate Vaccine

Materials used in this preparation include, serogroups A, C, W-135, and Y polysaccharide-diphtheria toxoid conjugates that are prepared in accordance with Example 5, sterile 100 mM sodium phosphate buffered physiological saline (0.85% sodium chloride).

An aliquot of sterile 100-500 mM sodium phosphate buffered physiological saline is added to physiological saline (0.85%) in a stainless steel bulking tank to yield a final vaccine concentration of 10 mM sodium phosphate. An aliquot of each of from two to four of the sterile monovalent meningococcal polysaccharide-diphtheria toxoid conjugates is added to the bulking tank containing 10 mM sterile sodium phosphate physiological saline to yield a final concentration of 8 µg of each serogroup polysaccharide per milliliter of buffer. The formulated tetravalent conjugate is mixed and filtered through a 0.2 .mu.m filter into a second bulking tank.

The quantity of each serogroup polysaccharide present in the multivalent formulation is determined by component saccharide analysis using high pH anion-exchange chromatography with pulsed amperometric detection. The quantity of protein is measured by the method of Lowry. Th pH of the vaccine is measured using a combination electrode connected to a pH meter. The antigenic character of the multivalent conjugate vaccine is measured by binding to anti-polysaccharide serogroup specific antibody using a double-sandwich ELISA method. Immunogenicity of the multivalent conjugate vaccine is measured the ability of each conjugate present in the vaccine to elicit both a primary and booster anti-polysaccharide IgG immune response in an animal model. The purity of the multivalent conjugate vaccine is determined by measuring the amount of unbound (unconjugated) polysaccharide using high pH anion-exchange chromatography with pulsed amperometric detection, sterility, LAL (endotoxin) content, pyrogenic content, and general safety.

### Example 7 Preparation of Aluminum-hydroxide Adjuvanted Multivalent Meningococcal Polysaccharide Diphtheria Toxoid Protein Conjugate

Preparation of conjugate adsorbed to aluminum hydroxide. Materials used in this preparation include serogroups A, C, W-135, and Y polysaccharide-diphtheria toxoid conjugates that are prepared in accordance with Example 5, sterile physiological saline (0.85% sodium chloride), and sterile aluminum hydroxide in physiological saline (0.85% sodium chloride).

An aliquot of each of the sterile monovalent meningococcal polysaccharide diphtheria toxoid conjugates is added to the bulking tank containing physiological saline to yield a final concentration of 8 µg of each serogroup polysaccharide per milliliter of buffer. An aliquot of sterile aluminum hydroxide in physiological saline (0.85% sodium chloride) is added to the multivalent conjugate vaccine to achieve a final concentration of 0.44 mg aluminum ion per milliliter vaccine.

### Example 8 Preparation of Aluminum Phosphate-adjuvanted Conjugate

Materials used in this preparation include serogroups A, C, W-135, and Y polysaccharide-diphtheria toxoid conjugates that are prepared according to Example 5, sterile physiological saline (0.85% sodium chloride), and sterile aluminum phosphate in physiological saline (0.85% sodium chloride).

An aliquot of each of the sterile monovalent meningococcal polysaccharide-diphtheria toxoid conjugates is added to the bulking tank containing physiological saline to yield a final concentration of 8 µg of each serogroup polysaccharide per milliliter of buffer. An aliquot of sterile aluminum phosphate in physiological saline (0.85% sodium chloride) is added to the multivalent conjugate vaccine to achieve a final concentration of 0.44 mg aluminum ion per milliliter vaccine.

### Example 9 General Description of Materials and Methods Used in Human Clinical Studies

### Immunogenicity of a Tetravalent Derivatized Conjugate Vaccines

The conjugate vaccine is studied for its ability to elicit an immune response in humans under a number of different clinical protocols. The following studies summarize the results. The materials and methods used in each of the following studies, unless indicated otherwise, are:

### TetraMenD

TetraMenD vaccine comprises four meningococcal capsular polysaccharides of serogroups A, C, Y, and W-135, 4 µg of each polysaccharide, covalently attached to a total of 48 µg diphtheria toxoid protein. The vaccine is formulated in sterile, pyrogen-free, phosphate-buffered physiological saline, with no preservative. The formula comprises 0.6 mg sodium phosphate, 4.4 mg sodium chloride and up to 0.5 mL water.

### Menomune®

Menomune® is licensed in the United States and elsewhere for use among persons aged 2 years and older. Menomune is a freeze-dried preparation, each dose of vaccine containing 50 ug of each A, C, Y and W-135 polysaccharide as antigens, reconstituted with a diluent of isotonic sodium chloride solution preserved with thimerosal and given subcutaneously as a 0.5 mL dose. Each 0.5 mL dose of vaccine contains 2.5 mg to 5 mg of lactose as a stabilizer. Menomune^{®} - A/C/Y/W-135, Meningococcal Polysaccharide Vaccine, Groups A, C, Y, and W-135 Combined, for subcutaneous use, is a freeze-dried preparation of the group-specific polysaccharide antigens from *Neisseria meningitidis,* Group A, Group C, Group Y, and Group W-135. The diluent is sterile, pyrogen-free, distilled water. After reconstitution of the lyophilized product with diluent as indicated on the label, each 0.5 mL dose is formulated to contain 50 µg of "isolated product" from each of the serogroups A, C, Y, and W-135 in isotonic sodium chloride solution.

Tetanus and Diphtheria Toxoids Adsorbed for Adult Use^{®} (referred to subsequently as Td) is a sterile suspension of alum precipitated toxoid in an isotonic sodium chloride solution containing sodium phosphate buffer to control pH. The vaccine is for intramuscular injection. Each 0.5mL dose is formulated to contain 5Lf of tetanus toxoid, 2Lf of diphtheria toxoid and not more than 0.28 mg of aluminum by assay. Tetanus and diphtheria toxoids induce at least 2 units and 0.5 units of antitoxin per mL respectively in the guinea pig potency test. At visit 1, Td is administered to all participants as a single 0.5mL dose by intramuscular injection using a one inch 25 gauge needle into the deltoid of the left arm. Each 0.5mL dose contains 5Lf of tetanus toxoid and 2Lf of diphtheria toxoid.

### Sera Samples

Blood specimens are drawn on the Days indicated after the baseline. For example, if the protocol indicates three time points, D0, D28 and 6 Month, then blood specimens are drawn on Day 0 prior to vaccination (baseline), at Day 28 post-vaccination (to assess primary immune response, and at 6 month post-vaccination (to assess the longevity of the immune response). Approximately 5 mL of whole blood is collected from each subject at each time point. The whole blood is centrifuged within four hours of collection. The serum is removed and stored at -20 deg. C. A 'Day 28' blood sample is taken at least 28 days but not yet 57 days after the Day 0 injection. A "6 month" blood sample is taken at 6 months plus or minus 28 days after the Day 0 injection. Thus, a Day 28 sera represents a sera is drawn between day 28 to day 56 after D0; and a 6 month sera represents a sera is drawn between day 149 to day 217 after D0.

### Assay Techniques

The present studies utilize a number of standard immunological assays. The following descriptions summarize the methodologies used herein. However, other similar assays, including variations of those presented herein, are well known by those in the art and may be utilized.

### Anti-Meningococcal Antibody Determination by a Serum Bactericidal Assay using Baby Rabbit Complement (SBA-BR)

Functional antibody activity for anti-meningococcal antibody to serogroups A, C, Y, and W-135 is measured using a serum bactericidal assay. Two-fold dilutions of test sera are prepared in sterile 96-well microtiter plates. Serogroup specific meningococcal bacteria along with baby rabbit complement are added to the serum dilutions and allowed to incubate. After this incubation period, an agar overlay medium is added to the serum/complement/bacteria mixture, allowed to harden, and then incubated overnight at 37°C with 5% CO₂, Bacterial colonies present in the wells are counted. The endpoint titer is determined by the reciprocal serum dilution yielding > 50% killing as compared to the mean of the complement control wells. The limit of detection for this assay, using rabbit complement, is a titer of 8.

### IgG Anti-Meningococcal Antibody Determination

IgG antibody activity for anti-meningococcal antibody to serogroups A, C, Y, and W-135 is measured using an indirect ELISA. This procedure involves reacting antibody in sera with excess meningococcal group specific polysaccharide (MenPs) antigen adsorbed to plastic microtiter wells by methylated human serum albumin. The amount of bound antibody is determined by a reaction with peroxidase-labeled mouse anti-human IgG specific monoclonal antibody. A subsequent reaction using peroxidase substrate generates a chromogenic product that is measured spectrophotometrically. The resulting optical density (OD) correlates with the amount of IgG antibody in the serum that is bound to the Meningococcal polysaccharide on the microtiter plate. The amount of IgG antibody is then calculated by comparison to a reference (Lot CDC 1992 or equivalent) with an assigned value using a 4-parameter logistic curve method.

### IgM Anti-Meningoeoccal Antibody Determination

IgM antibody activity for anti-meningococcal antibody to serogroups A, C, Y, and W-135 is measured using an indirect ELISA. This procedure involves reacting antibody in sera with excess MenPs antigen adsorbed to plastic microtiter wells by methylated human serum albumin. The amount of bound antibody is determined by a reaction with peroxidase-labeled mouse anti-human IgM specific monoclonal antibody. A subsequent reaction using peroxidase substrate generates a chromogenic product that is measured spectrophotometrically. The resulting OD correlates with the amount of IgM antibody in the serum that is bound to the meningococcal polysaccharide on the microtiter plate. The amount of IgM antibody is then calculated by comparison to a reference (Lot CDC 1992 or equivalent) with an assigned value using a 4-parameter logistic curve.

### High Avidity Anti-Meningoeoccal IgG Antibody Determination

High avidity IgG antibody activity for anti-meningococcal antibody to serogroups A, C, Y, and W-135 will be measured at Aventis Pasteur Inc. using a modified ELISA. This assay is currently under development at Aventis Pasteur Inc. and will be qualified prior to testing of clinical specimens. Briefly, 96 well microtiter plates are coated with MenPs antigen. After aspirating and ishing the coated plates, serial dilutions of clinical sera are prepared directly in the plates, using phosphate buffered saline (PBS) serum diluting buffer containing 75 mM ammonium thiocyanate, and allowed to incubate overnight. The amount of bound antibody is determined by a reaction with peroxidase-labeled mouse anti-human IgG specific monoclonal antibody. A subsequent reaction using peroxidase substrate generates a chromogenic product that is measured spectrophotometrically. The resulting OD correlates with the amount of high avidity IgG antibody in the serum that is bound to the meningococcal polysaccharide on the microtiter plate. The amount of high avidity IgG antibody is then calculated by comparison to a reference (Lot CDC 1992 or equivalent) using a 4-parameter logistic curve.

### IgG1 and IgG2 Subclass Meningococcal Antibody Determination

IgG1 and IgG2 subclass antibody distribution for anti-meningococcal antibody to serogroups A, C, Y and W-135 is measured using an ELISA. Antibody present in serial dilutions of sera is reacted with MenPs antigen adsorbed to the wells of microtiter plates. The amount of bound antibody will be determined using anti-human IgG1 Fc or IgG2 Fc specific reagents. A subsequent reaction with enzyme substrate generates a chromogenic product that is measured spectrophotometrically. The resulting OD correlates with the amount of IgG1 or IgG2 antibody in the serum that is bound to the meningococcal polysaccharide on the microtiter plate. The amount of antibody will be reported as the IgG1 :IgG2 ratio in the serum specimen or as the concentration of IgG1 or IgG2 in the specimen if a suitable reference is available.

### Anti-Diphtheria Antibody Determination By Metabolic Inhibition of VERO Cells

Anti-diphtheria antibody responses are measured by the ability of the test sera to protect VERO cells from a diphtheria toxin challenge. Using sterile 96-well microtiter plates, two-fold dilutions of test sera, beginning with a 1:4 dilution, are challenged with diphtheria toxin and allowed to incubate. VERO cells are then added, the wells sealed with sterile mineral oil and incubated for six to eight days. Antibody levels are then determined by observing a color change of the pH indicator in the media resulting from the byproducts of cell metabolism. Results are reported as International Units/mL by comparison to a calibrated WHO reference serum and determined by the highest serum dilution that allows cell metabolism in the presence of the challenge dose of diphtheria toxin. The lower limit of detection is determined by the minimum detectable antitoxin level of the reference serum, and the starting dilution of the test sera, and is typically 0.005 IU/mL.

### Anti Tetanus Antibody Determination By Elisa

Anti-Tetanus antibody levels are determined by an indirect Enzyme Linked Immunosorbent Assay (ELISA). The method involves reacting antibody in test sera with tetanus toxoid adsorbed to plastic microtiter wells. The amount of bound antibody is-determined by a reaction with Goat Anti-Human IgG-specific antibody conjugated to alkaline phosphatase. A subsequent reaction with alkaline phosphatase substrate generates a chromogenic product that is measured spectrophotometrically. The OD (optical density) correlates with the amount of antibody in the serum dilution that binds to the antigen coated microtiter plate. The antibody concentration is calculated by comparison to an international human reference (WHO Lot TE-3) with assigned unitage by a Parallel Line Analysis method. Results are reported as International Units per milliliter (IU/mL). The minimum level of quantitation for the anti-tetanus IgG ELISA is 0.01 IU/mL, with samples resulting in values lower than this level reported as < 0.01 IU/mL.

As used herein, "Adverse Event", "Serious Adverse Experience", and "Unexpected Adverse Experience" are terms well understood within the vaccine industry. The safety data are summarized and analyzed in accordance with standard clinical practice, which including assessing all participants who received vaccine for the duration of the clinical study. In general, each of the terms is understood to have the following meanings.

Adverse Event (AE) is defined as "any untoward medical occurrence in a patient or clinical investigation subject administered a pharmaceutical product and that does not necessarily have a causal relationship with this treatment. An adverse event can therefore be any unfavorable and unintended sign (including an abnormal laboratory finding), symptom, or disease temporally associated with the use of a medicinal (investigational) product, whether or not related to the medicinal (investigational) product." (ICH guidelines, GCP (E6) § 1.2). Serious Adverse Experience (SAE) is "any adverse drug experience occurring at any dose that results in any of the following outcomes: death, a life-threatening adverse drug experience, inpatient hospitalization or prolongation of existing hospitalization, a persistent or significant disability/incapacity, or a congenital anomaly/birth defect. Important medical events that may not result in death, be life-threatening, or require hospitalization may be considered a serious adverse drug experience when, based upon appropriate medical judgement, they may jeopardize the patient or subject and may require medical or surgical intervention to prevent one of the outcomes listed in this definition. Examples of such medical events include allergic bronchospasm requiring intensive treatment in an emergency room or at home, blood dyscrasias or convulsions that do not result in inpatient hospitalization, or the development of drug dependency or drug abuse." (21 CFR Ch. I, §312.32(a)).

Unexpected Adverse Experience (UAE) is "any adverse drug experience, the specificity or severity of which is not consistent with the current investigator's brochure; or, if an investigator brochure is not required or available, the specificity or severity of which is not consistent with the risk information described in the general investigational plan or elsewhere in the current application, as amended." (21 CFR Ch. I, §312.32(a)).

The studies conducted in accordance with standard clinical practice, and the criteria for enrollment or exclusion of patients in the studies are:
Inclusion criteria for patients:
   1. Participant is healthy, as determined by medical history and physical examination.
   2. Participant is at least 11 years of age but not yet 19 years of age at the time of vaccination.
   3. Parent/guardian or participant has signed Institutional Review Board (IRB) approved informed consent form where applicable.
   4. Participant has signed Institutional Review Board (IRB) approved assent form where applicable
Exclusion criteria for patients:
   1. Serious chronic disease (i.e. cardiac, renal, neurologic, metabolic, rheumatologic, etc.).
   2. Known or suspected impairment of immunologic function.
   3. Acute medical illness with or without fever within the last 72 hours or an oral temperature ≥38°C (100.4°F) at the time of inclusion.
   4. History of documented invasive meningococcal disease or previous meningococcal vaccination.
   5. Administration of immune globulin, other blood products within the last 3 months, or oral or injected corticosteroids or other immunomodulatory therapy within 6 weeks of the study vaccine. Individuals on a tapering dose schedule of oral steroids lasting < 7 days may be enrolled in the trial as long as they have not received more that one course within a two week period prior to enrollment
   6. Antibiotic therapy within the 72 hours prior to vaccination
   7. Received any vaccine in the 28-day period prior to enrollment, or scheduled to receive any vaccination in the 28-day period after enrollment, except where the study notes additional vaccinations.
   8. Suspected or known hypersensitivity to any of the vaccine components.
   9. Unavailable for the entire study period or unable to attend the scheduled visits or to comply with the study procedures.
   10. Enrolled in another clinical trial.
   11. Any condition which, in the opinion of the investigator, would pose a health risk to the participant or interfere with the evaluation of the vaccine.
   12. In females, a positive or equivocal urine pregnancy test at the time of vaccination.

### Example 10 Study A -Dosage Study

Study A is an unblinded, open-label, dose-escalation trial of three dosage levels of TetraMenD vaccine, administered to participants in three age groups. Ninety healthy adults (18 to 55 years of age) are enrolled in Stage I and received a single injection of TetraMenD vaccine. Thirty healthy children (12 to 22 months of age) are enrolled in Stage II and received 2 injections of a single dosage level of TetraMenD vaccine. Ninety healthy infants' (6 to 12 weeks of age) are enrolled in Stage III and received 3 injections of a single dosage level of TetraMenD vaccine.

### Stage IDosage Study in Adults 18 to 55 Years

This clinical trial is an unblinded, open-label, dose-escalation trial of three dosage levels of TetraMenD vaccine, which is administered to participants in three age groups. In Stage 1, ninety healthy adults (18 to 55 years of age) receive a single injection ofTetraMenD vaccine.

For adult participants, serum specimens for serologic analysis are obtained at baseline (day 0) prior to TetraMenD administration, and at day 28 after TetraMenD administration. All available specimens are analyzed for SBA against meningococcal polysaccharide ₅erogr_{OU}p_{S} A, C, Y, and W-135, and by ELISA for IgG antibody against these same serogroups. The SBA and IgG ELISA findings for all serogroups are summarized below.

One key immunogenicity endpoint is the proportion of participants with a ≥4-fold rise from baseline. To determine what effect the baseline SBA titer had on the proportion with a ≥4-fold rise in SBA, a subgroup analysis is performed for adults whose baseline titer for each particular antigen is less than 1:64, and adults whose baseline titer for that particular antigen is at least 1:64.

The safety profile of TetraMenD is comparable to that of Menomune^{®}. The results of this Study are summarized in the following Tables.

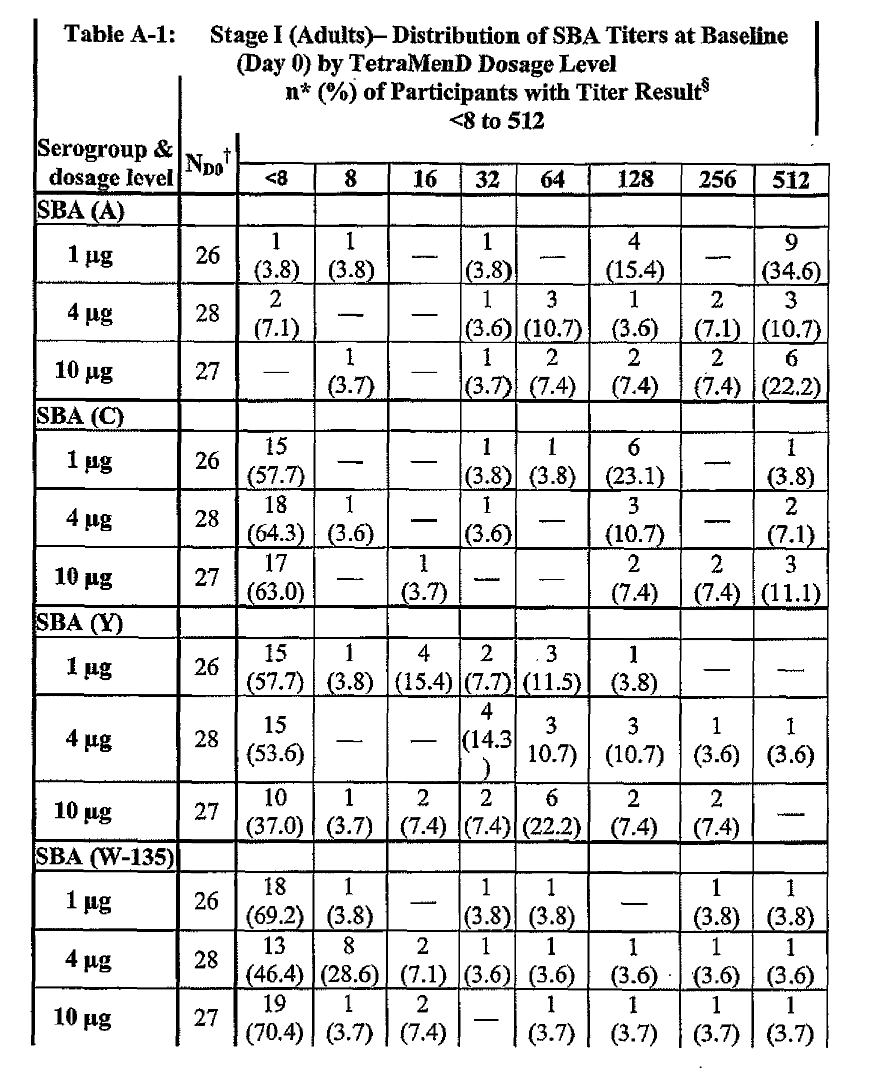

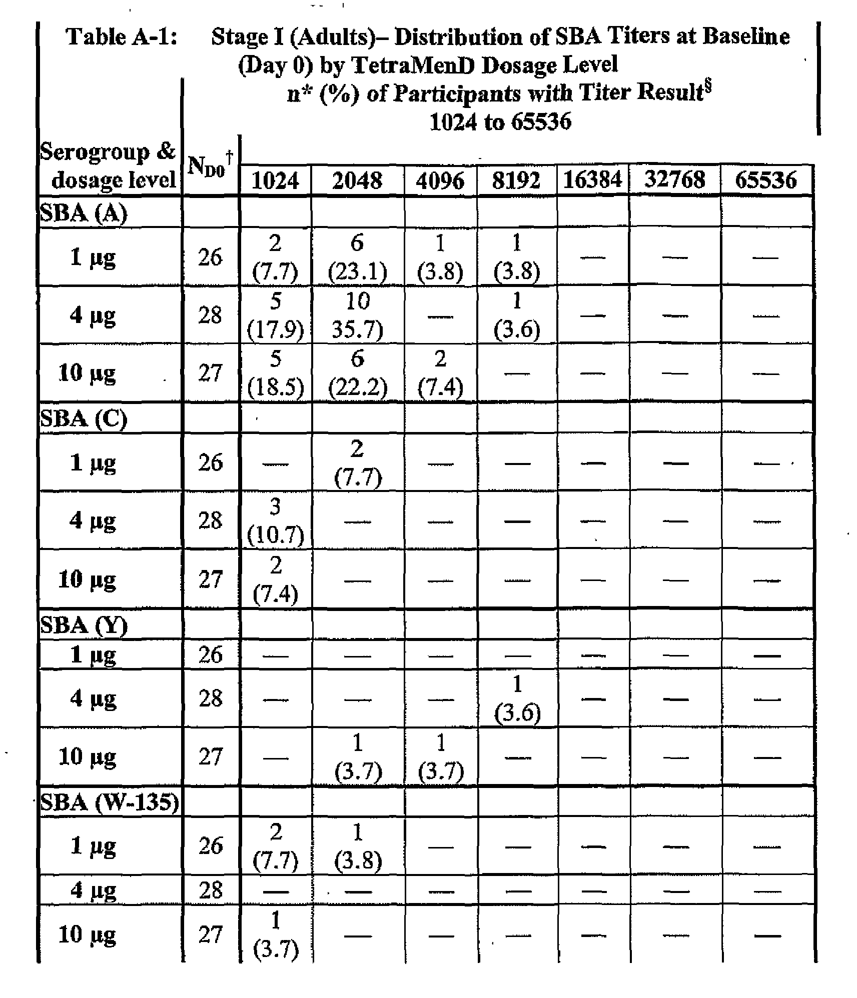

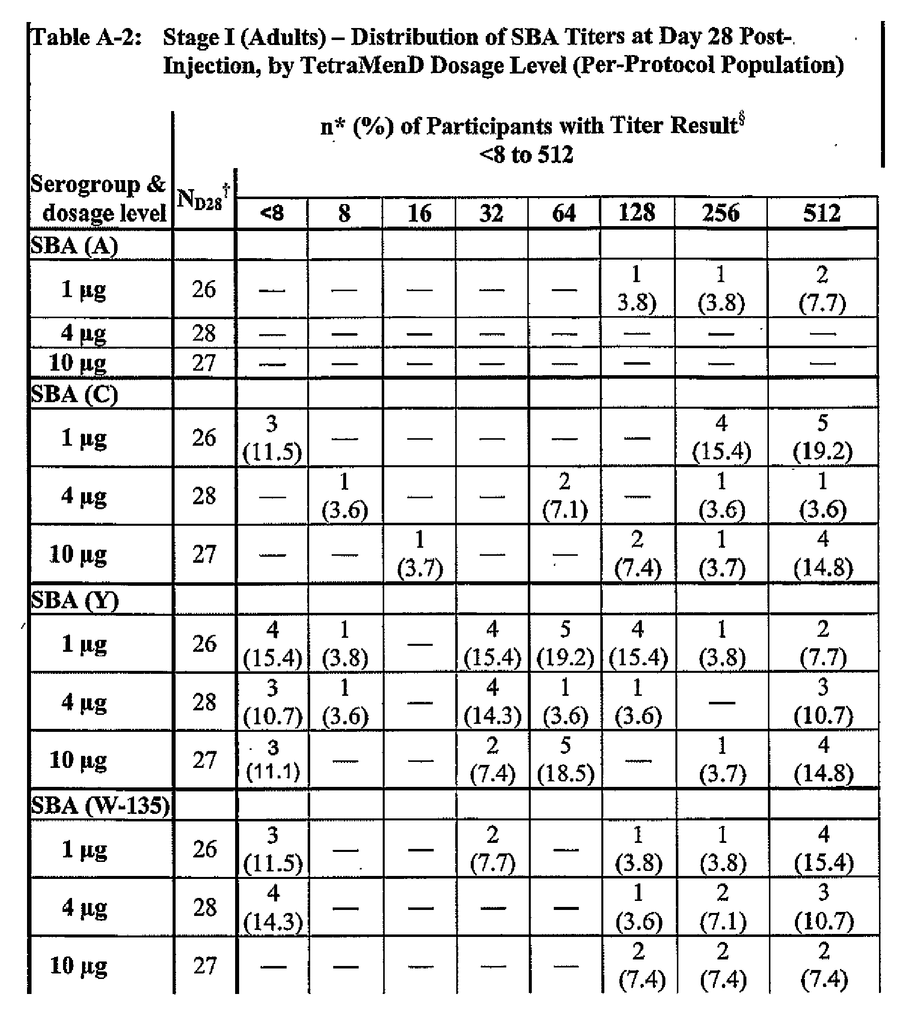

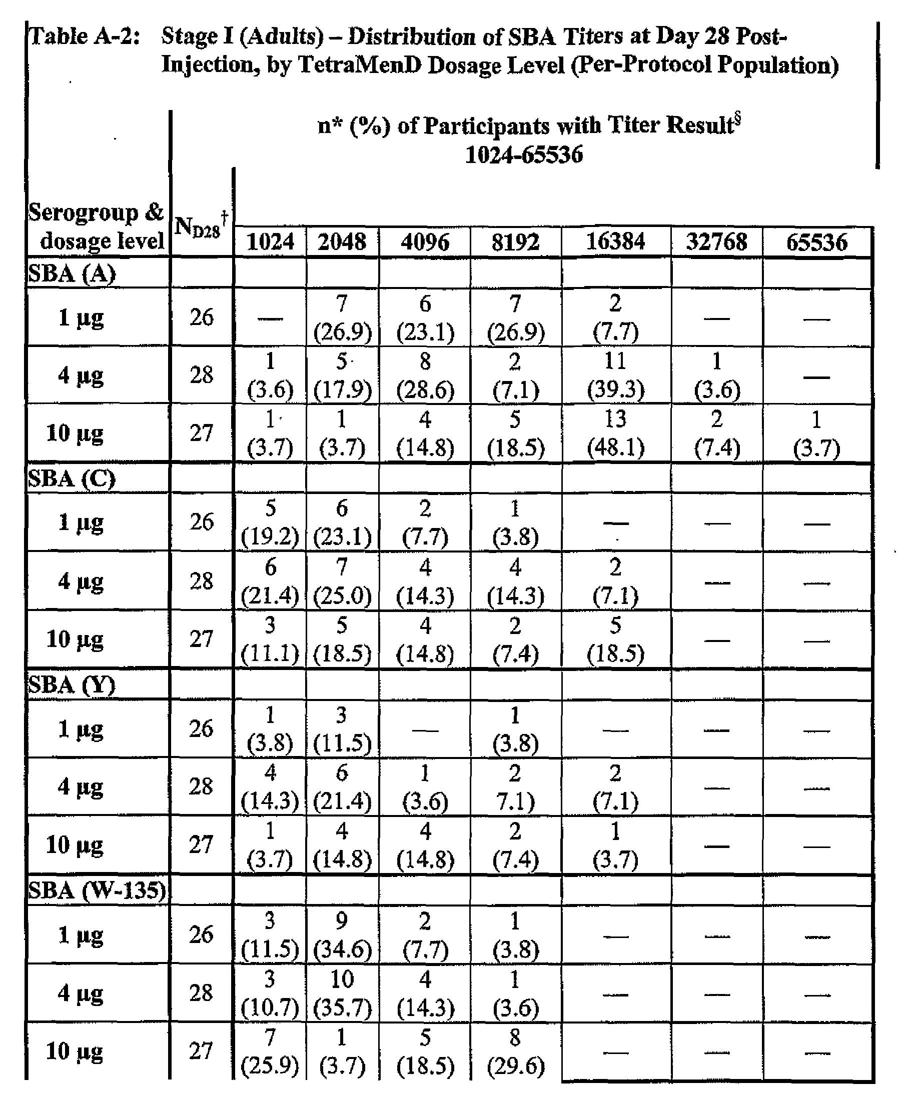

**Table A-3: Stage I (Adults) - Proportions Achieving SBA Thresholds at Baseline and at Day 28 Post-Injection, By TetraMenD Dosage Level (per-Protocol Population)**

| **Serogroup & dosage level** | **N_{D0}/N _{D28}** | **% Achieving Threshold** | | | | | | | |
|---|---|---|---|---|---|---|---|---|---|
| | | **≥1:8** | | **≥1:16** | | **≥1:32** | | **≥1:64** | |
| | | **Day 0** | **Day 28** | **Day 0** | **Day 28** | **Day 0** | **Day 28** | **Day 0** | **Day 28** |
| **SBA (A)** | | | | | | | | | |
| **1 µg** | 26/26 | 96.2 | 100.0 | 92.3 | 100.0 | 92.3 | 100.0 | 88.5 | 100.0 |
| **4 µg** | 28/28 | 92.9 | 100.0 | 92.9 | 100.0 | 92.9 | 100.0 | 89.3 | 100.0 |
| **10 µg** | 27/27 | 100.0 | 100.0 | 96.3 | 100.0 | 96.3 | 100.0 | 92.6 | 100.0 |
| **SBA (C)** | | | | | | | | | |
| **1 µg** | 26/26 | 42.3 | 88.5 | 42.3 | 88.5 | 42.3 | 88.5 | 38.5 | 88.5 |
| **4 µg** | 28/28 | 35.7 | 100.0 | 32.1 | 96.4 | 32.1 | 96.4 | 28.6 | 96.4 |
| **10 µg** | 27/27 | 37.0 | 100.0 | 37.0 | 100.0 | 33.3 | 96.3 | 33.3 | 96.3 |
| **SBA (Y)** | | | | | | | | | |
| **1 µg** | 26/26 | 42.3 | 84.6 | 38.5 | 80.8 | 23.1 | 80.8 | 15.4 | 65.4 |
| **4 µg** | 28/28 | 46.4 | 89.3 | 46.4 | 85.7 | 46.4 | 85.7 | 32.1 | 71.4 |
| **10 µg** | 27/27 | 63.0 | 88.9 | 59.3 | 88.9 | 51.9 | 88.9 | 44.4 | 81.5 |
| **SBA (W-135)** | | | | | | | | | |
| **1 µg** | 26/26 | 30.8 | 88.5 | 26.9 | 88.5 | 26.9 | 88.5 | 23.1 | 80.8 |
| **4 µg** | 28/28 | 53.6 | 85.7 | 25.0 | 85.7 | 17.9 | 85.7 | 14.3 | 85.7 |
| **10 µg** | 27/27 | 29.6 | 100.0 | 25.9 | 100.0 | 18.5 | 100.0 | 18.5 | 100.0 |

| | | | | | | | | | |
|---|---|---|---|---|---|---|---|---|---|
| N: number of evaluable participants at each time point (day 0; day 28) | | | | | | | | | |

**Table A-4: Stage I (Adults) - SBA and IgG ELISA Results at Baseline and at Day 28 Post-Injection, by TetraMenD Dosage Level (Per-Protocol Population)**

| **Serogroup & dosage level** | **N_{D0}/N_{D28}** | **GMT/GMC* (95% CI)** | | **Mean Fold** ↑ **at Day 28 (95% CI)** | **% ≥4-Fold** ↑ |
|---|---|---|---|---|---|
| | | **Day 0** | **Day 28** | | |
| **SBA (A)** | | | | | |
| **1 µg** | 26/26 | 460.2 | 3054.9 | 6.6 | 65.4 |
| | | (223.0-949.7) | (1872.9-4982.9) | (2.9-15.4) | |
| **4 µg** | 28/28 | 487.3 | 6720.2 | 13.8 | 71.4 |
| | | (231.2-1027.2) | (4666.5-9677.7) | (6.0-31.7) | |
| **10 µg** | 27/27 | 525.3 | 10865.1 | 20.7 | 96.3 |
| | | (286.6-962.9) | (7651.5-15428.2) | (11.7-36.6) | |
| **SBA (C)** | | | | | |
| **1 µg** | 26/26 | 20.9 | 540.0 | 25.9 | 73.1 |
| | | (8.8-49.6) | (238.1-1224.7) | (9.7-68.6) | |
| **4 µg** | 28/28 | 16.4 | 1559.8 | 95.1 | 89.3 |
| | | (7.1-37.7) | (799.9-3041.5) | (39.7-227.7) | |
| **10 µg** | 27/27 | 19.2 | 1755.6 | 91.7 | 96.3 |
| | | (8.0-45.8) | (880.5-3500.4) | (37.6-223.5) | |
| **SBA (Y)** | | | | | |
| **1 µg** | 26/26 | 9.4 | 95.5 | 10.2 | 73.1 |
| | | (5.9-14.9) | (40.5-225.0) | (4.9-20.9) | |
| **4 µg** | 28/28 | 19.0 | 390.0 | 20.5 | 82.1 |
| | | (8.8-41.2) | (143.3-1061.3) | (8.9-47.4) | |
| **10 µg** | 27/27 | 28.1 | 386.0 | 13.7 | 66.7 |
| | | (12.9-61.6) | (145.2-1026.2) | (5.8-32.7) | |
| **SBA (W-135)** | | | | | |
| **1 µg** | 26/26 | 13.6 | 498.5 | 36.6 | 76.9 |
| | | (5.7-32.4) | (203.2-1223.2) | (13.7-97.7) | |
| **4 µg** | 28/28 | 10.0 | 608.9 | 60.9 | 85.7 |
| | | (5.9-16.9) | (250.3-1480.9) | (23.7-156.7) | |
| **10 µg** | 27/27 | 9.8 | 1848.1 | 188.1 | 100.0 |
| | | (5.0-19.1) | (1075.4-3176.2) | (94.0-376.7) | |
| **IgG ELISA (A)** | | | | | |
| **1 µg** | 26/26 | 3.4 | 19.4 | 5.7 | 69.2 |
| | | (1.8-6.6) | (11.6-32.3) | (3.8-8.3) | |
| **4 µg** | 28/28 | 3.3 | 38.4 | 11.5 | 75.0 |
| | | (2.3-4.8) | (22.2-66.4) | (7.4-18.0) | |
| **10 µg** | 27/27 | 3.1 | 56.4 | 18.1 | 88.9 |
| | | (1.7-5.6) | (31.8-99.9) | (12.2-26.9) | |
| **IgG ELISA (C)** | | | | | |
| **1 µg** | 26/26 | 0.3 | 2.2 | 7.3 | 61.5 |
| | | (0.2-0.5) | (1.2-4.1) | (4.0-13.4) | |
| **4 µg** | 28/28 | 0.4 | 5.5 | 14.2 | 82.1 |
| | | (0.2-0.7) | (3.0-10.1) | (8.7-23.2) | |
| **10 µg** | 27/27 | 0.5 | 11.1 | 22.8 | 88.9 |
| | | (0.3-0.9) | (5.5-22.5) | (13.4-38.8) | |
| **IgG ELISA (Y)** | | | | | |
| **1 µg** | 26/26 | 0.6 | 2.8 | 4.6 | 53.8 |
| | | (0.4-1.0) | (1.5-5.2) | (2.7-7.6) | |
| **4 µg** | 28/28 | 1.3 | 6.8 | 5.4 | 53.6 |
| | | (0.7-2.5) | (3.2-14.6) | (3.2-9.0) | |
| **10 µg** | 27/27 | 1.0 | 7.7 | 7.4 | 77.8 |
| | | (0.5-2.1) | (3.4-17.2) | (5.1-10.8) | |
| **IgG ELISA (W-135)** | | | | | |
| **1 µg** | 26/26 | 0.5 | 2.3 | 4.4 | 46.2 |
| | | (0.3-0.9) | (1.0-5.3) | (2.4-7.9) | |
| **4 µg** | 28/28 | 0.6 | 5.8 | 10.2 | 71.4 |
| | | (0.3-1.0) | (2.9-11.7) | (6.1-16.9) | |
| **10 µg** | 27/27 | 0.4 | 9.3 | 22.7 | 85.2 |
| | | (0.2-0.7) | (4.8-18.1) | (11.9-43.2) | |

| | | | | | |
|---|---|---|---|---|---|
| Day 0: Baseline blood sample drawn prior to vaccination. Day 28: Blood sample drawn 28 days following vaccination. % ≥4-fold rise: the percent of adults who had a ≥ 4-fold rise in GMT at day 28 in comparison to day 0. N: number of evaluable participants *GMTs are computed for the SBA data; GMCs are computed for the IgG ELISA data. | | | | | |

**Table A-5 presents the GMT by dose, patient age and serogroup.**

| **Table A-5 GMT by Dose, patient age and Serogroup** | | | | | | | |
|---|---|---|---|---|---|---|---|
| **Age** | **Dose TetraMenD** | **No. of Subjects** | **Blood Day** | **A GMT** | **C GMT** | **W GMT** | **Y GMT** |
| **19** | **1 µg** | 2 | 0 | 1024.00 | 512.00 | 45.25 | 8.00 |
| | **1 µg** | 2 | 28 | 2896.31 | 2048.00 | 2048.00 | 362.04 |
| | **4 µg** | 2 | 0 | 2048.00 | 64.00 | 16.00 | 181.02 |
| | **4 µg** | 2 | 28 | 8192.00 | 8192.00 | 2048.00 | 2048.00 |
| | **10 µg** | 10 | 0 | 1097.50 | 34.30 | 21.11 | 128.00 |
| | **10 µg** | 10 | 28 | 18820.27 | 3821.70 | 2352.53 | 1176.27 |
| **20** | **1 µg** | 3 | 0 | 322.54 | 10.08 | 10.08 | 12.70 |
| | **1 µg** | 3 | 28 | 3251.00 | 203.19 | 2048.00 | 203.19 |
| | **4 µg** | 2 | 0 | 512.00 | 4.00 | 4.00 | 4.00 |
| | **4 µg** | 2 | 28 | 5792.62 | 128.00 | 22.63 | 22.63 |
| | **10 µg** | 1 | 0 | 128.00 | 4.00 | 4.00 | 4.00 |
| | **10 µg** | 1 | 28 | 16384.00 | 4096.00 | 128.00 | 2048.00 |
| **21** | **1 µg** | 2 | 0 | 1024.00 | 22.63 | 32.00 | 256.00 |
| | **1 µg** | 1 | 28 | 8192.00 | 8192.00 | 2048.00 | 1024.00 |
| | **4 µg** | 3 | 0 | 812.75 | 12.70 | 4.00 | 256.00 |
| | **4 ug** | 3 | 28 | 10321.27 | 1024.00 | 5160.64 | 8192.00 |
| **22** | **1 µg** | 2 | 0 | 1024.00 | 4.00 | 11.31 | 16.00 |
| | **1 µg** | 2 | 28 | 4096.00 | 362.04 | 2048.00 | 512.00 |
| | **4 µg** | 2 | 0 | 128.00 | 45.25 | 5.66 | 16.00 |
| | **4 µg** | 2 | 28 | 8192.00 | 2896.31 | 1024.00 | 2048.00 |
| | **10 µg** | 2 | 0 | 2048.00 | 90.51 | 4.00 | 22.63 |
| | **10 µg** | 2 | 28 | 16384.00 | 8192.00 | 512.00 | 512.00 |
| **23** | **1 µg** | 1 | 0 | 512.00 | 2048.00 | 2048.00 | 16.00 |
| | **1 µg** | 1 | 28 | 512.00 | 2048.00 | 2048.00 | 64.00 |
| | **4 µg** | 2 | 0 | 1448.15 | 4.00 | 4.00 | 45.25 |
| | **4 µg** | 2 | 28 | 4096.00 | 1448.15 | 1024.00 | 256.00 |
| | **10 µg** | 1 | 0 | 1024.00 | 128.00 | 4.00 | 64.00 |
| | **10 µg** | 1 | 28 | 16384.00 | 512.00 | 8192.00 | 2048.00 |
| **24** | **4 ug** | 2 | 0 | 181.02 | 11.31 | 90.51 | 4.00 |
| | **4 µg** | 2 | 28 | 11585.24 | 2896.31 | 1448.15 | 512.00 |
| | **10 µg** | 1 | 0 | 1024.00 | 4.00 | 4.00 | 4.00 |
| | **10 µg** | 1 | 28 | 16384.00 | 2048.00 | 4096.00 | 4.00 |
| **25** | **1 µg** | 1 | 0 | 128.00 | 4.00 | 4.00 | 4.00 |
| | **1 µg** | 1 | 28 | 4096.00 | 4096.00 | 2048.00 | 32.00 |
| | **10 µg** | 2 | 0 | 1024.00 | 32.00 | 4.00 | 22.63 |
| | **10 µg** | 2 | 28 | 8192.00 | 2896.31 | 1448.15 | 2048.00 |
| **26** | **1 µg** | 2 | 0 | 2048.00 | 4.00 | 64.00 | 4.00 |
| | **1 µg** | 2 | 28 | 4096.00 | 512.00 | 2896.31 | 1448.15 |
| | **10 µg** | 2 | 0 | 362.04 | 45.25 | 22.63 | 4.00 |
| | **10 µg** | 2 | 28 | 5792.62 | 2048.00 | 1448.15 | 32.00 |
| **27** | **1 µg** | 1 | 0 | 512.00 | 128.00 | 1024.00 | 4.00 |
| | **1 ug** | 1 | 28 | 4096.00 | 1024.00 | 4096.00 | 4.00 |
| | **4 µg** | 2 | 0 | 90.51 | 22.63 | 64.00 | 16.00 |
| | **4 µg** | 2 | 28 | 6384.00 | 4096.00 | 1448.15 | 90.51 |
| | **10 µg** | 1 | 0 | 512.00 | 4.00 | 128.00 | 64.00 |
| | **10 µg** | 1 | 28 | 8192.00 | 16384.00 | 8192.00 | 16384.00 |
| **28** | **1 µg** | 1 | 0 | 4.00 | 4.00 | 4.00 | 4.00 |
| | **1 µg** | 1 | 28 | 16384.00 | 1024.00 | 2048.00 | 256.00 |
| | **4 µg** | 2 | 0 | 724.08 | 64.00 | 8.00 | 11.31 |
| | **4 µg** | 2 | 28 | 5792.62 | 1448.15 | 2048.00 | 2048.00 |
| | **10 µg** | 1 | 0 | 64.00 | 4.00 | 4.00 | 4.00 |
| | **10 µg** | 1 | 28 | 4096.00 | 16.00 | 1024.00 | 4.00 |
| **30** | **4 µg** | 1 | 0 | 32.00 | 4.00 | 16.00 | 32.00 |
| | **4 µg** | 1 | 28 | 16384.00 | 16384.00 | 8192.00 | 4096.00 |
| **31** | **1 µg** | 1 | 0 | 32.00 | 4.00 | 4.00 | 4.00 |
| | **1 µg** | 1 | 28 | 8192.00 | 512.00 | 4.00 | 4.00 |
| **32** | **4 µg** | 2 | 0 | 724.08 | 64.00 | 8.00 | 11.31 |
| | **4 µg** | 2 | 28 | 4096.00 | 1024.00 | 1148.15 | 1148.15 |
| **33** | **1 ug** | 2 | 0 | 2896.31 | 4.00 | 4.00 | 22.63 |
| | **1 µg** | 2 | 28 | 2048.00 | 2048.00 | 724.08 | 512.00 |
| | **4 µg** | 1 | 0 | 1024.00 | 4.00 | 8.00 | 4.00 |
| | **4 µg** | 1 | 28 | 32768.00. | 1024.00 | 4096.00 | 8192.00 |
| **34** | **4 µg** | 1 | 0 | 64.00 | 128.00 | 4.00 | 32.00 |
| | **4 µg** | 1 | 28 | 16384.00 | 8192.00 | 2048.00 | 512.00 |
| **35** | **1 µg** | 2 | 0 | 724.08 | 16.00 | 5.66 | 32.00 |
| | **1 µg** | 2 | 28 | 2896.31 | 362.04 | 724.08 | 181.02 |
| | **4 µg** | 2 | 0 | 90.51 | 5.66 | 11.31 | 4.00 |
| | **4 µg** | 2 | 28 | 5792.62 | 5792.62 | 32.00 | 181.02 |
| **36** | **10 µg** | 2 | 0 | 256.00 | 22.63 | 4.00 | 4.00 |
| | **10 µg** | 2 | 28 | 11585.24 | 512.00 | 5792.62 | 256.00 |
| **37** | **1 µg** | 1 | 0 | 4096.00 | 4.00 | 4.00 | 32.00 |
| | **1 µg** | 1 | 28 | 8192.00 | 2048.00 | 2048.00 | 64.00 |
| | **10 µg** | 1 | 0 | 2048.00 | 4.00 | 8.00 | 4.00 |
| | **10 µg** | 1 | 28 | 16384.00 | 2048.00 | 4096.00 | 64.00 |
| **39** | **1 µg** | 2 | 0 | 512.00 | 4.00 | 45.25 | 4.00 |
| | **1 µg** | 2 | 28 | 5792.62 | 724.08 | 512.00 | 11.31 |
| | **4 µg** | 1 | 0 | 8192.00 | 4.00 | 8.00 | 4.00 |
| | **4 µg** | 1 | 28 | 16384.00 | 2048.00 | 256.00 | 1024.00 |
| **40** | **1 µg** | 1 | 0 | 2048.00 | 4.00 | 4.00 | 4.00 |
| | **1 µg** | 1 | 28 | 8192.00 | 4.00 | 128.00 | 64.00 |
| **41** | **1 µg** | 2 | 0 | 724.08 | 128.00 | 4.00 | 11.31 |
| | **1 µg** | 2 | 28 | 2896.31 | 362.04 | 128.00 | 128.00 |
| **42** | **4 µg** | 2 | 0 | 362.04 | 45.25 | 22.63 | 128.00 |
| | **4 µg** | 2 | 28 | 2048.00 | 4096.00 | 2896.31 | 724.08 |
| | **10 µg** | 1 | 0 | 128.00 | 4.00 | 4.00 | 8.00 |
| | **10 µg** | 1 | 28 | 8192.00 | 2048.00 | 512.00 | 512.00 |
| **44** | **1 µg** | 1 | 0 | 8.00 | 32.00 | 4.00 | 4.00 |
| | **1 µg** | 1 | 28 | 2048.00 | 512.00 | 4.00 | 8.00 |
| | **4 µg** | 1 | 0 | 512.00 | 4.00 | 16.00 | 4.00 |
| | **4 µg** | 1 | 28 | 2048.00 | 512.00 | 2048.00 | 8.00 |
| **45** | **4 µg** | 2 | 0 | 2048.00 | 4.00 | 5.66 | 4.00 |
| | **4 µg** | 2 | 28 | 2896.31 | 128.00 | 4.00 | 16.00 |
| | **10 µg** | 2 | 0 | 724.08 | 45.25 | 128.00 | 11.31 |
| | **10 µg** | 2 | 28 | 2896.31 | 724.08 | 2896.31 | 181.02 |
| **46** | **1 µg** | 1 | 0 | 2048.00 | 128.00 | 4.00 | 4.00 |
| | **1 µg** | 1 | 28 | 2048.00 | 256.00 | 4.00 | 4.00 |
| **47** | **10 µg** | 1 | 0 | 8.00 | 4.00 | 4.00 | 16.00 |
| | **10 µg** | 1 | 28 | 1024.00 | 128.00 | 512.00 | 4096.00 |
| **48** | **1 µg** | 1 | 0 | 128.00 | 512.00 | 4.00 | 16.00 |
| | **1 µg** | 1 | 28 | 128.00 | 2048.00 | 512.00 | 128.00 |
| **49** | **10 µg** | 1 | 0 | 32.00 | 512.00 | 4.00 | 4.00 |
| | **10 µg** | 1 | 28 | 8192.00 | 16384.00 | 4096.00 | 64.00 |
| **52** | **10 µg** | 1 | 0 | 512.00 | 4.00 | 4.00 | 16.00 |
| | **10 µg** | 1 | 28 | 32768.00 | 256.00 | 1024.00 | 32.00 |
| **54** | **1 µg** | 1 | 0 | 512.00 | 4.00 | 4.00 | 16.00 |
| | **1 µg** | 1 | 28 | 256.00 | 4.00 | 32.00 | 64.00 |

### Stage II Dosage Study in Toddlers Aged 12 Months to 22 Months

This clinical trial is an unblinded, open-label, dose-escalation trial of three dosage levels of TetraMenD vaccine, which is administered to participants in three age groups. In Stage II, thirty healthy children (12 to 22 months of age) receive two injections of a single dosage level of TetraMenD vaccine.

For toddler participants, serum specimens for serologic analysis are obtained at three timepoints: at baseline (day 0) prior to TetraMenD injection #1, at day 60 after enrollment (60 days after injection #1 and immediately prior to TetraMenD injection #2), and at day 90 after enrollment (30 days after injection #2). All available specimens are analyzed for SBA against meningococcal polysaccharide serogroups A, C, Y, and W-135, and by ELISA for IgG antibody against these same serogroups. The SBA and IgG ELISA findings for all serogroups are summarized below. The results are summarized in the following Tables.

| **Table A-6: Stage II (Toddlers) - Distribution of SBA Titers at Baseline (Day 0) by TetraMenD Dosage Level (Per-Protocol Population)** | | | | | | | | | |
|---|---|---|---|---|---|---|---|---|---|
| | **n* (%) of Participants <8 with to 256 Titer Result^{§}** | | | | | | | | |
| **Serogroup & dosage level** | **N_{DO}^{†}** | | | | | | | | |
| | | **<8** | **8** | **16** | **32** | **64** | **128** | **256** | |
| **SBA (A)** | | | | | | | | | |
| **1 µg** | 9 | 4 | 2 | ― | ― | 1 | ― | 1 | |
| | | (44.4) | (22.2) | | | (11.1) | | (11.1) | |
| **4 µg** | 8 | 7 | ― | ― | ― | ― | ― | ― | |
| | | (87.5) | | | | | | | |
| **10 µg** | 10 | 8 | ― | ― | 1 | ― | ― | ― | |
| | | (80.0) | | | (10.0) | | | | |
| **SBA (C)** | | | | | | | | | |
| **1 µg** | 9 | 9 | ― | ― | ― | ― | ― | ― | |
| | | (100.0) | | | | | | | |
| **4 µg** | 8 | 7 | ― | ― | ― | ― | ― | 1 | |
| | | (87.5) | | | | | | (12.5) | |
| **10 µg** | 10 | 7 | ― | 1 | ― | 2 | ― | ― | |
| | | (70.0) | | (10.0) | | 20.0) | | | |
| **SBA (Y)** | | | | | | | | | |
| **1 µg** | 9 | 4 | 1 | 1 | 2 | 1 | ― | ― | |
| | | (44.4) | (11.1) | (11.1) | (22.2) | (11.1) | | | |
| **4 µg** | 8 | 5 | 2 | 1 | ― | ― | ― | ― | |
| | | (62.5) | (25.0) | (12.5) | | | | | |
| **10 µg** | 10 | 8 | 1 | ― | ― | 1 | ― | ― | |
| | | (80.0) | (10.0) | | | (10.0) | | | |
| **SBA (W-135)** | | | | | | | | | |
| **1 µg** | 9 | 7 | ― | ― | ― | 1 | ― | — | |
| | | (77.8) | | | | (11.1) | | | |
| **4 µg** | 8 | 7 | ― | ― | ― | ― | 1 | ― | |
| | | (87.5) | | | | | (12.5) | | |
| **10 µg** | 10 | 8 | ― | ― | 1 | ― | 1 | — | |
| | | (80.0) | | | (10.0) | | (10.0) | | |

| | **n* (%) of Participants with Titer Result^{§} 512 to 65536** | | | | | | | | |
|---|---|---|---|---|---|---|---|---|---|
| **Serogroup & dosage level** | **N_{DO}^{†}** | | | | | | | | |
| | | **512** | **1024** | **2048** | **4096** | **8192** | **16384** | **32768** | **65536** |
| **SBA (A)** | | | | | | | | | |
| **1 µg** | 9 | 1 | ― | ― | ― | ― | ― | ― | ― |
| | | (11.1) | | | | | | | |
| **4 µg** | 8 | ― | ― | 1 | ― | ― | ― | ― | ― |
| | | | | (12.5) | | | | | |
| **10 µg** | 10 | ― | ― | 1 | ― | ― | ― | ― | ― |
| | | | | (10.0) | | | | | |
| **SBA (C)** | | | | | | | | | |
| **1 µg** | 9 | ― | ― | ― | ― | ― | ― | ― | ― |
| **4 µg** | 8 | ― | ― | ― | ― | ― | ― | ― | ― |
| **10 µg** | 10 | ― | ― | ― | ― | ― | — | ― | ― |
| **SBA (Y)** | | | | | | | | | |
| **1 µg** | 9 | ― | ― | ― | ― | ― | ― | ― | ― |
| **4 µg** | 8 | ― | ― | ― | ― | ― | ― | ― | ― |
| **10 µg** | 10 | ― | ― | ― | ― | ― | ― | ― | ― |
| **SBA (W-135)** | | | | | | | | | |
| **1 µg** | 9 | 1 | ― | ― | ― | ― | ― | ― | ― |
| | | (11.1) | | | | | | | |
| **4 µg** | 8 | ― | ― | ― | ― | ― | ― | ― | ― |
| **10 µg** | 10 | ― | ― | ― | ― | ― | ― | ― | ― |

| **Table A-7: Stage II (Toddlers) - Distribution of SBA Titers 60 Days After the 1st Injection (Day 60), by TetraMenD Dosage Level (Per-Protocol Population)** | | | | | | | | | |
|---|---|---|---|---|---|---|---|---|---|
| | **n* (%) of Participants with Titer Result^{§} <8 to 256** | | | | | | | | |
| **Serogroup & dosage level** | **N_{D60}^{†}** | | | | | | | | |
| | | **<8** | **8** | **16** | **32** | **64** | **128** | **256** | |
| **SBA (A)** | | | | | | | | | |
| **1 µg** | 9 | ― | ― | ― | ― | 1 | 1 | 1 | |
| | | | | | | (11.1) | (11.1) | (11.1) | |
| **4 µg** | 8 | 1 | ― | ― | ― | ― | ― | ― | |
| | | (12.5) | | | | | | | |
| **10 µg** | 10 | ― | ― | ― | ― | ― | ― | 1 | |
| | | | | | | | | (10.0) | |
| **SBA (C)** | | | | | | | | | |
| **1 µg** | 9 | 3 | 1 | ― | 1 | 2 | 1 | ― | |
| | | (33.3) | (11.1) | | (11.1) | (22.2) | (11.1) | | |
| **4 µg** | 8 | ― | 1 | ― | ― | 2 | 2 | 1 | |
| | | | (12.5) | | | (25.0) | (25.0) | (12.5) | |
| **10 µg** | 10 | 2 | ― | ― | 1 | 1 | 2 | 1 | |
| | | (20.0) | | | (10.0) | (10.0) | 20.0) | (10.0) | |
| **SBA (Y)** | | | | | | | | | |
| **1 µg** | 9 | 2 | ― | 1 | 2 | 1 | 2 | 1 | |
| | | (22.2) | | (11.1) | (22.2) | (11.1) | (22.2) | (11.1) | |
| **4 µg** | 8 | 1 | ― | 1 | ― | 1 | 3 | ― | |
| | | (12.5) | | (12.5) | | (12.5) | (37.5) | | |
| **10 µg** | 10 | 4 | ― | 2 | 1 | 3 | ― | ― | |
| | | (40.0) | | (20.0) | (10.0) | (30.0) | | | |
| **SBA (W-135)** | | | | | | | | | |
| **1 µg** | 9 | 5 | ― | ― | ― | 3 | ― | ― | |
| | | (55.6) | | | | (33.3) | | | |
| **4 µg** | 8 | 5 | ― | ― | ― | ― | 1 | 1 | |
| | | (62.5) | | | | | (12.5) | (12.5) | |
| **10 µg** | 10 | 2 | ― | ― | 1 | 3 | ― | ― | |
| | | (20.0) | | | (10.0) | (30.0) | | | |

| | **n* (%) of Participants with Titer Result^{§} 512 to 65536** | | | | | | | | |
|---|---|---|---|---|---|---|---|---|---|
| **Serogroup & dosage level** | **N_{D60}^{†}** | | | | | | | | |
| | | **512** | **1024** | **2048** | **4096** | **8192** | **16384** | **32768** | **65536** |
| **SBA (A)** | | | | | | | | | |
| **1 µg** | 9 | 2 | ― | 4 | ― | ― | ― | ― | ― |
| | | (22.2) | | (44.4) | | | | | |
| **4 µg** | 8 | ― | ― | 4 | 2 | 1 | ― | ― | ― |
| | | | | (50.0) | (25.0) | (12.5) | | | |
| **10 µg** | 10 | 2 | ― | 5 | 2 | ― | ― | ― | ― |
| | | (20.0) | | (50.0) | (20.0) | | | | |
| **SBA (C)** | | | | | | | | | |
| **1 µg** | 9 | ― | ― | 1 | ― | ― | ― | ― | ― |
| | | | | (11.1) | | | | | |
| **4 µg** | 8 | 2 | ― | ― | ― | ― | ― | ― | ― |
| | | (25.0) | | | | | | | |
| **10 µg** | 10 | 1 | 2 | ― | ― | ― | ― | ― | ― |
| | | (10.0) | (20.0) | | | | | | |
| **SBA (Y)** | | | | | | | | | |
| **1 µg** | 9 | ― | ― | ― | ― | ― | ― | ― | ― |
| **4 µg** | 8 | ― | 2 | ― | ― | ― | ― | ― | ― |
| | | | (25.0) | | | | | | |
| **10 µg** | 10 | ― | ― | ― | ― | ― | ― | ― | ― |
| **SBA (W-135)** | | | | | | | | | |
| **1 µg** | 9 | ― | ― | 1 | ― | ― | ― | ― | ― |
| | | | | (11.1) | | | | | |
| **4 µg** | 8 | ― | ― | 1 | ― | ― | ― | ― | ― |
| | | | | (12.5) | | | | | |
| **10 µg** | 10 | 3 | — | 1 | ― | ― | ― | ― | ― |
| | | (30.0) | | (10.0) | | | | | |

| **Table A-8:** | **Stage II (Toddlers) - Distribution of SBA Titers 30 Days After the 2nd Injection (Day 90), by TetraMenD Dosage Level (Per-Protocol Population)** | | | | | | | | |
|---|---|---|---|---|---|---|---|---|---|
| | **n*(%) of Participants with Titer Result^{§} <8 to 256** | | | | | | | | |
| **Serogroup & dosage level** | **N_{D90}^{†}** | | | | | | | | |
| | | **<8** | **8** | **16** | **32** | **64** | **128** | **256** | |
| **SBA (A)** | | | | | | | | | |
| **1 µg** | 9 | ― | ― | ― | ― | ― | ― | ― | |
| **4 µg** | 8 | ― | ― | ― | ― | ― | ― | ― | |
| **10 µg** | 10 | ― | ― | ― | ― | ― | ― | ― | |
| **SBA (C)** | | | | | | | | | |
| **1 µg** | 9 | 2 | ― | 1 | ― | 1 | 2 | 1 | |
| | | (22.2) | | (11.1) | | (11.1) | (22.2) | (11.1) | |
| **4 µg** | 8 | ― | ― | ― | ― | 1 | 2 | 1 | |
| | | | | | | (12.5) | (25.0) | (12.5) | |
| **10 µg** | 10 | 3 | 1 | ― | 1 | ― | ― | 2 | |
| | | (30.0) | (10.0) | | (10.0) | | | (20.0) | |
| **SBA (Y)** | | | | | | | | | |
| **1 µg** | 9 | ― | ― | ― | 3 | ― | 2 | 1 | |
| | | | | | (33.3) | | (22.2) | (11.1) | |
| **4 µg** | 8 | ― | ― | ― | 1 | ― | 2 | 1 | |
| | | | | | (12.5) | | (25.0) | (12.5) | |
| **10 µg** | 10 | 1 | ― | 1 | 3 | 2 | 2 | ― | |
| | | (10.0) | | (10.0) | (30.0) | (20.0) | (20.0) | | |
| **SBA (W-135)** | | | | | | | | | |
| **1 µg** | 9 | 1 | 1 | 1 | ― | 1 ) | 2 | ― | |
| | | (11.1) | (11.1) | (11.1) | | (11.1) | (22.2) | | |
| **4 µg** | 8 | ― | ― | ― | ― | 1 | 1 | ― | |
| | | | | | | (12.5) | (12.5) | | |
| **10 µg** | 10 | ― | ― | ― | 1 | 1 | 3 | 1 | |
| | | | | | (10.0) | (10.0) | (30.0) | (10.0) | |

| | **n * (%) of Participants with Titer Result^{§} 512 to 65536** | | | | | | | | |
|---|---|---|---|---|---|---|---|---|---|
| **Serogroup dosage level** | **N_{D90}^{†}** | | | | | | | | |
| | | **512** | **1024** | **2048** | **4096** | **8192** | **16384** | **32768** | **65536** |
| **SBA (A)** | | | | | | | | | |
| **1 µg** | 9 | ― | ― | 8 | ― | 1 | ― | ― | ― |
| | | | | (88.9) | | (11.1) | | | |
| **4 µg** | 8 | ― | ― | 5 | 1 | 2 | ― | ― | ― |
| | | | | (62.5) | (12.5) | (25.0) | | | |
| **10 µg** | 10 | 2 | ― | 4 | 4 | ― | ― | ― | ― |
| | | (20.0) | | (40.0) | (40.0) | | | | |
| **SBA (C)** | | | | | | | | | |
| **1 µg** | 9 | 1 | 1 | ― | ― | ― | ― | ― | ― |
| | | (11.1) | (11.1) | | | | | | |
| **4 µg** | 8 | 2 | 2 | ― | ― | ― | ― | ― | ― |
| | | (25.0) | (25.0) | | | | | | |
| **10 µg** | 10 | ― | 2 | 1 | ― | ― | ― | ― | ― |
| | | | (20.0) | (10.0) | | | | | |
| **SBA (Y)** | | | | | | | | | |
| **1 µg** | 9 | 1 | 1 | 1 | ― | ― | ― | ― | ― |
| | | (11.1) | (11.1) | (11.1) | | | | | |
| **4 µg** | 8 | 2 | 1 | 1 ) | ― | ― | ― | ― | ― |
| | | (25.0) | (12.5) | (12.5) | | | | | |
| **10 µg** | 10 | 1 | ― | ― | ― | ― | ― | ― | ― |
| | | (10.0) | | | | | | | |
| **SBA (W-135)** | | | | | | | | | |
| **1 µg** | 9 | ― | 2 | 1 | — | ― | ― | ― | ― |
| | | | (22.2) | (11.1) | | | | | |
| **4 µg** | 8 | 4 | 1 | 1 (12.5) | ― | ― | ― | ― | ― |
| | | (50.0) | (12.5) | (12.5) | | | | | |
| **10 µg** | 10 | 1 | 1 | 2 | ― | ― | ― | ― | ― |
| | | (10.0) | (10.0) | (20.0) | | | | | |

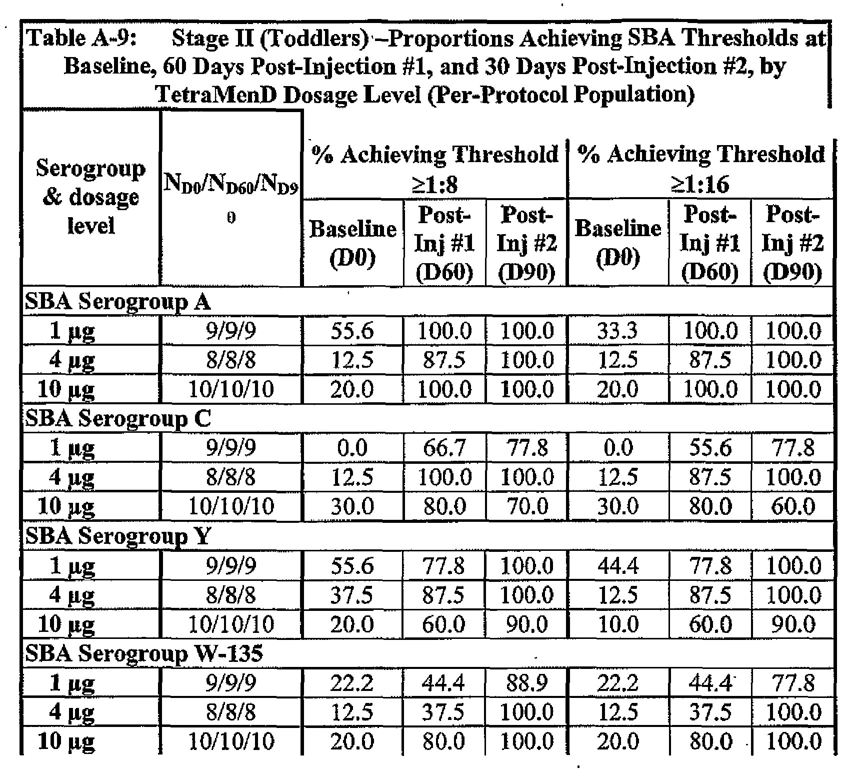

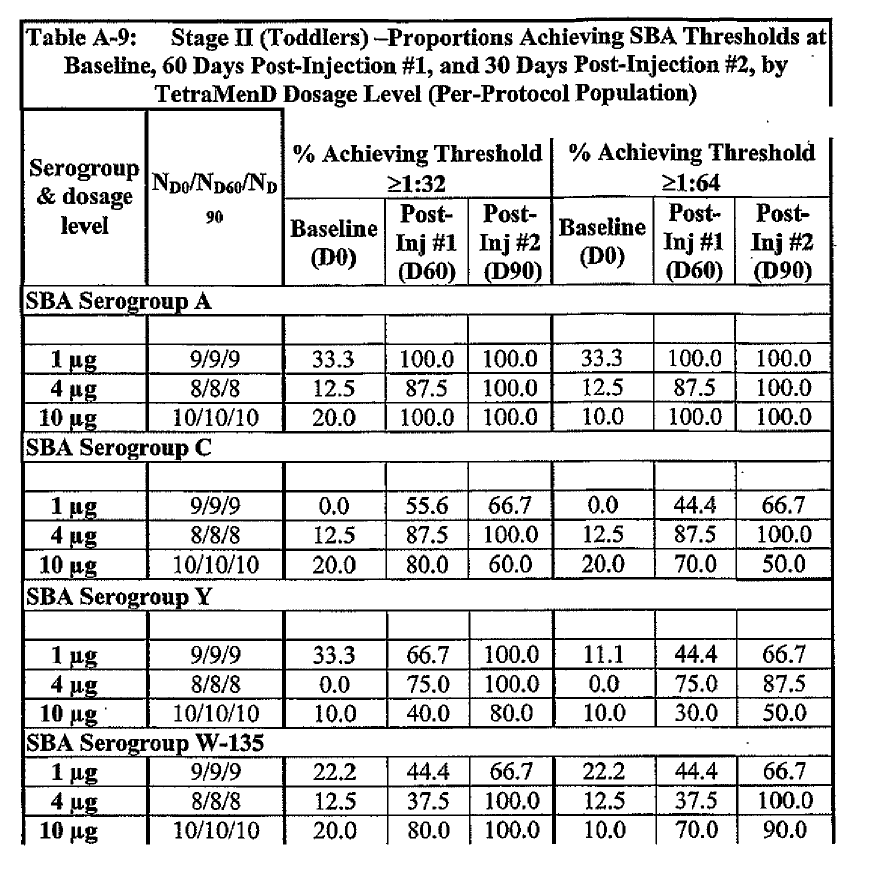

**Table A-10: Stage II (Toddlers) - SBA and IgG ELISA Results Among Toddlers by TetraMenD Dosage Level At Baseline, 60 Days Post-injection #1, and 30 Days Post-injection #2 (Per-Protocol Population)**

| **Serogroup & dosage level** | **N_{D0}N_{D60}N_{D90}** | **GMT/GMC* (95% Cl)** | | | **Mean Fold ↑ (from baseline)** | | **%≥4 Fold ↑ (from baseline)** | |
|---|---|---|---|---|---|---|---|---|
| | | **Baseline (D0)** | **Post-Inj #1 (D60)** | **Post-Inj #2 (D90)** | **Post-Inj #1 (D60)** | **Post-Inj #2 (D90)** | **Post-Inj #1 (D60)** | **Post-Inj #2 (D90)** |
| **SBA (A)** | | | | | | | | |
| **1 µg** | 9/9/9 | 17.3 | 597.3 | 2389.1 | 34.6 | 138.2 | 100.0 | 100.0 |
| | | (3.9-77.0) | (214.5 1663.1) | (1674.8-3407.9) | | | | |
| **4 µg** | 8/8/8 | 8.7 | 1328.0 | 3158.4 | 152.2 | 362.0 | 75.0 | 87.5 |
| | | (1.4-55.1) | (178.7 9870.5) | (1857.4-5370.7) | | | | |
| **10 µg** | 10/10/10 | 9.2 | 1448.2 | 2048.0 | 157.6 | 222.9 | 90.0 | 90.0 |
| | | (2.2-38.7) | (740.0 2833.9) | (1155.2-3630.7) | | | | |
| **SBA (C)** | | | | | | | | |
| **1 µg** | 9/9/9 | 4.0 | 29.6 | 69.1 | 7.4 | 17.3 | 55.6 | 77.8 |
| | | (4.0-4.0) | (5.9 -148.0) | (14.8 -322.6) | | | | |
| **4 µg** | 8/8/8 | 6.7 | 117.4 | 304.4 | 17.4 | 45.3 | 75.0 | 100.0 |
| | | (2.0-23.0) | (37.7-365.3) | (128.5-721.1) | | | | |
| **10 µg** | 10/10/10 | 8.0 | 97.0 | 68.6 | 12.1 | 8.6 | 80.0 | 50.0 |
| | | (3.4-18.6) | (22.9-411.4). | (11.0-428.6) | | | | |
| **SBA(Y)** | | | | | | | | |
| **1 µg** | 9/9/9 | 10.9 | 34.6 | 174.2 | 3.2 | 16.0 | 44.4 | 77.8 |
| | | (4.7-25.4) | (11.0-108.5) | (52.7-575.2) | | | | |
| **4 µg** | 8/8/8 | 5.7 | 98.7 | 304.4 | 17.4 | 53.8 | 87.5 | 100.0 |
| | | (3.7-8.8) | (20.4-478.0) | (100.7-920.1) | | | | |
| **10 µg** | 10/10/10 | 5.7 | 14.9 | 48.5 | 2.6 | 8.6 | 50.0 | 80.0 |
| | | (3.0-10.6) | (6.1-36.3) | (18.9-124.3) | | | | |
| **SBA (W-135)** | | | | | | | | |
| **1 µg** | 9/9/9 | 9.3 | 20.2 | 101.6 | 2.2 | 10.9 | 33.3 | 66.7 |
| | | (2.4― 36.1) | (3.7 108.7) | (18.1― 571.0) | | | | |
| **4 µg** | 8/8/8 | 6.2 | 22.6 | 430.5 | 3.7 | 69.8 | 37.5 | 100.0 |
| | | (2.2― 17.2) | (2.8― 184.9) | (172.2― 1076.3) | | | | |
| **10 µg** | 10/10/10 | 7.0 | 90.5 | 274.4 | 13.0 | 39.4 | 70.0 | 100.0 |
| | | (2.9― 16.6) | (20.2-406.1) | (97.9-769.2) | | | | |
| **IgG ELISA (A)** | | | | | | | | |
| **1 µg** | 9/9/9 | 0.3 | 0.8 | 1.9 | 2.6 | 6.3 | 22.2 | 44.4 |
| | | (0.1-0.7) | (0.3 ― 1.9) | (0.6 ― 6.0) | | | | |
| **4 µg** | 8/8/8 | 0.2 | 2.1 | 4.4 | 12.4 | 26.1 | 87.5 | 100.0 |
| | | (0.1 ― 0.4) | (0.9-4.8) | (2.1 ― 9.1) | | | | |
| **10 µg** | 10/10/10 | 0.2 | 4.4 | 6.2 | 23.4 | 33.1 | 100.0 | 100.0 |
| | | (0.1― 0.3) | (2.9― 6.5) | (4.2― 9.1) | | | | |
| **IgG ELISA (C)** | | | | | | | | |
| **1 µg** | 9/9/9 | 0.1 | 0.3 | 0.5 | 2.6 | 3.8 | 33.3 | 44.4 |
| | | (0.1 ― 0.2) | (0.1 ― 0.9) | (0.2― 1.3) | | | | |
| **4 µg** | 8/8/8 | 0.2 | 1.0 | 1.5 | 5.6 | 8.3 | 75.0 | 87.5 |
| | | (0.0 ― 0.7) | (0.3― 3.1) | (0.6― 3.6) | | | | |
| **10 µg** | 10/10/10 | 0.2 | 0.7 | 1.2 | 4.3 | 7.5 | 60.0 | 70.0 |
| | | (0.1― 0.3) | (0.3 ― 1.5) | (0.7― 2.0) | | | | |
| **IgG ELISA (Y)** | | | | | | | | |
| **1 µg** | 9/9/9 | 0.4 | 0.7 | 1.4 | 1.9 | 3.8 | 11.1 | 33.3 |
| | | (0.2-0.6) | (0.5-1.0) | (0.8-2.4) | | | | |
| **4 µg** | 8/8/8 | 0.3 | 1.2 | 4.5 | 4.4 | 16.4 | 37.5 | 100.0 |
| | | (0.2-0.4) | (0.5- 2.8) | (2.7-7.6) | | | | |
| **10 µg** | 10/10/10 | 0.2 | 0.8 | 1.8 | 4.3 | 10.2 | 70.0 | 90.0 |
| | | (0.1-0.3) | (0.4-1.3) | (0.7-4.5) | | | | |
| **IgG ELISA (W-135)** | | | | | | | | |
| **1 µg** | 9/9/9 | 0.2 | 0.3 | 0.8 | 1.9 | 5.2 | 22.2 | 55.6 |
| | | (0.1-0.3) | (0.2-0.5) | (0.4 -1.6) | | | | |
| **4 µg** | 8/8/8 | 0.1 | 0.6 | 1.5 | 5.3 | 12.7 | 62.5 | 100.0 |
| | | (0.1-0.2) | (0.2-1.9) | (0.8 -3.1) | | | | |
| **10 µg** | 10/10/10 | 0.1 | 0.5 | 1.3 | 4.5 | 11.8 | 60.0 | 80.0 |
| | | (0.1-0.2) | (0.3― 0.9) | (0.8―2.2) | | | | |

| | | | | | | | | |
|---|---|---|---|---|---|---|---|---|
| Day 0: Baseline blood sample drawn prior to injection #1. Day 60: Blood sample drawn 60 days following injection #1 and prior to injection #2. Day 90: Blood sample drawn 30 days following injection #2. % ≥ 4-fold rise: Post-Inj #1: the percentage of toddlers which had a ≥ 4-fold rise in GMT at day 60 in comparison to day 0; Post-Inj #2: the percentage of toddlers which had a ≥ 4-fold rise in GMT at day 90 in comparison to day 0. N: number of evaluable participants *GMTs are computed for the SBA data; GMCs are computed for the IgG ELISA data. | | | | | | | | |

**Table A-11. summarizes the GMT by Dose, Patient Age and Serogroup**

| **Table A-11 Summary of GMT by Patient Age and Serogroup** | | | | | | | |
|---|---|---|---|---|---|---|---|
| **Age** | **Dose TetraMenD** | **No. of Subjects** | **Blood Day** | **A GMT** | **C GMT** | **W GMT** | **Y GMT** |
| **12** | **4 µg** | 4 | 0 | 13.45 | 4.00 | 4.00 | 4.00 |
| | **4 µg** | 4 | 60 | 1448.15 | 107.63 | 32.00 | 53.82 |
| | **4 µg** | 4 | 90 | 4096.00 | 215.27 | 724.08 | 304.44 |
| | **10 µg** | 6 | 0 | 16.00 | 12.70 | 10.08 | 4.00 |
| | **10 µg** | 6 | 60 | 1448.15 | 64.00 | 101.59 | 12.70 |
| | **10 µg** | 6 | 90 | 2298.80 | 80.63 | 256.00 | 40.32 |
| **13** | **4 µg** | 1 | 0 | 4.00 | 4.00 | 4.00 | 16.00 |
| | **4 µg** | 1 | 60 | 4.00 | 128.00 | 4.00 | 128.00 |
| | **4 µg** | 1 | 90 | 2048.00 | 64.00 | 512.00 | 128.00 |
| | **10 µg** | 1 | 0 | 4.00 | 4.00 | 4.00 | 64.00 |
| | **10 µg** | 1 | 60 | 4096.00 | 256.00 | 512.00 | 4.00 |
| | **10 µg** | 1 | 90 | 4096.00 | 1024.00 | 1024.00 | 16.00 |
| **14** | **10 µg** | 2 | 0 | 4.00 | 4.00 | 4.00 | 5.66 |
| | **10 µg** | 2 | 60 | 724.08 | 181.02 | 128.00 | 32.00 |
| | **10 µg** | 2 | 90 | 1024.00 | 5.66 | 512.00 | 128.00 |
| **15** | **4 µg** | 2 | 0 | 90.51 | 4.00 | 4.00 | 5.66 |
| | **4 µg** | 2 | 60 | 2896.31 | 45.25 | 32.00 | 256.00 |
| | **4 µg** | 2 | 90 | 2896.31 | 362.04 | 181.02 | 362.04 |
| **16** | **4 µg** | 2 | 0 | 4.00 | 4.00 | 4.00 | 4.00 |
| | **4 µg** | 2 | 60 | 2896.31 | 90.51 | 4.00 | 45.25 |
| | **4 µg** | 2 | 90 | 4096.00 | 256.00 | 256.00 | 128.00 |
| **18** | **4 µg** | 1 | 0 | 4.00 | 256.00 | 128.00 | 8.00 |
| | **4 µg** | 1 | 60 | 2048.00 | 512.00 | 2048.00 | 1024.00 |
| | **4 µg** | 1 | 90 | 2048.00 | 1024.00 | 2048.00 | 2048.00 |
| | **10 µg** | 1 | 0 | 4.00 | 4.00 | 4.00 | 4.00 |
| | **10 µg** | 1 | 60 | 2048.00 | 128.00 | 4.00 | 32.00 |
| | **10 µg** | 1 | 90 | 2048.00 | 256.00 | 32.00 | 64.00 |

### Stage III Dosage Study in Infants

This clinical trial is an unblinded, open-label, dose-escalation trial of three dosage levels of TetraMenD vaccine, which is administered to participants in three age groups. In Stage III, ninety healthy infants (6 to 12 weeks of age) receive three injections of a single dosage level of TetraMenD vaccine.

Infant participants received TetraMenD injections at age 2 months (injection #1), at age 4 months (injection #2), and at age 6 months (injection #3). Serum specimens for serologic analysis are obtained at two time points: at age 6 months (2 months following injection #2), and at age 7 months (one month after injection #3). All available specimens are analyzed for SBA against meningococcal polysaccharide serogroups A, C, Y, and W-135, and by ELISA for IgG antibody against these same serogroups. The SBA and IgG ELISA findings for all serogroups are summarized below. The results are summarize in the following

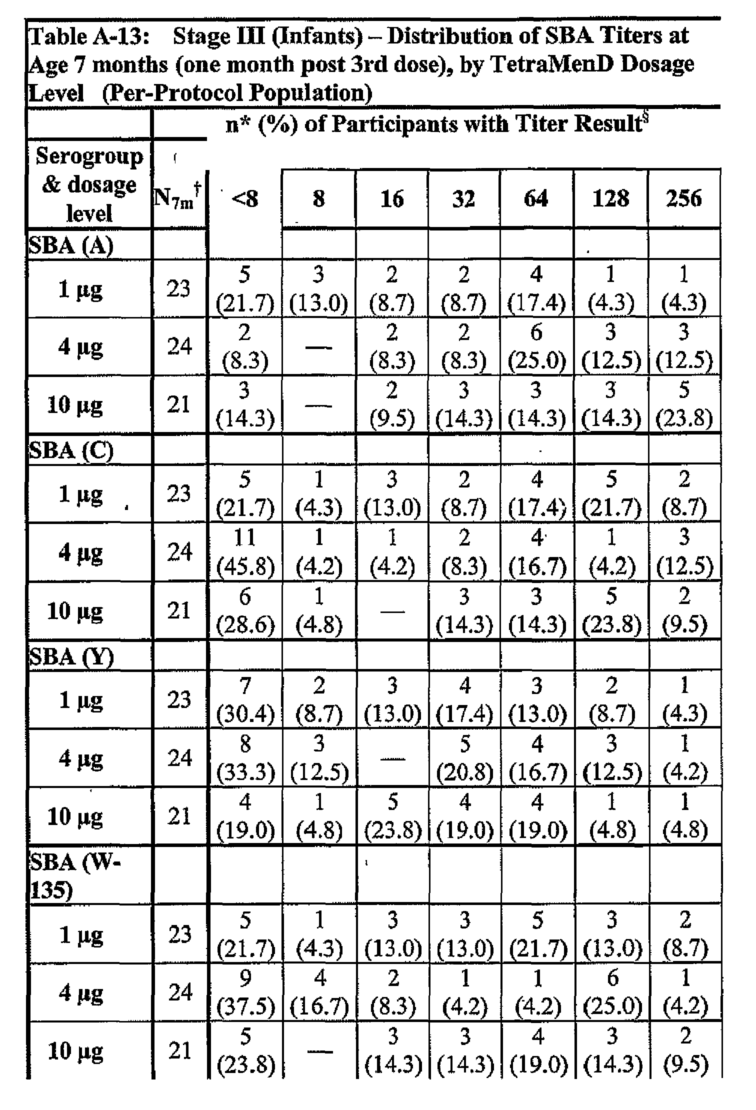

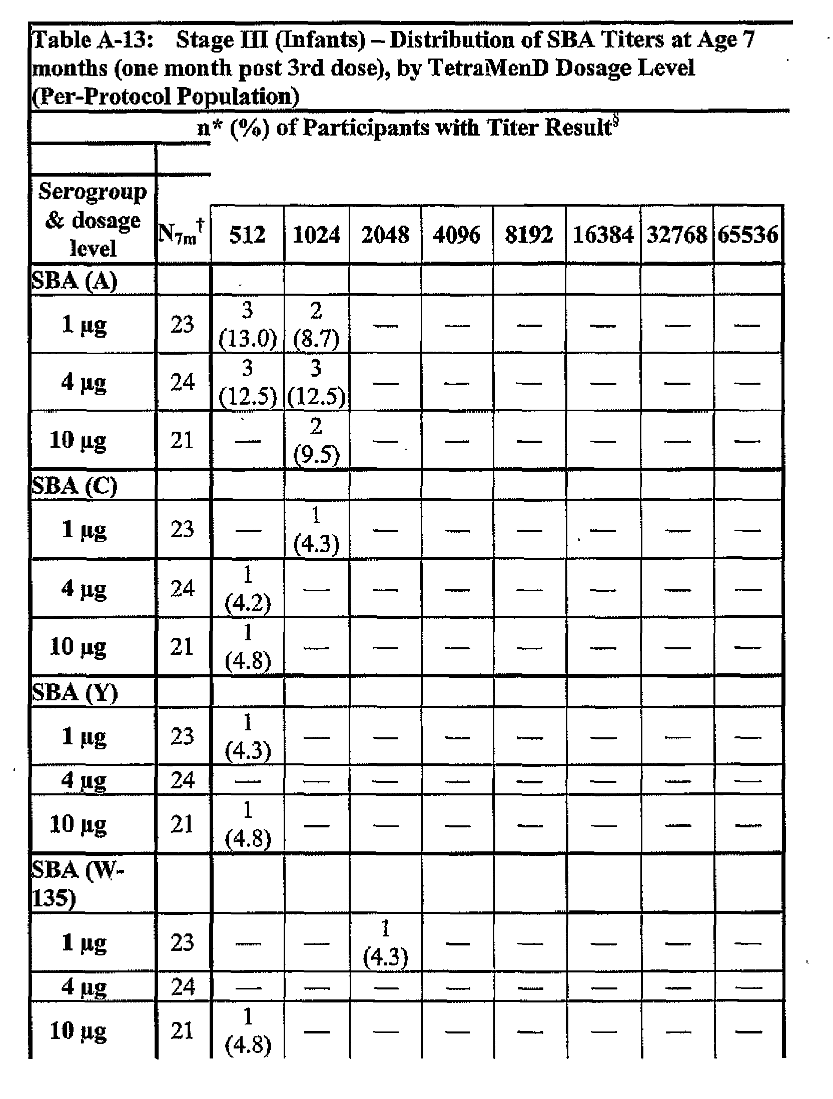

**Table A-14: Stage III (Infants) - Proportions Achieving SBA Thresholds at Age 6 months (pre 3^{rd} dose) and at Age 7 months (post 3^{rd} dose) by TetraMenD Dosage Level (Per-Protocol Population)**

| **Serogroup & dosage level** | **N₆ₘ /N₇ₘ** | **% Achieving Threshold** | | | | | | | |
|---|---|---|---|---|---|---|---|---|---|
| | | **≥1:8** | | **≥1:16** | | **≥1:32** | | **≥1:64** | |
| | | **6 mos** | **7 mos** | **6 mos** | **7 mos** | **6 mos** | **7 mos** | **6 mos** | **7 mos** |
| **SBA (A)** | | | | | | | | | |
| **1 µg** | 22/23 | 54.5 | 78.3 | 36.4 | 65.2 | 27.3 | 56.5 | 13.6. | 47.8 |
| **4 µg** | 23/24 | 69.6 | 91.7 | 69.6 | 91.7 | 56.5 | 83.3 | 30.4 | 75.0 |
| **10 µg** | 21/21 | 85.7 | 85.7 | 66.7 | 85.7 | 52.4 | 76.2 | 19.0 | 61.9 |
| **SBA (C)** | | | | | | | | | |
| **1 µg** | 22/23 | 54.5 | 78.3 | 50.0 | 73.9 | 45.5 | 60.9 | 40.9 | 52.2 |
| **4 µg** | 23/24 | 60.9 | 54.2 | 52.2 | 50.0 | 47.8 | 45.8 | 43.5 | 37.5 |
| **10 µg** | 21/21 | 85.7 | 71.4 | 81.0 | 66.7 | 71.4 | 66.7 | 61.9 | 52.4 |
| **SBA(Y)** | | | | | | | | | |
| **1 µg** | 22/23 | 40.9 | 69.6 | 27.3 | 60.9 | 18.2 | 47.8 | 9.1 | 30.4 |
| **4 µg** | 23/24 | 34.8 | 66.7 | 26.1 | 54.2. | 21.7 | 54.2 | 21.7 | 33.3 |
| **10 µg** | 21/21 | 47.6 | 81.0 | 42.9 | 76.2 | 23.8 | 52.4 | 4.8 | 33.3 |
| **SBA (W-135)** | | | | | | | | | |
| **1 µg** | 22/23 | 27.3 | 78.3 | 18.2 | 73.9 | 4.5 | 60.9 | 4.5 | 47.8 |
| **4 µg** | 23/24 | 30.4 | 62.5 | 21.7 | 45.8 | 17.4 | 37.5 | 8.7 | 33.3 |
| **10 µg** | 21/21 | 42.9 | 76.2 | 38.1 | 76.2 | 23.8 | 61.9 | 19.0 | 47_{.}6 |

| | | | | | | | | | |
|---|---|---|---|---|---|---|---|---|---|
| N: number of evaluable participants at each time point (6 months of age; 7 months of age) | | | | | | | | | |

**Table A-15: Stage III (Infants) - SBA and IgG ELISA Results Among Infants At Age 6 months (pre 3^{rd} dose) and at Age 7 months (post 3^{rd} dose), by TetraMenD Dosage Level (Per-Protocol Population)**

| **Serogroup & dosage level** | **N₆ₘ/N₇ₘ** | **GMT/GMC* (95% CI)** | |
|---|---|---|---|
| | | **Age 6 mos** | **Age 7 mos** |
| **SBA (A)** | | | |
| **1 µg** | 22/23 | 11.3 | 40.7 |
| | | (6.2-20.6) | (17.6-94.0) |
| **4 µg** | 23/24 | 25.1 | 101.6 |
| | | (12.9-49.0) | (51.9-199.0) |
| **10 µg** | 21/21 | 18.9 | 68.4 |
| | | (2.2-29.1) | (32.2-145.1) |
| **SBA(C)** | | | |
| **1 µg** | 22/23 | 19.3 | 37.2 |
| | | (9.3-40.1) | (18.6-74.5) |
| **4 µg** | 23/24 | 24.4 | 19.6 |
| | | (11.0-54.1) | (9.4-40.6) |
| **10 µg** | 21/21 | 43.1 | 35.3 |
| | | (23.2-80.0) | (16.6-75.2) |
| **SBA (Y)** | | | |
| **1 µg** | 22/23 | 7.8 | 21.0 |
| | | (5.0-11.9) | (11.0-40.0) |
| **4 µg** | 23/24 | 8.5 | 19.6 |
| | | (5.0-14.4) | (10.7-35.7) |
| **10 µg** | 21/21 | 9.1 | 26.3 |
| | | (5.9-14.2) | (14.2-48.6) |
| **SBA (W-135)** | | | |
| **1 µg** | 22/23 | 5.8 | 35.0 |
| | | (4.2-8.1) | (17.3-71.1) |
| **4 µg** | 23/24 | 6.9 | 17.4 |
| | | (4.6-10.4) | (9.1-33.5) |
| **10 µg** | 21/21 | 9.8 | 34.2 |
| | | (5.6-16.9) | (17.0-68.7) |
| **IgG ELISA (A)** | | | |
| **1 µg** | 22/22 | 0.5 | 0.5 |
| | | (0.4-0.6) | (0.4-0.6) |
| **4 µg** | 21/21 | 0.7 | 0.8 |
| | | (0.4-1.3) | (0.5-1.3) |
| **10 µg** | 19/19 | 1.3 | 1.3 |
| | | (1.0-1.8) | (0.8-2.1) |
| **IgG ELISA (C)** | | | |
| **1 µg** | 21/21 | 0.5 | 0.7 |
| | | (0.4-0.8) | (0.5-0.9) |
| **4 µg** | 21/21 | 0.4 | 0.8 |
| | | (0.3-0.7) | (0.5-1.1) |
| **10 µg** | 19/19 | 1.1 | 1.2 |
| | | (0.8-1.6) | (0.7-2.0) |
| **IgG ELISA (Y)** | | | |
| **1 µg** | 20/20 | 0.5 | 1.2 |
| | | (0.3-0.8) | (0.8-1.6) |
| **4 µg** | 20/21 | 0.7 | 1.0 |
| | | (0.4-1.2) | (0.6-1.8) |
| **10 µg** | 18/19 | 1.2 | 1.8 |
| | | (0.8-1.8) | (1.1-3.1) |
| **IgG ELISA (W-135)** | | | |
| **1 µg** | 20/20 | 0.5 | 1.1 |
| | | (0.3-0.8) | (0.8-1.6) |
| **4 µg** | 20/21 | 0.5 | 0.9 |
| | | (0.3-0.9) | (0.6-1.3) |
| **10 µg** | 18/19 | 0.9 | 1.5 |
| | | (0.7-1.3) | (0.8-2.5) |

| | | | |
|---|---|---|---|
| N: number of evaluable participants *GMTs are computed for the SBA data; GMCs are computed for the IgG ELISA data. | | | |

**Table A-16 presents a summary of GMT by patient age and serogroup**

| **Table A-16 Summary of GMT by Patient Age and Serogroup** | | | | | | | |
|---|---|---|---|---|---|---|---|
| **Age (weeks)** | **Dose TetraMen D** | **No. of Subjects** | **Blood Day** | **A GMT** | **C GMT** | **W GMT** | **Y GMT** |
| **7** | **4 µg** | 1 | 6-12 weeks | 512.00 | 128.00 | 4.00 | 4.00 |
| | **4 µg** | 1 | 6 month | 512.00 | 128.00 | 4.00 | 32.00 |
| **8** | **1 µg** | 1 | 6-12 weeks | 4.00 | 4.00 | 4.00 | 4.00 |
| | **1 µg** | 1 | 6 month | 32.00 | 16.00 | 16.00 | 4.00 |
| | **4 µg** | 6 | 6-12 weeks | 11.31 | 7.13 | 5.66 | 4.49 |
| | **4 µg** | 6 | 6 month | 71.84 | 7.13 | 22.63 | 6.35 |
| | **10 µg** | 1 | 6-12 weeks | 9.51 | 19.03 | 5.66 | 9.51 |
| | **10 µg** | 1 | 6 month | 32.00 | 38.05 | 19.03 | 32.00 |
| **9** | **1 µg** | 13 | 6-12 weeks | 11.02 | 18.78 | 5.51 | 7.58 |
| | **1 µg** | 14 | 6 month | 55.17 | 55.17 | 40.99 | 22.63 |
| | **4 µg** | 13 | 6-12 weeks | 30.34 | 27.27 | 7.19 | 14.38 |
| | **4 µg** | 13 | 6 month | 60.66 | 19.80 | 14.38 | 27.27 |
| | **10 µg** | 1 | 6-12 weeks | 21.53 | 35.33 | 10.25 | 7.61 |
| | **10 µg** | 1 | 6 month | 70.66 | 26.25 | 26.25 | 22.63 |
| **10** | **1 µg** | 10 | 6-12 weeks | 19.70 | 29.86 | 12.13 | 7.46 |
| | **1 µg** | 10 | 6 month | 39.40 | 19.70 | 45.25 | 14.93 |
| | **4 µg** | 1 | 6-12 weeks | 12.13 | 32.00 | 4.00 | 4.00 |
| | **4 µg** | 1 | 6 month | 114.04 | 22.63 | 28.51 | 25.40 |
| | **10 µg** | 1 | 6-12 weeks | 19.50 | 43.07 | 8.00 | 14.49 |
| | **10 µg** | 1 | 6 month | 52.50 | 35.33 | 39.01 | 32.00 |
| **11** | **1 µg** | 1 | 6-12 weeks | 4.00 | 4.00 | 4.00 | 16.00 |
| | **1 µg** | 1 | 6 month | 8.00 | 4.00 | 4.00 | 32.00 |
| | **4 µg** | 1 | 6-12 weeks | 512.00 | 512.00 | 64.00 | 64.00 |
| | **4 µg** | 1 | 6 month | 1024.00 | 256.00 | 128.00 | 128.00 |
| **12** | **4 µg** | 1 | 6-12 weeks | 32.00 | 4.00 | 4.00 | 64.00 |
| | **4 µg** | 1 | 6 month | 1024.00 | 64.00 | 256.00 | 256.00 |
| **13** | **10 µg** | 1 | 6-12 weeks | 512.00 | 45.25 | 64.00 | 22.63 |
| | **10 µg** | 1 | 6 month | 724.08 | 90.51 | 256.00 | 181.02 |

### Pediatric Vaccines Administered Concomitantly with TetraMenD in Infants

Infants currently receive routine pediatric vaccinations per current ACIP recommendation and local practice. In this study, infants receive TetraMenD with pediatric vaccinations. DTacP (Tripedia®) and Hib (ActHIB®) are administered at ages 2, 4, and 6 months. Either IPV or OPV may be given; IPV is administered with the first and second injections of TetraMenD (at ages 2 and 4 months). Hepatitis B vaccine is given per local practice; hepatitis B vaccine is administered at age 2 months to some participants, but not administered to any participant at ages 4 months or 6 months. During the conduct of the infant stage of this trial, RotaShield® became licensed and received an ACIP recommendation for routine use. A single participant received RotaShield® at ages 4 months and 6 months in the context of this trial.

Antibody responses to routinely administered pediatric vaccine antigens are assessed at age 6 months and 7 months. The results are summarized in separate Tables.

Infants participating in this trial received DTacP and PRP vaccines at 2, 4, and 6 months of age; the 7-month blood draw occurred one month after the third injection of these vaccines. For each of these vaccine antigens (diphtheria, tetanus, pertussis FHA, pertussis PT, and PRP), the observed antibody levels do not demonstrate a statistically significant difference among the 3 TetraMenD dosage groups (all p-values >0.05). (See Table A-17)

In the context of this trial, IPV is administered at 2 months and at 4 months of age. The 7-month blood draw occurs three months after the second injection of IPV. For polio type 1 and polio type 2, the observed GMTs, proportions with NA ≥1:4, and proportions with NA ≥1:8 do not demonstrate a statistically significant difference among the 3 TetraMenD dosage groups (all p-values >0.05). At least 95.0% of all 3 TetraMenD dosage groups demonstrate protection against polio types 1 and 2 by proportion with NA ≥1:8. For polio type 3, the GMTs in the 1 µg, 4 µg, and 10 µg groups are 562.7, 164.0, and 113.3, respectively. The difference among the groups in the polio type 3 GMTs is statistically significant (p=0.001, ANOVA). However, all three TetraMenD dosage groups demonstrate protection against polio type 3 by proportion with NA ≥1:8 (100.0% [22/22], 100.0% [21/21], and 94.1% [16/17], respectively). These proportions are not statistically different (p=0.283, Fisher's exact test). Moreover, the observed GMTs for the three polio serotypes are well within published ranges following two doses of IPV at 2 and 4 months of age, the IPV vaccination schedule utilized in this trial.

The 7-month blood draw occurs at a minimum of 5 months after the most recent hepatitis B vaccination. The observed levels of hepatitis B surface antibody by GMT and proportion ≥10 mIU/mL do not demonstrate a statistically significant difference among the 3 TetraMenD dosage groups (both p-values ≥0.649). Notably, no infants in this trial received hepatitis B vaccine at the 6-month visit, which is the earliest recommended age for the third dose of this vaccine. This may explain why the proportions of 7-month-old infants with hepatitis B surface antibody titers ≥10 mIU/mL are consistent with published ranges for detectable antibody following the initial doses of the vaccine, but lower than would have been expected for protective antibody levels following the complete three-vaccination series. The results of this Study are summarized in the following Tables.

**Table A-17: Stage III (Infants) - Immunogenicity of Concomitant Vaccines Among Infants At Age 7 Months, by TetraMenD Dosage Level (Per-Protocol Population)**

| **Antigen & Immunologic Criteria** | **N₇ₘ 1 µg/4 µg/10 µg** | **TetraMenD Dosage Level** | | | **p-value*** |
|---|---|---|---|---|---|
| | | **1 µg** | **4 µg** | **10 µg** | |
| **Diphtheria (IU/mL)** | | | | | |
| **GMT (95% CI)** | 23/23/21 | 0.16 | 0.09 | 0.08 | 0.150 |
| | | (0.10-0.25) | (0.06-0.14) | (0.05-0.15) | |
| **%≥0.01** | | 100.0 | 100.0 | 95.2 | 0.313 |
| **%≥0.10** | | 56.5 | 43.5 | 47.6 | 0.750 |
| **Tetanus (IU/mL)** | | | | | |
| **GMT (95% CI)** | 23/24/21 | 1.52 | 1.26 | 1.23 | 0.618 |
| | | (1.08-2.15) | (0.88-1.78) | (0.888-1.74) | |
| **%≥0.01** | | 100.0 | 100.0 | 100.0 | Not calculable |
| **%≥0.10** | | 100.0 | 100.0 | 100.0 | Not calculable |
| **Pertussis FHA ELISA (EU/mL)** | | | | | |
| **GMC (95% CI)** | 20/20/19 | 81.6 | 69.6 | 63.4 | 0.455 |
| | | (61.4-108.4) | (55.6-87.2) | (43.5-92.3) | |

| **Antigens & Immunologic Criteria** | **N₇ₘ 1 µg/4 µg/10 µg** | **TetraMenD Dosage Level** | | | **p-value*** |
|---|---|---|---|---|---|
| | | **1 µg** | **4 µg** | **10 µg** | |
| **Pertussis PT ELISA (EU/mL)** | | | | | |
| **GMC (95% CI)** | 20/21/19 | 66.4 | 56.5 | 80.0 | 0.441 |
| | | (43.6- 101.2) | (37.2 - 85.8) | (56.7-112.7) | |
| **Pertussis PT CHO (titer)** | | | | | |
| **GMT (95% CI)** | 20/20/16 | 222.9 | 256.0 | 332.0 | 0.476 |
| | | (130.8- 379.6) | (175.2- 374.2) | (200.9- 548.7) | |
| **Polio type 1 (titer)** | | | | | |
| **GMT (95% CI)** | 22/22/20 | 169.9 | 122.1 | 93.7 | 0.350 |
| | | (95.3- 303.0) | (72.6- 205.4) | (46.5- 185.7) | |
| **% NA ≥1:4** | | 100.0 | 100.0 | 100.0 | Not calculable |
| **% NA ≥1:8** | | 100.0 | 95.5 | 95.0 | 0.760 |
| **Polio type 2 (titer)** | | | | | |
| **GMT (95% CI)** | 22/21/18 | 183.9 | 220.7 | 211.2 | 0.893 |
| | | (96.2- 351.4) | (135.9- 358.5) | (107.3- 415.6) | |
| **% NA ≥1:4** | | 100.0 | 100.0 | 100.0 | Not calculable |
| | | 100.0 | 100.0 | 100.0 | Not calculable |
| **Polio type 3 (titer)** | | | | | |
| **GMT (95% CI)** | 22/21/17 | 562.7 | 164.0 | 113.3 | 0.001 ^{†} |
| | | (363.3- 871.8) | (97.8- 274.8) | (44.6- 287.7) | |
| **% NA ≥1:4** | | 100.0 | 100.0 | 94.1 | 0.283 |
| **% NA ≥1:8** | | 100.0 | 100.0 | 94.1 | 0.283 |
| **PRP (µg/mL)** | | | | | |
| **GMT (95% CI)** | 23/22/19 | 4.87 | 4.41 | 3.39 | 0.648 |
| | | (3.04- 7.78) | (2.52- 7.69) | (1.64- 6.99) | |
| **% ≥0.15** | | 100.0 | 100.0 | 100.0 | Not calculable |
| **% ≥1.0 µg/mL** | | 95.7 | 81.8 | 78.9 | 0.209 |

| **Antigen & Immunologic Criteria** | **N₇ₘ 1 µg/4 µg/10 µg** | **TetraMenD Dosage Level** | | | **p-value*** |
|---|---|---|---|---|---|
| | | **1 µg** | **4 µg** | **10 µg** | |
| **Hep B Surface Ab** | | | | | |
| **GMT (95% CI)** | 21/23/19 | 46.9 | 36.9 | 48.3 | 0.916 |
| | | (12.9-170.1) | (12.4-110.2) | (28.1-83.2) | |
| **% ≥10** | | 81.0 | 78.3 | 89.5 | 0.649 |

| | | | | | |
|---|---|---|---|---|---|
| * GMT comparisons use the F-test. Comparisons of percentages use the Fisher's exact test. ^{†} p-value < 0.05 | | | | | |

### Example 11 Study B One and Six Month Study in Children Aged 2 to 10

This is a randomized, active-controlled sutyd of healthy children between the ages of 2 and 10, comparing a single dose of TetraMenD with a single dose of Menomune. Blood specimens are drawn on D0, before vaccination, D28 and at 6-months post D0. The overall safety of TetxaMenD compared with Menomune is comparable. The results of this study are summarized in the following Tables.

### Distribution of SBA-BR Antibody Titers

Table B-1 shows the frequency distribution of baseline, Day 28 and Month 6 SBA-BR antibody titers for each serogroups.

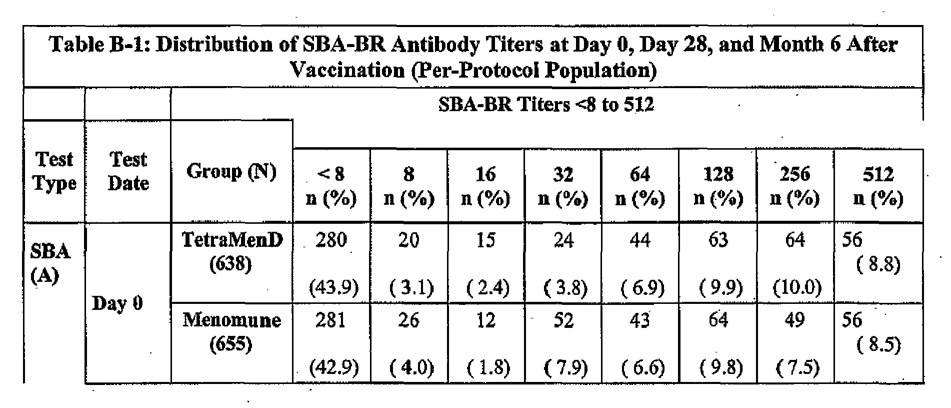

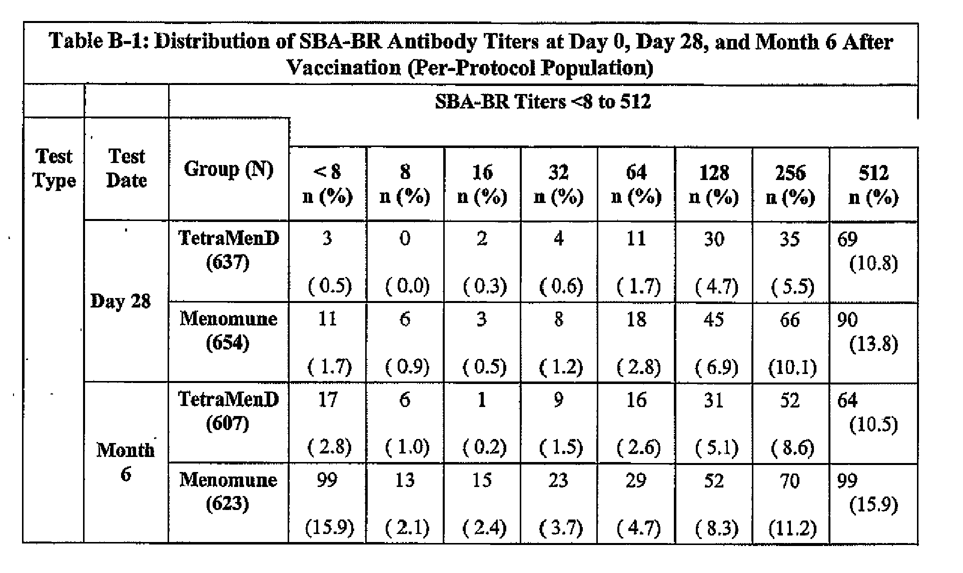

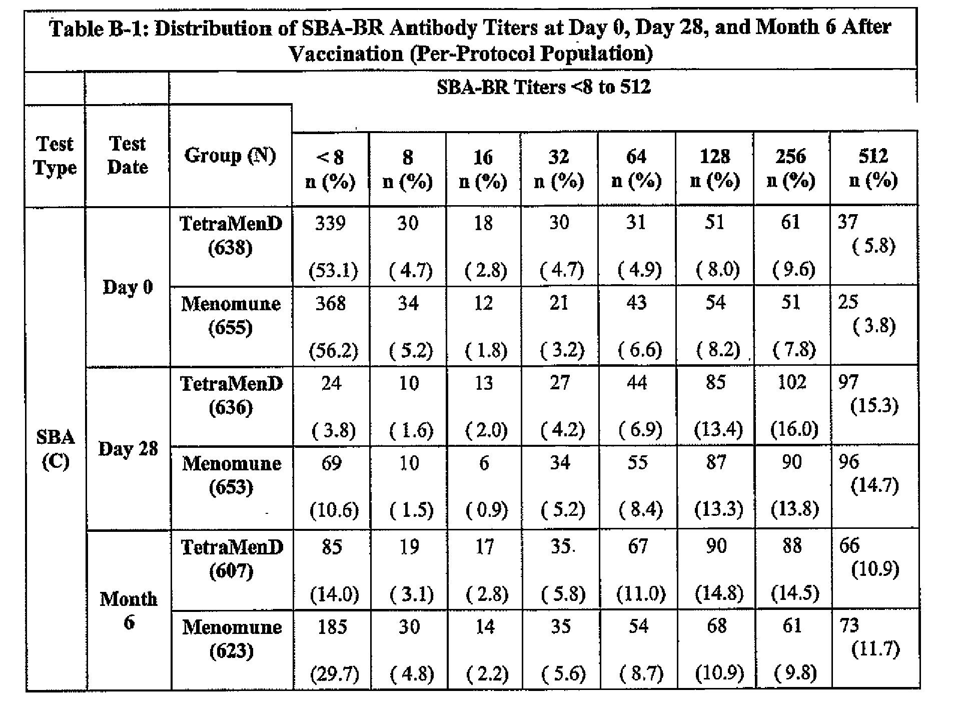

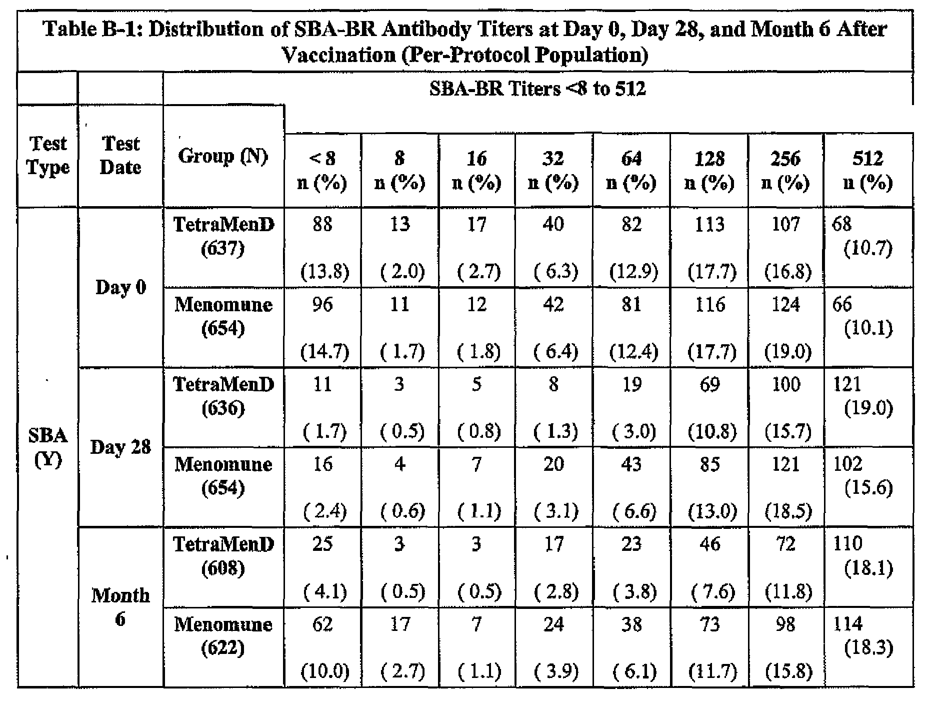

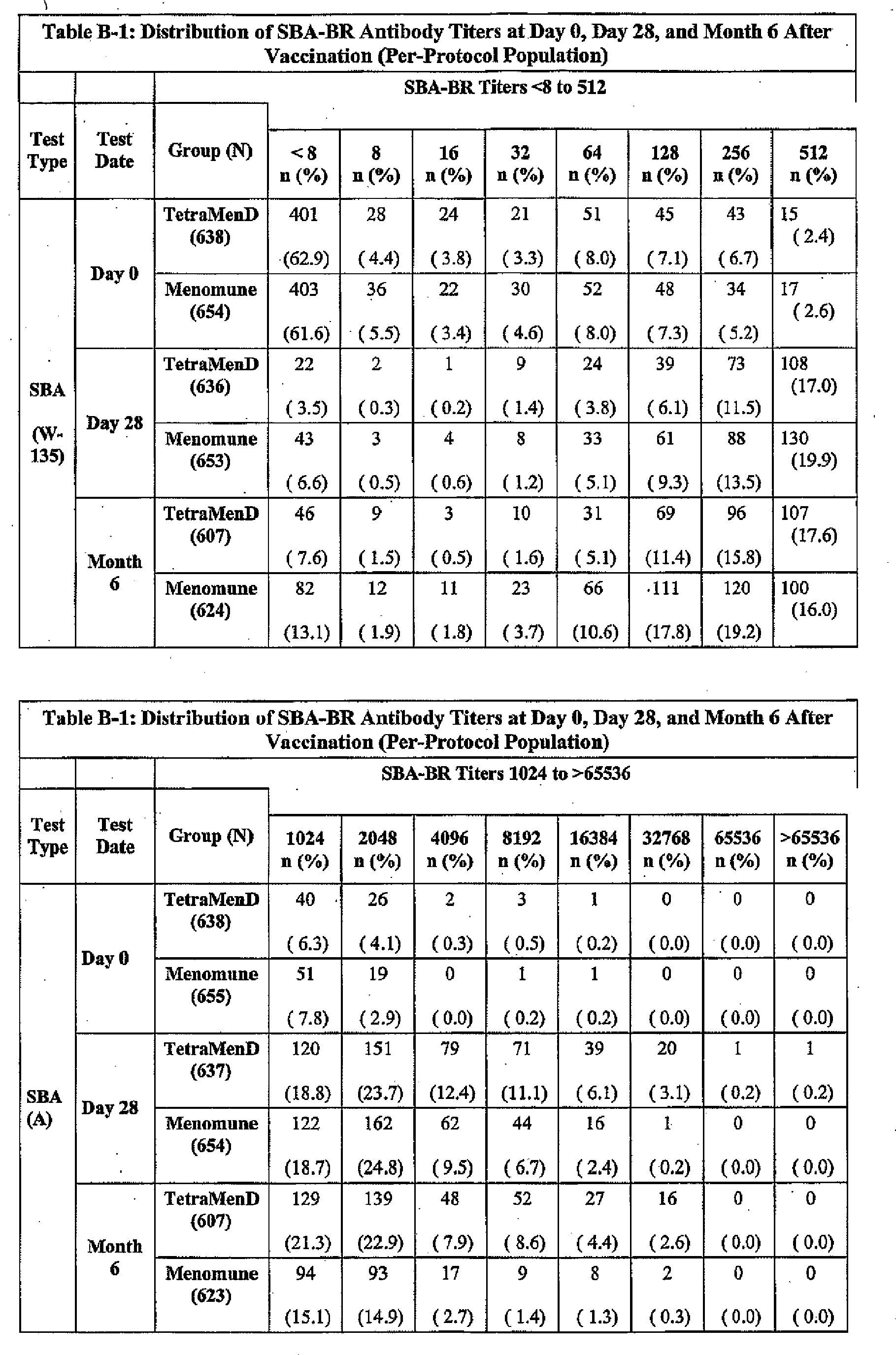

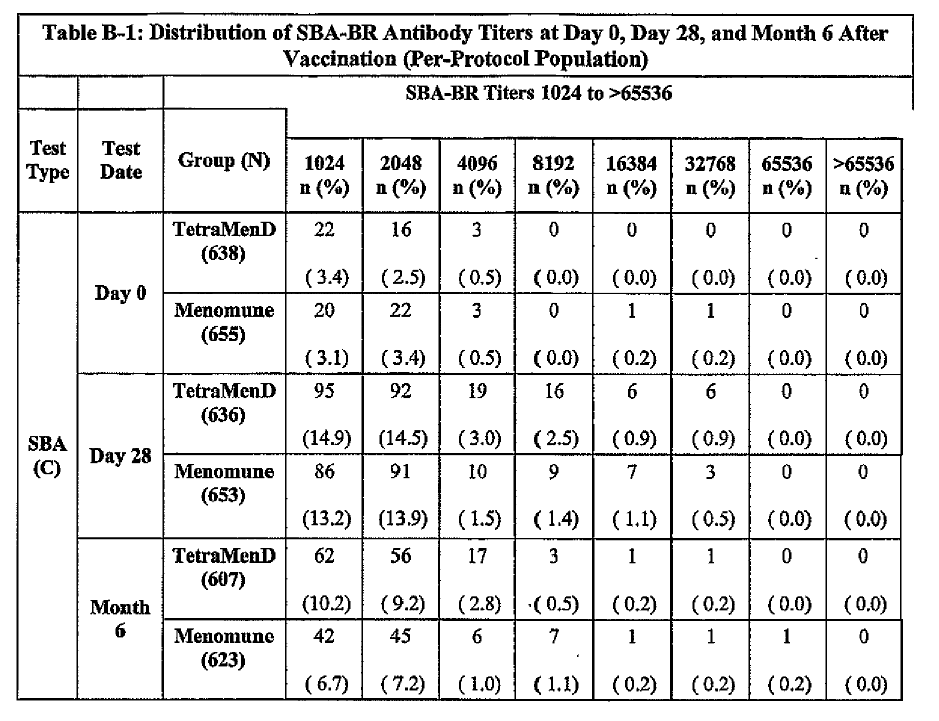

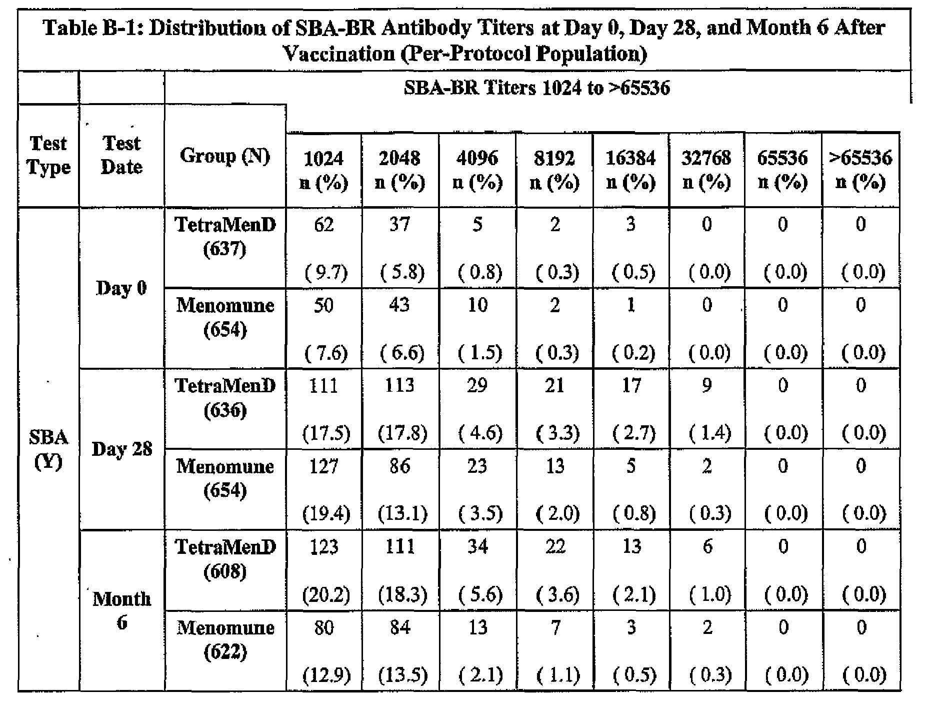

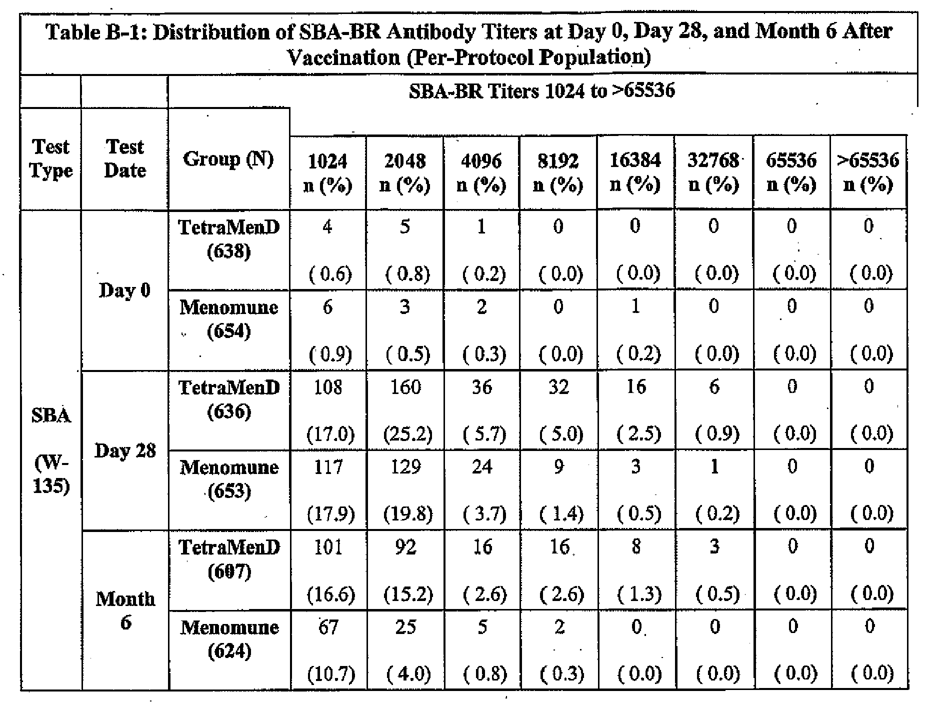

**Table B-2 summarizes of Geometric Mean Titer (GMT) by Subject Age and Serogroup for TetraMenD**

| **Table B-2 Summary of GMT by Subject Age and Serogroup for TetraMenD** | | | | | | |
|---|---|---|---|---|---|---|
| Age (in Year) | Blood Day | No. of Subjects | Serogroup A GMT | Serogroup C GMT | Serogroup W GMT | Serogroup Y-135 GMT |
| 2 | Day 0 | 264 | 20.59 | 16.04 | 8.75 | 91.23 |
| | Day 28 | 260 | 940.26 | 151.41 | 332.43 | 393.88 |
| | 6 Month | 244 | 460.92 | 64.92 | 153.52 | 312.95 |
| 3 | Day 0 | 235 | 33.84 | 14.60 | 12.64 | 148.78 |
| | Day 28 | 228 | 1610.74 | 292.64 | 795.64 | 662.97 |
| | 6 Month | 228 | 1033.38 | 105.69 | 408.85 | 631.50 |
| 4 | Day 0 | 35 | 74.99 | 29.56 | 9.95 | 128.00 |
| | Day 28 | 34 | 3479.60 | 795.86 | 1731.22 | 906.10 |
| | 6 Month | 29 | 3812.58 | 302.62 | 634.88 | 908.65 |
| 5 | Day 0 | 27 | 118.51 | 32.00 | 29.63 | 121.59 |
| | Day 28 | 27 | 3010.02 | 612.79 | 1323.71 | 1050.63 |
| | 6 Month | 27 | 3010.02 | 406.37 | 679.07 | 948.10 |
| 6 | Day 0 | 38 | 38.40 | 34.42 | 13.83 | 112.66 |
| | Day 28 | 37 | 4414.32 | 950.07 | 2086.73 | 898.15 |
| | 6 Month | 37 | 3729.75 | 386.57 | 1005.00 | 849.06 |
| 7 | Day 0 | 30 | 111.43 | 78.79 | 16.00 | 111.43 |
| | Day 28 | 29 | 3999.26 | 1612.60 | 1691.56 | 1537.33 |
| | 6 Month | 28 | 2375.94 | 524.83 | 1049.67 | 1638.97 |
| 8 | Day 0 | 18 | 118.51 | 54.86 | 17.28 | 188.13 |
| | Day 28 | 16 | 6049.08 | 1878.02 | 2543.32 | 1069.34 |
| | 6 Month | 17 | 2837.86 | 453.05 | 1111.00 | 1418.93 |
| 9 | Day 0 | 29 | 116.33 | 91.60 | 21.31 | 87.32 |
| | Day 28 | 28 | 4870.99 | 2825.49 | 3119.59 | 1521.66 |
| | 6 Month | 28 | 3995.85 | 974.54 | 1187.97 | 1412.75 |
| 10 | Day 0 | 21 | 98.30 | 27.13 | 18.26 | 45.25 |
| | Day 28 | 21 | 4233.45 | 3251.00 | 2337.06 | 1333.45 |
| | 6 Month | 20 | 3821.70 | 989.12 | 1782.89 | 1499.22 |

Table B-3shows the numbers and proportions of participants with a ≥4-fold rise in SBA-BR titer from baseline to Day 28 for the serogroups A, C, Y, and W-135. For each serogroup, these percentages are higher in the TetraMenD group than in the Menomune^{®} group. The differences in the proportions are: -0.0397, -0.0452, ―0.1092 and -0.0562, for serogroups A, C, Y, and W-135, respectively.

**Table B-3: Summary of Primary Hypothesis Testing for the Per-Protocol Population**

| **Serogroup** | **≥4-fold rise in SBA titer** | | | | | |
|---|---|---|---|---|---|---|
| | **TetraMenD n/N Pₜ** | | **Menomune**^{®} **n/N Pₘ** | | **Difference (Pₘ - Pₜ)** | **Upper One-sided 95% CL of the Difference** |
| Serogroup A | 558/636 | 0.8774 | 547/653 | 0.8377 | -0.0397 | -0.0077 |
| Serogroup C | 466/635 | 0.7339 | 449/652 | 0.6887 | -0.0452 | -0.0037 |
| Serogroup Y | 359/634 | 0.5662 | 298/652 | 0.4571 | -0.1092 | -0.0636 |
| Serogroup W-135 | 578/635 | 0.9102 | 556/651 | 0.8541 | -0.0562 | -0.0267 |

| | | | | | | |
|---|---|---|---|---|---|---|
| n: number of participants with a ≥ 4-fold rise from baseline titer. N: total number of participants in the used population. Pₜ and Pₘ: proportions of participants with a ≥4-fold rise in SBA post-vacaination titer from the TetraMenD and Menomune^{®} groups, respectively. | | | | | | |

The proportion of participants with SBA antibody titers ≥32 at Day 28 after vaccination is summarized in Table B-3.

| **Table B-3: Percentage and Number of Participants with an SBA-BR Antibody Titer ≥ 32 at Day 28 Post-Vaccination (Per-Protocol Population)** | | | | |
|---|---|---|---|---|
| | **TetraMenD** | | **Menomune**^{®} | |
| | **%^{*} (n^{†}/N^{‡})** | **95% CI for the percentage** | **%^{*} (n^{†}/N^{‡})** | **95% CI for the percentage** |
| **Serogroup A** | 99.22 (632 / 637) | (98.18, 100.00) | 96.94 (634 / 654) | (95.32, 100.00) |
| **Serogroup C** | 92.61 (589/636) | (90.29, 100.00) | 86.98 (568 / 653) | (84.16, 100.00) |
| **Serogroup Y** | 97.01 (617/636) | (95.37,100.00) | 95.87 (627/654) | (94.05, 100.00) |
| **Serogroup W-135** | 96.07 (611/636) | (94.25,100.00) | 92.34 (603/653) | (90.03, 100.00) |

| | | | | |
|---|---|---|---|---|
| ^{*}%: _{n/N.} ^{†}n: number of participants with titer≥32 at Day 28 post-vaccination. ^{‡} N: total number of participants with valid blood sample at Day 28 in this groups. | | | | |

The proportion of participants with SBA antibody titers ≥ 128 at Day 28 after vaccination is summarized in Table B-4.

**Table B-4: Percentage and Number of Participants with an SBA-BR Antibody Titer ≥ 128 at Day 28 Post-Vaccination (Per-Protocol Population)**

| | **TetraMenD** | | **Menomune^{®}** | |
|---|---|---|---|---|
| | **%* (n^{†}/N^{‡})** | **95% CI for the percentage** | **%^{*} (n^{†}/N^{‡})** | **95% CI for the percentage** |
| **Serogroup A** | 96.86 (617/637) | (95.19, 100.00) | 92.97 (608 / 654) | (90.73, 100.00) |
| **Serogroup C** | 81.45 (518/636) | (78.20, 100.00) | 73.35 (479/653) | (69.79, 100.00) |
| **Serogroup Y** | 92.77 (590 / 636) | (90.47, 100.00) | 86.24 (564 / 654) | (83.36,100.00) |
| **Serogroup W-135** | 90.88 (578/636) | (88.37, 100.00) | 86.06 (562/653) | (83.17, 100.00) |

| | **TetraMenD** | | **Menomune**^{®} | |
|---|---|---|---|---|
| | **%^{*} (n^{†}/N^{‡})** | **95% CI for the percentage** | **%^{*}(n^{†}/N^{‡})** | **95% CI for the percentage** |

| | | | | |
|---|---|---|---|---|
| ^{*}%: n/N. ^{†} n: number of participants with titer≥128 at Day 28 post-vaccination. ^{‡} N: total number of participants with valid blood sample at Day 28 in this group. | | | | |

### Proportion of Participants with at least a 4-fold rise in SBA-BR Antibody-Titers

Table B-5 shows the proportion of participants with a ≥4-fold rise in Day 28 and Month 6 SBA antibody titers from baseline. Twenty-eight to 56 days after receiving TetraMenD, the majority of participants experienced a ≥ 4-fold rise in the SBA-BR antibody titer for each of the serogroups contained in the vaccine.

**Table B-5: Percentage and Number of Participants with a ≥4-Fold Rise in Day 28 and Month 6 SBA-BR Antibody Titers From Baseline (Per-Protocol Population)**

| **Test Type** | **Test Date** | **TetraMenD** | | **Menomune**^{®} | |
|---|---|---|---|---|---|
| | | **%^{*} (n^{†}/N^{‡})** | **(95% CI)** | **%^{*} (n^{†}/N^{‡})** | **(95% CI)** |
| **Serogroup A SBA** | **Day 28** | 87.7 | (84.9, 90.2) | 83.8 | (80.7, 86.5). |
| | | (558/636) | | (547/653) | |
| | **Month 6** | 79.1 | (75.6, 82.2) | 56.9 | (52.9, 60.8) |
| | | (480/607) | | (354/622) | |
| **Serogroup C SBA** | **Day 28** | 73.4 | (69.8, 76.8) | 68.9 | (65.2, 72.4) |
| | | (466/635) | | (449/652) | |
| | **Month 6** | 55.7 | (51.6, 59.7) | 43.7 | (39.8, 47.7) |
| | | (338/607) | | (272/622) | |
| **Serogroup Y SBA** | **Day 28** | 56.6 | (52.7, 60.5) | 45.7 | (41.8, 49.6) |
| | | (359/634) | | (298/652) | |
| | **Month 6** | 57.3 | (53.3, 61.3) | 39.2 | (35.3, 43.2) |
| | | (348/607) | | (243/620) | |
| **Serogroup W-** | **Day 28** | 91.0 | (88.5, 93.1) | 85.4 | (82.5, 88.0) |
| **135 SBA** | | (578/635) | | (556/651) | |

| **% ^{*}(n^{†}/N^{‡})** | **(95% CI)** | **%^{*} (n^{†}/N^{‡})** | **(95% CI)** | | |
|---|---|---|---|---|---|
| | **Month 6** | 82.7 | (79.5, 85.6) | 69.5 | (65.7, 73.1) |
| | | (502/607) | | (432/622) | |

| | | | | | |
|---|---|---|---|---|---|
| ^{*}%: n/N. ^{†} n: number of participants with ≥4-fold rise from baseline titer. ^{‡} N: total number of participants in the used population. | | | | | |

### Proportion of Participants with Undetectable Titers (<8) at Day 0 Achieving a ≥4-Fold Rise in Day 28 SBA-BR Antibody Titers

In both treatment groups and for all vaccine serogroups, most participants with an undetectable (<8) SBA-BR titer at baseline achieved a ≥4-fold rise in Day 28 SBA titers. (Table B-6) The proportions of participants with an SBA titer <8 at Day 0 who had a≥4-fold rise from baseline to Day 28 ranged from 86.21% to 98.57% in the TetraMenD group; and from 75.00 to 94.64 in the Menomune^{®} group.

**Table B-6: Number and Percentage of Participants with Undetectable Titers (<8) at Day 0 Achieving a ≥4-Fold Rise in Day 28 SBA-BR Antibody Titers.**

| | **TetraMenD** | | **Menomune**^{®} | |
|---|---|---|---|---|
| **Serogroup** | **% (n/N*)** | **95% CI^{*}** | **% (n/N)** | **95% CI^{†}** |
| **A** | 98.57 | (96.37, 100.00) | 94.64 | (91.32, 100.00) |
| | (275/279) | | (265/280) | |
| **C** | 87.87 | (83.91, 100.00) | 80.05 | (75.59, 100.00) |
| | (297/338) | | (293/366) | |
| **Y** | 86.21 | (77.15, 100.00) | 75.00 | (65.12,100.00) |
| | (75 / 87) | | (72/96) | |
| **W-135** | 96.00 | (93.59, 100.00) | 89.53 | (86.11, 100.00) |
| | (384/400) | | (359/401) | |

| | | | | |
|---|---|---|---|---|
| *n = The number of participants with titers < 8 at Day 0 and titers ≥32 at Day 28 within each serogroup N = The number of participants with titers < 8 at Day 0 within each serogroup ^{†} Exact 95% confidence interval for the percentage | | | | |

### SBA-BR Antibody GMTs and mean fold rises

Table B-7 shows the SBA GMTs at baseline and on Day 28 and Month 6 after vaccination and the fold rises in SBA GMTs.

**Table B-7: SBA-BR Serology Results at Baseline, Day 28, and Month 6 After Vaccination (Per-Protocol Population)**

| **Test Type** | **Parameter^{*}** | **Bleed** | **TetraMenD** | | | **Menomune^{®}** | | |
|---|---|---|---|---|---|---|---|---|
| | | | **N^{†}** | **Geometric Mean** | **(95% CI)** | **N^{†}** | **Geometric Mean** | **(95% CI)** |
| **Serogroup A SBA** | **Titer** | **Day 0** | 638 | 35.44 | (29.77, 42.20) | 655 | 32.72 | (27.71, 38.63) |
| | | **Day 28** | 637 | 1700.27 | (1512.07, 1911.89) | 654 | 892.20 | (789.97, 1007.66) |
| | | **Month 6** | 607 | 1053.65 | (912.93, 1216.07) | 623 | 214.97 | (179.84, 256.97) |
| | **Fold rise** | **Day 0** | 638 | 1.00 | (1.00, 1.00) | 655 | 1.00 | (1.00, 1.00) |
| | | **Day 28** | 636 | 35.18 | (29.72, 41.65) | 653 | 20.21 | (17.43, 23.44) |
| | | **Month 6** | 607 | 23.19 | (19.20, 28.00) | 622 | 5.04 | (4.21, 6.03) |
| **Serogroup C SBA** | **Titer** | **Day 0** | 638 | 20.63 | (17.59, 24.20) | 655 | 18.69 | (15.95, 21.90) |
| | | **Day 28** | 636 | 353.85 | (307.95, 406.58) | 653 | 230.71 | (197.72, 269.20) |
| | | **Month 6** | 607 | 136.92 | (116.40, 161.06) | 623 | 65.51 | (54.64, 78.55) |
| | **Fold rise** | **Day 0** | 638 | 1.00 | (1.00, 1.00) | 655 | 1.00 | (1.00, 1.00) |
| | | **Day 28** | 635 | 11.86 | (10.19, 13.81) | 652 | 8.40 | (7.23, 9.77) |
| | | **Month 6** | 607 | 4.49 | (3.85, 5.25) | 622 | 2.41 | (2.05, 2.83) |
| **Serogroup Y SBA** | **Titer** | **Day 0** | 637 | 118.61 | (102.49, 137.27) | 654 | 117.84 | (101.98, 136.18) |
| | | **Day 28** | 636 | 636.70 | (563.06, 719.97) | 654 | 408.10 | (362.19, 459.84) |
| | | **Month 6** | 608 | 591.77 | (514.65, 680.43) | 622 | 239.18 | (204.91, 279.17) |
| | **Fold rise** | **Day 0** | 637 | 1.00 | (1.00, 1.00) | 654 | 1.00 | (1.00, 1.00) |
| | | **Day 28** | 634 | 4.83 | (4.25, 5.49) | 652 | 3.14 | (2.79, 3.52) |
| | | **Month 6** | 607 | 4.63 | (4.00, 5.37) | 620 | 1.85 | (1.60, 2.14) |
| **Serogroup W-135 SBA** | **Titer** | **Day 0** | 638 | 12.09 | (10.62, 13.76) | 654 | 12.15 | (10.69, 13.80) |
| | | **Day 28** | | 749.78 855.18) | (657.37, | 653 | 424.75 | (371.47, 485.67) |
| | | **Month 6** | 607 | 362.25 | (311.67, 421.03) | 624 | 136.06 | (118.08, 156.78) |
| | **Fold rise** | **Day 0** | 638 | 1.00 | (1.00, 1.00) | 654 | 1.00 | (1.00, 1.00) |
| | | **Day 28** | 635 | 40.24 | (34.30, 47.21) | 651 | 22.98 | (19.73, 26.76) |
| | | **Month 6** | 607 | 19.19 | (16.31, 22.56) | 622 | 7.42 | (6.33, 8.69) |
| **Test Type** | **Parameter^{*}** | **Bleed** | **TetraMenD** | | | **Menomune^{®}** | | |
| | | | **N^{†}** | **Geometric Mean** | **(95% CI)** | **N^{†}** | **Geometric Mean** | **(95% CI)** |

| | | | | | | | | |
|---|---|---|---|---|---|---|---|---|
| Titer or fold rise, where fold rise = titer at Day 28/titer at Day 0 ^{†}N: total number of participants used in the calculation. | | | | | | | | |

### ELISA IgG for serogroups A, C, W-135, and Y

Table B-8 shows the IgG GMCs at baseline and on Day 28 and Month 6 after vaccination and the fold rises in IgG GMCs.

**Table B-8: IgG Serology Results at Baseline, Day 28, and Month 6 After Vaccination (Per-Protocol Population)**

| **Test Type** | **Parame ter^{*}** | **Bleed** | **TetraMenD** | | | **Menomune**^{®} | | |
|---|---|---|---|---|---|---|---|---|
| | | | **N^{†}** | **Geom etric Mean** | **(95% CI)** | **N^{†}** | **Geometric Mean** | **(95% CI)** |
| **Serogroup A (IgG) ELISA** | **Titer** | **Day 0** | 115 | 0.36 | (0.31, 0.43) | 113 | 0.33 | (0.28, 0.38) |
| | | **Day 28** | 115 | 7.65 | (6.27, 9.33) | 110 | 6.81 | (5.51, 8.42) |
| | | **Month 6** | 112 | 1.70 | (1.37, 2.11) | 109 | 4.53 | (3.60, 5.70) |
| | **Fold rise** | **Day 0** | 115 | 1.00 | (1.00, 1.00) | 113 | 1.00 | (1.00, 1.00) |
| | | **Day 28** | 115 | 21.00 | (16.60, 26.58) | 108 | 21.09 | (16.78, 26.50) |
| | | **Month 6** | 112 | 4.58 | (3.65, 5.75) | 107 | 14.40 | (11.22, 18.49) |
| **Serogroup C (IgG) ELISA** | **Titer** | **Day 0** | 115 | 0.23 | (0.20, 0.25) | 113 | 0.25 | (0.22, 0.29) |
| | | **Day 28** | 115 | 1.24 | (1.03, 1.50) | 110 | 7.62 | (6.33, 9.19) |
| | | **Month 6** | 111 | 0.36 | (0.31, 0.43) | 109 | 3.49 | (2.82, 4.32) |
| | **Fold rise** | **Day 0** | 115 | 1.00 | (1.00, 1.00) | 113 | 1.00 | (1.00, 1.00) |
| | | **Day 28** | 115 | 5.50 | (4.58, 6.62) | 109 | 30.18 | (24.26, 37.55) |
| | | **Month 6** | 111 | 1.60 | (1.36, 1.89) | 107 | 14.42 | (11.46, 18.15) |
| **Serogroup Y (IgG) ELISA** | **Titer** | **Day 0** | 115 | 0.38 | (0.34, 0.43) | 114 | 0.34 | (0.31, 0.38) |
| | | **Day 28** | 115 | 1.54 | (1.26, 1.88) | 110 | 4.15 | (3.30, 5.22) |
| | | **Month 6** | 112 | 0.76 | (0.65, 0.89) | 109 | 2.90 | (2.25, 3.73) |
| | **Fold rise** | **Day 0** | 115 | 1.00 | (1.00, 1.00) | 114 | 1.00 | (1,00, 1.00) |
| | | **Day 28** | 115 | 4.04 | (3.30, 4.93) | 109 | 12.43 | (9.85, 15.68) |
| | | **Month 6** | 112 | 1.98 | (1.68, 2.32) | 108 | 8.72 | (6.79, 11.21) |
| **Serogroup W-135 (IgG) ELISA** | **Titer** | **Day 0** | 115 | 0.25 | (0.22, 0.28) | 113 | 0.22 | (0.19, 0.25) |
| | | **Day 28** | 115 | 0.90 | (0.72, 1.12) | 110 | 2.53 | (2.06, 3.11) |
| | | **Month 6** | 112 | 0.55 | (0.47, 0.65) | 109 | 1.88 | (1.53, 2.31) |
| | **Fold rise** | **Day 0** | 115 | 1.00 | (1.00, 1.00) | 113 | 1.00 | (1.00, 1.00) |
| | | **Day 28** | 115 | 3.60 | (2.90, 4.47) | 108 | 11.67 | (9.34, 14.58) |
| | | **Month 6** | 112 | 2.18 | (1.84, 2.58) | 107 | 8.70 | (6.96, 10.87) |

| | | | | | | | | |
|---|---|---|---|---|---|---|---|---|
| ^{*}Titer or fold rise, where fold rise = titer at Day 28/titer at Day 0 ^{†}N: total number of participants used in the calculation. | | | | | | | | |

Twenty-eight to 56 days after receiving the study vaccination, TetraMenD, the majority of participants experience a≥4-fold rise in the SBA-BR antibody titer for each of the serogroups contained in the vaccine. Overall, 77% of TetraMenD recipients experience a 4-fold rise in antibody titer across all serogroups. Higher pre-vaccination antibody levels are observed for serogroup Y than for C or W-135. This may be related to the fact that natural exposure to serogroup Y at this age may be more common than previously thought. Higher circulating antibody levels reflect recent natural exposure and may reduce the proportion of vaccine recipients exhibiting 4-fold or higher antibody responses. This clearly appears to be the case for serogroup Y responses when compared to other serogroups. The 4-fold rise for serogroup Y is 56.6% compared with 73.4% for serogroup C, 87.7% for serogroup A, and 91.0% for serogroup W-135. High pre-vaccination antibody levels are also observed for serogroup A. This may be the result of intermittent exposure over a prolonged period of time to several naturally occurring cross-reacting antigens.

To further evaluate the impact of pre-existing titers and to investigate the rate of seroconversion (as defined by the proportion of vaccine recipients who achieve a 4-fold rise in antibody titer when the pre-vaccination titer for any serogroup is < 1:8), a separate analysis is performed on participants who had pre-vaccination antibody titers of <1:8 to any one of the 4 serogroups contained in the vaccine. A titer of < 1:8 by the SBA assay using baby rabbit as the complement source is considered to represent an undetectable level of circulating antibody. When participants are evaluated using this criterion, it is observed that there is a 98.6% seroconversion rate for serogroup A, 87.9% for serogroup C, 96.0% for serogroup W-135, and 86.2% for serogroup Y after vaccination with TetraMenD.

Based on observations in military recruits, Goldschneider proposed that a minimum titer of ≥ 1:4 using an SBA assay with a human complement source correlated with protection from invasive disease against Serogroup C. However, because of the need for standardization of the assay and the lack of a reliable source of human complement, baby rabbit complement is suggested as an alternative source. Meningococci appear to be more sensitive to the baby rabbit complement than human complement, resulting in higher measured antibody titers. Several authors have suggested that titers ≥:128 using the rabbit complement assay are predictive of protection while titers of < 1:8 are predictive of susceptibility at least for serogroup C. Although this level may be appropriate when evaluating polysaccharide vaccines, it may not be applicable for conjugate vaccines. Borrow suggested that, in subjects receiving a monovalent C conjugate vaccine who demonstrated post vaccination SBA titers between 8 and 64, the demonstration of a memory response using a reduced dose (10 µg) of a meningococcal polysaccharide vaccine given several months later showed that these individuals are also protected, having achieved an antibody level ≥1:128. The results for subjects who received the TetraMenD vaccine with SBA-BR titers ≥1:128 for each serogroup are presented the Tables. When these criteria are applied to each of the serogroups contained in the vaccine, overall, 96.2% of participants who received TetraMenD achieved a post-vaccination SBA-BR titer of ≥1:32 and 90.5% achieved a titer ≥1:128. A subset of sera from this clinical study is also used to evaluate the correlation between the SBA assay using baby rabbit complement and human complement and the results are provided in a subsequent Study.

Total IgG responses are significantly higher for serogroups C, Y, and W-135 in the Menomune^{®} group than in the group receiving TetraMenD. However, the post-vaccination SBA GMT levels for serogroups A, C, Y, and W-135, are significantly higher in the TetraMenD group.

**Table B-9 provides a comparison of GMC versus GMT titers by serogroup.**

| **Table B-9 Comparison of IgG GMC and SBA GMTs Titers by Serogroup** | | | |
|---|---|---|---|
| **Serogroup** | **Day 28 Results:** | **IgG GMC** | **SBA GMT** |
| **A** | **TetraMenD** | 7.65 | 1700.3 |
| | **Menomune**^{®} | 6.81 | 892.2 |
| **C** | **TetraMenD** | 1.24 | 353.9 |
| | **Menomune**^{®} | 7.62 | 230.7 |
| **Y** | **TetraMenD** | 1.54 | 636.7 |
| | **Menomune**^{®} | 4.15 | 408.1 |
| **W-135** | **TetraMenD** | 0.90 | 749.8 |
| | **Menomune**^{®} | 2.53 | 424.8 |

The observation that the lower levels of IgG produced by the conjugate generated a higher level of bactericidal activity than the polysaccharide vaccine strongly suggests that the quality and affinity of the antibody response to the conjugate vaccine is superior to that generated by unconjugated polysaccharide vaccine. High affinity antibody is associated with functional activity and memory response. This effect has also been observed in several published studies. These data demonstrate that TetraMenD is highly immunogenic in children aged 2 to 10 years, the observed GMTs in the TetraMenD group are superior to those observed in the Menomune group for each of the four serogroups, and the titers achieved are predictive of protection. Finally, it appears that TetraMenD generates higher affinity antibody responses for each serogroup contained in the vaccine.

Safety is monitored at 4 specific time points during the trial: Immediate reactions (within 30 minutes of vaccination), solicited local and systemic reactions within the first 7 days post-vaccination, all adverse events in the 28-day period after vaccination and continuing AEs (from Days 0-28) and serious adverse events from Day 0 to 6 months post-vaccination are reported.

For all participants, most local solicited reactions for both treatment groups are reported as mild and resolved within 3 days of vaccination. The frequency of local reactions is similar for each treatment group. In the group receiving TetraMenD 58.8% reported at least one local reaction while the group receiving Menomune^{®} 58.3% reported the same. In addition, experience with the monovalent C CRM ₁₉₇ conjugate vaccine given intramuscularly to adolescents shows that the rate of local reactions is very similar to that observed for TetraMenD in this study.

The majority of reported AEs are not serious, reversible, and unrelated to vaccination. There are no reports in this study of new onset bronchial asthma, diabetes mellitus, or autoimmune disease.

### Example 12 Study C One Month Study in Children Aged 11 to 18

Study C is a randomized, active-controlled study of healthy children aged 11 to 18 years as of D0 of a single dose of TetraMenD versus a single dose of Menomune^{®}. Blood serum is drawn on D0, prior to vaccination and D28 and analyzed, and a subset of sera from patients is further evaluated as described in the results.

For all participants, most local solicited reactions for both treatment groups are reported as mild and resolved within 2 days of vaccination. The frequency of local reactions is more common in the group receiving TetraMenD (72.4%) than in the group receiving Menomune^{®} (34.7%). This result is probably due to the nature of the conjugate vaccine (diphtheria carrier protein) rather than the route of administration (intramuscular). The results of this Study are summarized in the following Tables.

**Table C-1 shows the frequency distribution of baseline and Day 28 SBA-BR antibody titers for each serogroup.**

| **Table C-1: Distribution of SBA-BR Antibody Titers at Day 0 and Day 28 After Vaccination (Per-Protocol Population)** | | | | | | | | | | |
|---|---|---|---|---|---|---|---|---|---|---|
| | | | **SBA-BR Titers <8 to 512** | | | | | | | |
| **Test Type** | **Test Date** | **Group (N) *** | | | | | | | | |
| | | | **<8 n (%) ^{†}** | **8 n (% )** | **16 n (% )** | **32 n (% )** | **64 n (% )** | **128 n (% )** | **256 n (% )** | **512 n (% )** |
| **SBA (A)** | **Day 0** | **TetraMen D (425)** | 81 | 19 | 4 (0.9) | 9 | 33 | 76 | 86 | 51 |
| | | | (19.1) | (4.5) | (0.9) | (2.1) | (7.8) | (17.9) | (20.2) | (12.0) |
| | | **Menomune^{®} (423)** | 93 | 12 | 10 | 13 | 38 | 72 | 72 | 56 |
| | | | (22.0) | (2.8) | (2.4) | (3.1) | (9.0) | (17.0) | (17.0) | (13.2) |
| | **Day 28** | **TetraMen D (423)** | _‡ | _ | _ | _ | 1 | _ | 1 | 15 |
| | | | | | | | (0.2) | | (0.2) | (3.5) |
| | | **Menomune^{®} (423)** | _ | _ | _ | _ | _ | 8 | 12 | 19 |
| | | | | | | | | (1.9) | (2.8) | (4.5) |
| **SBA (C)** | **Day 0** | **TetraMen D (425)** | 157 | 37 | 18 | 24 | 36 | 40 | 39 | 35 |
| | | | (36.9) | (8.7) | (4.2) | (5.6) | (8.5) | (9.4) | (9.2) | (8.2) |
| | | **Menomune^{®} (423)** | 152 | 35 | 15 | 19 | 40 | 46 | 42 | 25 |
| | | | (35.9) | (8.3) | (3.5) | (4.5) | (9.5) | (10.9) | (9.9) | (5.9) |
| | **Day 28** | **TetraMen D (423)** | 1 | 1 (0.2) | ― | 1 (0.2) | 2 | 17 | 33 | 58 |
| | | | (0.2) | (0.2) | | (0.2) | (0.5) | (4.0) | (7.8) | (13.7) |
| | | **Menomune^{®} (423)** | 1 | ― | 1 (0.2) | 1 (0.2) | 4 | 26 | 47 | 56 |
| | | | (0.2) | | (0.2) | (0.2) | (0.9) | (6.1) | (11.1) | (13.2) |
| **SBA (Y)** | **Day 0** | TetraMenD D (425) | 61 | 6 | 1 | 22 | 64 | 94 | 101 | 50 |
| | | | (14.4) | (1.4) | (0.2) | (5.2) | (15.1) | (22.1) | (23.8) | (11.8) |
| | | Menomune® (423) | 47 | 3 | 7 | 27 | 74 | 94 | 85 | 51 |
| | | | (11.1) | (0.7) | (1.7) | (6.4) | (17.5) | (22.2) | (20.1) | (12.1) |
| | **Day 28** | TetraMen D (423) | _ | ― | 1 | ― | 1 | 23 | 53 | 71 |
| | | | | | (0.2) | | (0.2) | (5.4) | (12.5) | (16.8) |
| | | Menomune® (423) | 1 | ― | ― | ― | 2 | 11 | 59 | 81 |
| | | | (0.2) | | | | (0.5) | (2.6) | (13.9) | (19.1) |
| **SBA (W 135)** | **Day 0** | TetraMen D (425) | 165 | 37 | 28 | 36 | 60 | 56 | 22 | 15 |
| | | | (38.8) | (8.7) | (6.6) | (8.5) | (14.1) | (13.2) | (5.2) | (3.5) |
| | | Menomune^{®} (423) | 139 | 52 | 25 | 34 | 67 | 43 | 46 | 11 |
| | | | (32.9) | (12.3) | (5.9) | (8.0) | (15.8) | (10.2) | (10.9) | (2.6) |
| | **Day 28** | TetraMen D (423) | 4 | ― | 1 | ― | 1 | 19 | 34 | 63 |
| | | | (0.9) | | (0.2) | | (0.2) | (4.5) | (8.0) | (14.9) |
| | | Menomune^{®} (423) | 1 | 1 (0.2) | ― | 1 (0.2) | 2 | 12 | 21 | 51 |
| | | | (0.2) | (0.2) | | (0.2) | (0.5) | (2.8) | (5.0) | (12.1) |

| | | | **SBA-BR Titers 1024 to > 65536** | | | | | | | |
|---|---|---|---|---|---|---|---|---|---|---|
| **Test Type** | **Test Date** | **Group (N) *** | | | | | | | | |
| | | | **1024 n (%** ) | **2048 n(% )** | **4096 n(% )** | **8192 n(% )** | **1638 4 n(%** ) | **32768 n(%)** | **65536 n %)** | **> 6553 6 n(%)** |
| **SBA (A)** | **Day 0** | TetraMen D (425) | 32 | 29 | 2 | 1 | 2 | ― | ― | ― |
| | | | (7.5) | (6.8) | (0.5) | (0.2) | (0.5) | | | |
| | | Menomune^{®} (423) | 38 | 14 | 2 | 2 | 1 | ― | ― | ― |
| | | | (9.0) | (3.3) | (0.5) | (0.5) | (0.2) | | | |
| | **Day 28** | TetraMen D (423) | 36 | 66 | 90 | 108 | 63 | 37 | 4 | 2 |
| | | | (8.5) | (15.6) | (21.3) | (25.5) | (14.9 ) | (8.7) | (0.9) | (0.5) |
| | | Menomune^{®} (423) | 46 | 100 | 108 | 76 | 43 | 11 | ― | ― |
| | | | (10.9) | (23.6) | (25.5) | (18.0) | (10.2 ) | (2.6) | | |
| **SBA (C)** | **Day 0** | TetraMen D (425) | 20 | 13 | 4 | 2 | ― | ― | ― | ― |
| | | | (4.7) | (3.1) | (0.9) | (0.5) | | | | |
| | | Menomune® (423) | 23 | 22 | 2 | 1 | . 1 | ― | ― | ― |
| | | | (5.4) | (5.2) | (0.5) | (0.2) | (0.2) | | | |
| | **Day 28** | TetraMen D (423) | 66 | 82 | 66 | 45 | 24 . | 21 | 4 | 2 |
| | | | (15.6) | (19.4) | (15.6) | (10.6) | (5.7) | (5.0) | (0.9) | (0.5) |
| | | Menomune® (423) | 70 | 64 | 55 | 41 | 35 | 19 | 3 | ― |
| | | | (16.5) | (15.1) | (13.0) | (9.7) | (8.3) | (4.5) | (0.7) | |
| **SBA (Y)** | **Day 0** | TetraMen D (425) | 13 | 6 | 2 | 2 | 2 | 1 | ― | ― |
| | | | (3.1) | (1.4) | (0.5) | (0.5) | (0.5) | (0.2) | | |
| | | Menomune^{®} (423) | 24 | 6 | 2 | 1 | 1 | 1 | ― | ― |
| | | | (5.7) | (1.4) | (0.5) | (0.2) | (0.2) | (0.2) | | |
| | **Day 28** | TetraMen D (423) | 77 | 80 | 52 | 41 | 16 | 7 | 1 | ― |
| | | | (18.2) | (18.9) | (12.3) | (9.7) | (3.8) | (1.7) | (0.2) | |
| | | Menomune^{®} (423) | 90 | 74 | 53 | 35 | 11 | 6 | ― | ― |
| | | | (21.3) | (17.5) | (12.5) | (8.3) | (2.6) | (1.4) | | |
| **SBA (W-135)** | **Day 0** | TetraMen D (425) | 4 | 2 | ― | ― | ― | ― | ― | ― |
| | | | (0.9) | (0.5) | | | | | | |
| | | Menomun e® (423) | 4 | 1 | 1 | ― | ― | ― | ― | ― |
| | | | (0.9) | (0.2) | (0.2) | | | | | |
| | **Day 28** | TetraMen D (423) | 90 | 88 | 64 | 36 | 16 | 6 | 1 | ― |
| | | | (21.3) | (20.8) | (15.1) | (8.5) | (3.8) | (1.4) | (0.2) | |
| | | Menomune® (423) | 103 | 114 | 67 | 42 | 6 | 2 | - | ― |
| | | | (24.3) | (27.0) | (15.8) | (9.9) | (1.4) | (0.5) | | |

**Table C-2 summarizes GMT levels by Subject Age and Serogroup for TetraMenD**

| **Table C-2 Summary of GMT by Subject Age and Serogroup for TetraMenD** | | | | | | |
|---|---|---|---|---|---|---|
| Age (in Year) | Blood Day | No. of Subjects | Serogroup A GMT | Serogroup C GMT | Serogroup . W GMT | Serogroup Y-135 GMT. |
| 11 | Day 0 | 45 | 101.59 | 37.33 | 21.77 | 91.21 |
| | Day 28 | 45 | 4705.07 | 1372.15 | 1482.00 | 1024.00 |
| 12 | Day 0 | 54 | 104.24 | 42.99 | 14.63 | 72.77 |
| | Day 28 | 53 | 5049.37 | 2157.99 | 1245.94 | 1198.00 |
| 13 | Day 0 | 65 | 145.47 | 32.34 | 20.23 | 128.00 |
| | Day 28 | 65 | 7363.39 | 1880.53 | 2206.73 | 1782.89 |
| 14 | Day 0 | 67 | 85.50 | 31.67 | 24.71 | 107.36 |
| | Day 28 | 67 | 5124.87 | 2006.06 | 1753.62 | 1159.35 |
| 15 | Day 0 | 70 | 129.27 | 42.22 | 27.31 | 126.74 |
| | Day 28 | 68 | 4870.99 | 2090.18 | 1193.17 | 1193.17 |
| 16 | Day 0 | 69 | 103.66 | 21.41 | 22.74 | 129.29 |
| | Day 28 | 69 | 7189.09 | 2357.27 | 1455.45 | 2068.68 |
| 17 | Day 0 | 69 | 85.64 | 38.73 | 13.90 | 72.93 |
| | Day 28 | 67 | 4269.06 | 1665.21. | 841.27 | 904.45 |
| 18 | Day 0 | 1 | 4 | 8 | 16 | 64 |
| | Day 28 | 1 | 8192.00 | 256.00 | 512.00 | 8192.00 |

Table C-3 shows the numbers and percentages of participants with a ≥ 4-fold rise in SBA-BR titer from baseline to Day 28 for the serogroups A, C, Y, and W-135. For each serogroup, these percentages are higher in the TetraMenD group than in the Menomune^{®} group.

**Table C-3: Numbers and Percentages of participants with a ≥4-fold rise in SBA-BR titer from Baseline to Day 28**

| **≥ 4-fold rise in SBA titer for serogroup** | **TetraMenD n/N Pt Proportion** | | **Menomune^{®} n/N Pₘ Proportion** | | **Difference (Pₘ - Pₜ)** | **Upper bound of the one sided 95% CI of the Difference** |
|---|---|---|---|---|---|---|
| A | 392/423 | 0.9267 | 391/423 | 0.9243 | NA | NA |
| C | 388/423 | 0.9173 | 375/423 | 0.8865 | -0.0307 | 0.0029 |
| Y | 346/423 | 0.8180 | 339/423 | 0.8014 | -0.0165 | 0.0278 |
| W-135 | 409/423 | 0.9669 | 403/423 | 0.9527 | -0.0142 | 0.0080 |

### Frequency of SBA-BR Antibody Titers ≥32

The proportion of participants with SBA antibody titers≥ 32 at Day 28 after vaccination is summarized in Table C-4.

**Table C-4: Percentage and Number of Participants with an SBA Antibody Titer ≥ 32 at Day 28 Post-Vaccination (Per-Protocol Population)**

| | **TetraMenD** | | **Menomune^{®}** | |
|---|---|---|---|---|
| | **% (n^{†}/N^{‡})** | **95% CI for the percentage** | **%* (n^{†}/ N^{‡})** | **95% CI for the percentage** |
| **Serogroup A** | 100.00 (423/423) | (99.13, 100.00) | 100.00 (423/423) | (99.13,100.00) |
| **Serogroup C** | 99.53 (421/423) | (98.30,100.00) | 99.53 (421/423) | (98.30,100.00) |
| **Serogroups Y** | 99.76 (422/423) | (98.69, 100.00) | 99.76 (422/423) | (98.69,100.00) |
| **Serogroups W-135** | 98.82 (418/423) | (97.26, 00.00) | 99.53 (421/423) | (98.30,100.00) |

| | | | | |
|---|---|---|---|---|
| *%:n/N. ^{†}n: number of participants with titer≥32 at Day 28 post vaccination. ^{‡}N: total number of participants with valid blood sample at Day 28 in this group. | | | | |

### Frequency of SBA-BR Antibody Titers ≥ 128

The proportion of participants with SBA antibody titers ≥28 at Day 28 after vaccination is summarized in Table C-5.

**Table C-5: Percentage and Number of Participants with an SBA Antibody Titer ≥ 128 at Day 28 Post-Vaccination (Per-Protocol Population)**

| | **TetraMenD** | | **Menomune^{®}** | |
|---|---|---|---|---|
| | **%* (n^{†}/N^{‡})** | **95% CI for the percentage** | **%* (n^{†}/N^{‡})** | **95% CI for the percentage** |
| **Serogroup A** | 99.76 (422/423) | (98.69, 100.00) | 100.00 (423/423) | (99.13, 100.00) |
| **Serogroup C** | 98.82 (418/423) | (97.26,100.00) | 98.35 (416/423) | (96.62,100.00) |
| **Serogroups Y** | 99.53 (421/423) | (98.30,100.00) | 99.29 (420/423) | (97.94, 100.00) |
| **Serogroup W-135** | 98.58 (417/423) | (96.94,100.00) | 98.82 (418/423) | (97.26,100.00) |

| | | | | |
|---|---|---|---|---|
| ^{*} %: n/N expressed as a percentage. ^{†} n: number of participants with titer≥ 28 at Day 28 post-vaccination. ^{‡} N: total number of participants with valid blood sample at Day 28 in this group. | | | | |

### Percentage of Participants with ≥ 4-fold Rise in SBA-BR Antibody Titers

Table C-6 shows the proportion of participants with a ≥ 4-fold rise in Day 28 SBA antibody titers from baseline.

**Table C-6: Percentage and Number of Participants with a ≥-Fold Rise in Day 28 SBA Antibody Titers From Baseline**

| **Test Type** | **TetraMenD** | | **Menomune^{®}** | |
|---|---|---|---|---|
| | **%* (n^{†}/N^{‡})** | **(95% CI)** | **%* (n^{†}/N^{‡})** | **(95% CI)** |
| **Serogroup A SBA** | 92.7 (392/423) | (89.8, 95.0) | 92.4 (391/423) | (89.5, 94.8) |
| **Serogroup C SBA** | 91.7 (388/423) | (88.7, 94.2) | 88.7 (375/423) | (85.2, 91.5) |
| **Serogroup Y SBA** | 81.8 (346/423) | (77.8,85.4) | 80.1 (339/423) | (76.0, 83.8) |
| **Serogroup W-135 SBA** | 96.7 (409/423) | (94.5, 98.2) | 95.3 (403/423) | (92.8,97.1) |

| | | | | |
|---|---|---|---|---|
| ^{*}%: n/N expressed as a percentage. ^{†}n: number of participants with ≥4-fold rise from baseline titer. ^{‡}N: total number of participants in the used population | | | | |

### Percentage of Participants with Undetectable Titers (<8) at Day 0 achieving a ≥4-Fold Rise in Day 28 SBA-BR Antibody Titers

In both treatment groups and for all vaccine serogroups, most participants with an undetectable (< 8) SBA titer at baseline achieve a ≥4 -fold rise in Day 28 SBA titers. The proportions of participants with an SBA titer <8 at Day 0 who had a ≥ 4-fold rise from baseline to Day 28 range from 98.17% to 100.0% (Table C-7).

**Table C-7: Number and Percentage of Participants with Undetectable Titers (<8) at Day 0 Achieving a ≥4-Fold Rise in Day 28 SBA-BR Antibody Titers**

| | **TetraMenD** | | | **Menomune^{®}** | | |
|---|---|---|---|---|---|---|
| **Serogroup** | **Percent** | **n/N*** | **95% CI** | **Percent** | **n/N** | **95% CI** |
| **A** | 100.00 | 81/81 | (95.55, 100.00) | 100.00 | 93/93 | (96.11, 100.00) |
| **C** | 98.71 | 153/155 | (95.42, 00.00) | 99.34 | 151/152 | (96.39, 00.00) |
| **Y** | 98.17 | 161/164 | (94.75, 00.00) | 99.28 | 138/139 | (96.06,100.00) |
| **W-135** | 98.36 | 60/61 | (91.20,100.00) | 100.00 | 47/47 | (92.45, 100.00) |

| | | | | | | |
|---|---|---|---|---|---|---|
| *n = The number of participants with titers < 8 at Day 0 and titers ≥ 32 at Day 28 within each serogroup N = The number of participants with titers < 8 at Day 0 within each serogroup ^{†} Exact 95% confidence interval for the percentage | | | | | | |

### SBA-BR Antibody GMTs and mean fold rises

Table C-8 shows the SBA GMTs at baseline and on Day 28 after vaccination and the fold rises in SBA GMTs.

**Table C-8: SBA Serology Results at Baseline and at Day 28**

| **After Vaccination (Per-Protocol Population)** | | | | | | | | |
|---|---|---|---|---|---|---|---|---|
| Test Type | Parameter* | Bleed | TetraMenD | | | Menomune^{®} | | |
| | | | N^{†} | Geometric Mean | (95% CI) (95% | N^{†} | Geometric Mean | (95% CI |
| Serogroup A SBA | Titer | Day 0 | 425 | 106.28 | (87.60, 128.95) | 423 | 88.67 | (73.05, 107.64) |
| | | Day 28 | 423 | 5483.21 | (4920.12, 6110.74) | 423 | 3245.6 7 | (2909.97, 3260.11) |
| | Fold rise | Day 28 | 423 | 44.92 | (36.98, 54.57) | 423 | 31.43 | (26.62, 37.10) |
| Serogroup . Serogroup CSBA | Titer | Day 0 | 425 | 33.71 | (27.54, 41.28) | 423 | 37.39 | (30.40, 45.98) |
| | | Day 28 | 423 | 1924.36 | (1662.08, 2228.03) | 423 | 1638.8 7 | (1405.55, 1910.93) |
| | Fold rise | Day 28 | 423 | 43.83 | (36.40, 52.78) | 423 | 34.17 | (28.31, 41.24) |
| Y Serogroup SBA YSBA | Titer | Day 0 | 425 | 103.21 | (87.80, 121.32) | 423 | 111.91 | (96.03, 130.41) |
| | | Day 28 | 423 | 1322.26 | (1161.85, 1504.82) | 423 | 1228.2 7 | (1088.20, 1386.37) |
| | Fold rise | Day 28 | 423 | 11.62 | (9.94, 13.60) | 423 | 10.16 | (8.76, 11.79) |
| Serogroup W-135 SBA | Titer | Day 0 | 425 | 20.70 | (17.70, 24.22) | 423 | 23.90 | (20.40, 28.02) |
| | | Day 28 | 423 | 1407.22 | (1232.07, 1607.27) - | 423 | 1544.9 9 | (1383.63, 1725.16) |
| | Fold rise | Day 28 | 423 | 51.98 | (44.36, 60.90) | 423 | 51.47 | (44.32, 59.76) |

| | | | | | | | | |
|---|---|---|---|---|---|---|---|---|
| * Titer or fold rise, where fold rise = titer at Day 28/titer at Day 0 ^{†}N. total number of participants used in the calculation. | | | | | | | | |

### ELISA IgG for serogroup A, C, W-135, and Y

Table C-9 shows the IgG GMCs (in µg/mL) at baseline and on Day 28 after vaccination and the fold rises in IgG GMCs.

**Table C-9: IgG Serology Results at Baseline and at Day 28 After Vaccination (Per-Protocol Population)**

| **Test Type** | **Parameter^{*}** | **Bleed** | **TetraMenD** | | | **Menomune^{®}** | | |
|---|---|---|---|---|---|---|---|---|
| | | | **N^{†}** | **Geometric Mean (µ g/mL)** | **(95% CI)** | **N^{†}** | **Geometric Mean (µg/mL)** | **(95% CI)** |
| **Serogroup A (IgG) ELISA** | **Titer** | **Day 0** | 82 | 0.84 | (0.61, 1.16) | 79 | 0.62 | (0.45, 0.84) |
| | | **Day 28** | 82 | 18.09 | (13.56, 24.12) | 79 | 11.61 | (8.81, 15.29) |
| | **Fold rise** | **Day 28** | 82 | 21.49 | (16.35, 28.24) | 79 | 18.87 | (14.23, 25.00) |
| **Serogroup. C (IgG)ELISA** | **Titer** | **Day 0** | 82 | 0.27 | (0.23, 0.31) | 79 | 0.30 | (0.24, 0.37) |
| | | **Day 28** | 82 | 5.54 | (3.85, 7.97) | 79 | 8.08 | (5.37, 12.18) |
| | **Fold rise** | **Day 28** | 82 | 20.78 | (14.74, 29.28) | 79 | 26.97 | (18.93, 38.41) |
| **Serogroup Y (IgG)ELISA** | **Titer** | **Day 0** | 82 | 0.41 | (0.32, 0.53) | 79 | 0.39 | (0.30, 0.50) |
| | | **Day 28** | 82 | 4.41 | (2.74, 7.08) | 79 | 9.17 | (6.58, 12.78) |
| | **Fold rise** | **Day 28** | 82 | 10.81 | (7.32, 15.95) | 79 | 23.55 | (16.93, 32.75) |
| **Serogroup W-135(IgG) ELISA** | **Titer** | **Day 0** | 82 | 0.24 | (0.20, 0.29) | 79 | 0.24 | (0.19, 0.30) |
| | | **Day 28** | 82 | 2.95 | (2.02, 4.30) | 79 | 4.93 | (3.47, 7.00) |
| | **Fold rise** | **Day 28** | 82 | 12.26 | (8.48, 17.73) | 79 | 20.40 | (14.62, 28.46) |

| | | | | | | | | |
|---|---|---|---|---|---|---|---|---|
| ^{*} Titer or fold rise, where fold rise = titer at Day 28/titer at Day 0 ^{†}N: total number of participants used in the calculation. | | | | | | | | |

### ELISA IgM for serogroups A, C, Y, and W-135

**Table C-10 shows the IgM GMCs at baseline and on Day 28 after vaccination and the fold rises in IgM GMCs. Table C-10: IgM Serology Results at Baseline and at Day 28 After Vaccination (Per-Protocol Population)**

| **Test Type** | **Parameter^{*}** | **Bleed** | **TetraMenD** | | | **Menomune^{®}** | | |
|---|---|---|---|---|---|---|---|---|
| | | | **N^{†}** | **Geometric Mean** | **(95% CI)** | **N^{†}** | **Geometric Mean** | **(95% CI)** |
| **Serogroup A (IgM) ELISA** | **Titer** | **Day 0** | 81 | 1.66 | (1.34 2.06) | 79 | 1.42 | (1.15, 1.75) |
| | | **Day 28** | 80 | 17.80 | (14.67, 21.59) | 79 | 12.00 | (9.67, 14.89) |

**Table C-10 shows the IgM GMCs at baseline and on Day 28 after vaccination and the fold rises in IgM GMCs. Table C-10: IgM Serology Results at Baseline and at Day 28 After Vaccination (Per-Protocol Population)**

| **TestType** | **Parameter^{*}** | **Bleed** | **TetraMenD** | | | **Menomune^{®}** | | |
|---|---|---|---|---|---|---|---|---|
| | | | **N^{†}** | **Geometric Mean** | **(95% CI)** | **N^{†}** | **Geometric Mean** | **(95% CI)** |
| | **Fold rise** | **Day 28** | 79 | 11.22 | (8.54, 14.73) | 79 | 8.47 | (7.06, 10.16) |
| **Serogroup C (IgM) ELISA** . | **Titer** | **Day 0** | 82 | 0.19 | (0.14, 0.24) | 79 | 0.16 | (0.12, 0.22) |
| | | **Day 28** | 80 | 1.55 | (1.20, 2.00) | 79 | 1.71 | (1.39, 2.10) |
| | **Fold rise** | **Day 28** | 80 | 8.42 | (6.34, 11.18) | 79 | 10.60 | (8.04, 13.97) |
| **Serogroup (IgM) ELISA** | **Titer** | **Day 0** | 82 | 0.37 | (0.29, 0.46) | 79 | 0.40 | (0.32, 0.50) |
| | | **Day 28** | 80 | 3.47 | (2.81, 4.27) | 79 | 3.45 | (2.85, 4.17) |
| | **Fold rise** | **Day 28** | 80 | 9.47 | (7.43, 12.05) | 79 | 8.65 | (6.79, 11.03) |
| **Serogroup W-135 (IgM) ELISA** | **Titer** | **Day 0** | 82 | 0.17 | (0.15, 0.20) | 79 | 0.18 | (0.16, 0.21) |
| | | **Day 28** | 82 | 1.92 | (1.60, 2.29) | 79 | 1.68 | (1.41, 1.99) |
| | **Fold rise** | **Day 28** | 82 | 11.01 | (9.06, 13.39) | 79 | 9.16 | (7.61, 11.03) |

| | | | | | | | | |
|---|---|---|---|---|---|---|---|---|
| ^{*} Titer or fold rise, where fold rise = titer at Day 28/titer at Day 0 ^{†}N: total number of participants used in the calculation. | | | | | | | | |

Twenty-eight to 56 days after receiving the study vaccination, TetraMenD, the majority of participants experience a ≥ 4-fold rise in the SBA-BR antibody titer for each of the serogroups contained in the vaccine. Overall, 90.7% of TetraMenD recipients experience a 4-fold rise in antibody titer across all serogroups. Higher pre-vaccination antibody levels are observed for serogroup Y than for C or W-135. This may be related to the fact that serogroup Y is currently the most common serogroup associated with invasive meningococcal disease in this age group in the U.S. and that natural exposure to this serogroup may be more common. Higher circulating antibody levels reflect recent natural exposure and may reduce the proportion of vaccine recipients exhibiting 4-fold or higher antibody responses. This appears to be the case for serogroup Y responses when compared to other serogroups. The 4-fold rise for serogroup Y is 81.8% compared with 91.7% for serogroup C and 96.7% for serogroup W-135. High pre-vaccination antibody levels are also observed for serogroup A. This may be the result of intermittent exposure over a prolonged time to several naturally occurring cross-reacting antigens.

To further evaluate the impact of pre-existing titers and to investigate the rate of seroconversion (as defined by the proportion of vaccine recipients who achieve a 4-fold rise in antibody titer when the pre-vaccination titer for any serogroup is< 1:8),a separate analysis is performed on participants who had pre-vaccination antibody titers of <1:8 to any one of the 4 serogroups contained in the vaccine. A titer of < 1:8 by the SBA assay using baby rabbit as the complement source is considered to represent an undetectable level of circulating antibody. When participants are evaluated using this criterion, it is observed that there is a 100% seroconversion rate for serogroup A, 98.1% for serogroup C, 98.1% for serogroup W-135, and 98.3% for serogroup Y after vaccination with TetraMenD.

As previously discussed in another Study, Goldschneider proposed that a minimum titer of ≥ 1:4 using an SBA assay with a human complement source correlated with protection from invasive disease against Serogroup C based on observations in military recruits. However, because of the need for standardization of the assay and the lack of a reliable source of human complement, baby rabbit complement is suggested as an alternative source. Meningococci appear to be more sensitive to the baby rabbit complement than human complement, resulting in higher measured antibody titers. Several authors have suggested that titers ≥ 1:128 using the rabbit complement assay are predictive of protection while titers of < 1:8 are predictive of susceptibility at least for serogroup C. Although this level may be appropriate when evaluating polysaccharide vaccines, it may not be applicable for conjugate vaccines. Borrow suggested that, in subjects receiving a monovalent C conjugate vaccine who demonstrated post vaccination SBA titers between 8 and 64, the demonstration of a memory response using a reduced dose (10 µ.g) of a meningococcal polysaccharide vaccine given several months later showed that these individuals are also protected, having achieved an antibody level ≥1: :128. The results for subjects who received the TetraMenD vaccine with SBA-BR titers ≥ 1:128 for each serogroup is presented in the Tables. When these criteria are applied to each of the serogroups contained in the vaccine, overall, 99.2% of participants who received TetraMenD achieved a post-vaccination SBA-BR titer of 2 ≥ 1:128. IgG and IgM responses are evaluated in a subset of participants using a standard ELISA assay. Post-vaccination, the mean level of IgG antibody in the TetraMenD recipients is > 2 µg for each serogroup. IgM responses are very similar for each serogroup in both treatment arms. The IgG responses are generally higher for serogroups C, Y, and W-135 in the Menomune^{®} group than in the group receiving TetraMenD. The post-vaccination SBA GMT levels for serogroups C, Y, and W-135, however, are very similar in each treatment group. Table C-11.

**Table C-11 : Relative Contribution of IgG and IgM to Total Bactericidal Activity**

| **Serogroups** | **Day 28 Results:** | **IgG GMC*** | **IgM GMC** | **SEA GMT** |
|---|---|---|---|---|
| **A** | TetraMenD | 18.09 | 17.80 | 5483.21 |
| | Menomune^{®} | 11.61 | 12.00 | 3245.67 |
| **C** | TetraMenD | 5.54 | 1.55 | 1924.36 |
| | Menomune^{®} | 8.08 | 1.71 | 1638.87 |
| **Y** | TetraMenD | 4.41 | 3.47 | 1322.26 |
| | Menomune^{®} | 9.17 | 3.45 | 1228.27 |
| **W-135** | TetraMenD | 2.95 | 1.92 | 1407.22 |
| | Menomune^{®} | 4.93 | 1.68 | 1544.99 |

| | | | | |
|---|---|---|---|---|
| * GMC units are µg/mL | | | | |

The observation that the lower levels of IgG produced by the conjugate generated a similar level of bactericidal activity as the polysaccharide vaccine strongly suggests that the quality and affinity of the antibody response to the conjugate vaccine is superior to that generated by the polysaccharide. It is the high affinity antibody that is associated with functional activity and memory response. This effect has also been observed in several published studies.

These data demonstrate that TetraMenD is highly immunogenic in the adolescent population. The GMTs are essentially equivalent for each of the four serogroups for both vaccines, and the titers achieved are predictive of protection, and it appears that TetraMenD generates higher affinity antibody responses for each serogroup contained in the vaccine.

### Study D

Study D is a randomized, active-controlled study of healthy adults aged 18 to 55 years as of D0 of a single dose of TetraMenD versus a single dose of Menomune^{®}. Blood serum is drawn on D0, prior to vaccination and D28 and analyzed.

Generally, the safety profile of TetraMenD is comparable to Menomun, specifically, the percentages reported for Solicited Local Reactions (Days 0-7), Solicited Systemic Reactions (Days 0-) Unsolicited Adverse Events (Days 0-28) Unsolicited Significant Adverse Events and SAEs (Day 29-Month 6) Serious Adverse Events (Day 0-Month 6) are all within 2-3% of the percentages reported for Menomune. The results of the Study are provided in the following Tables.

### Distribution of SBA-BR Antibody Titers

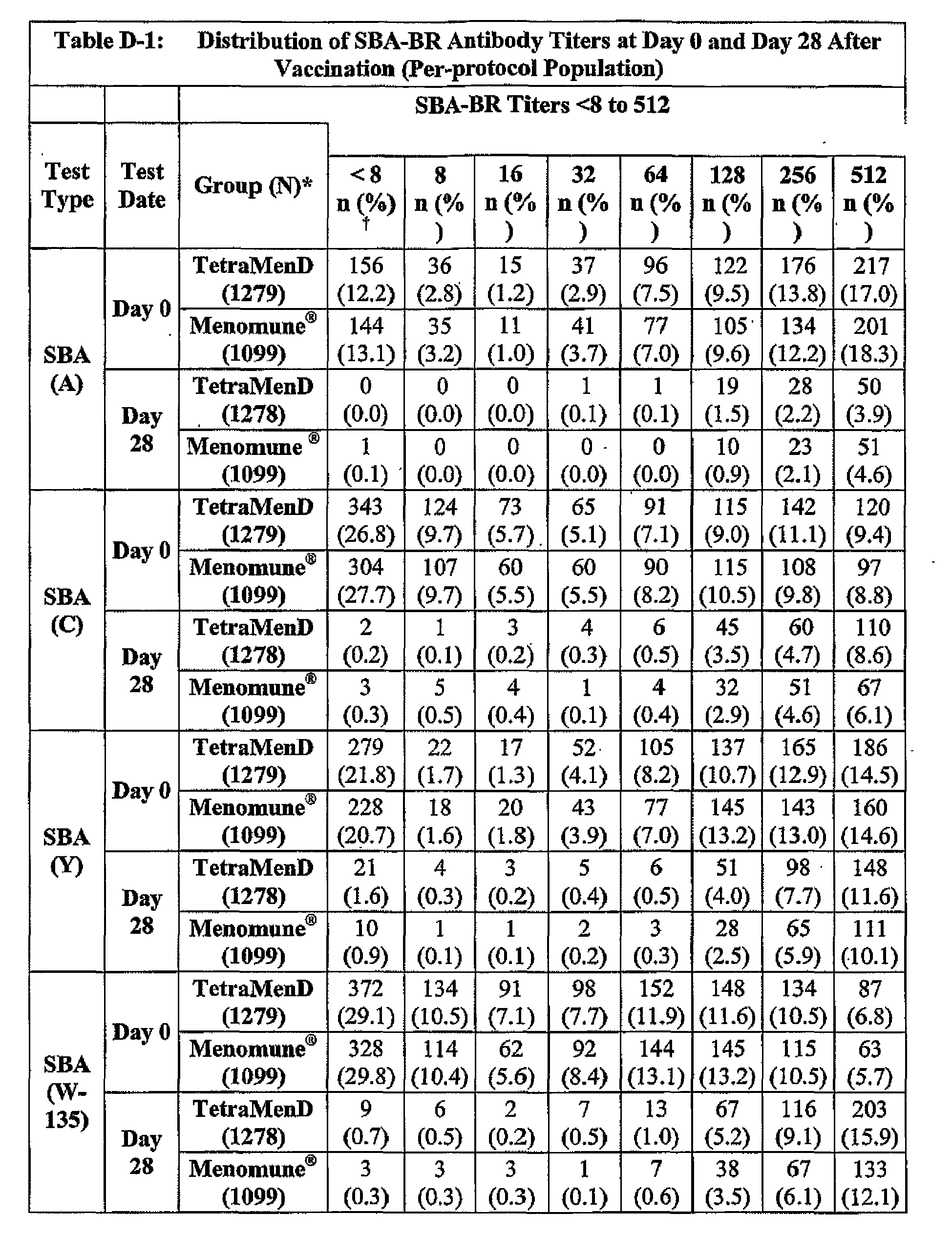

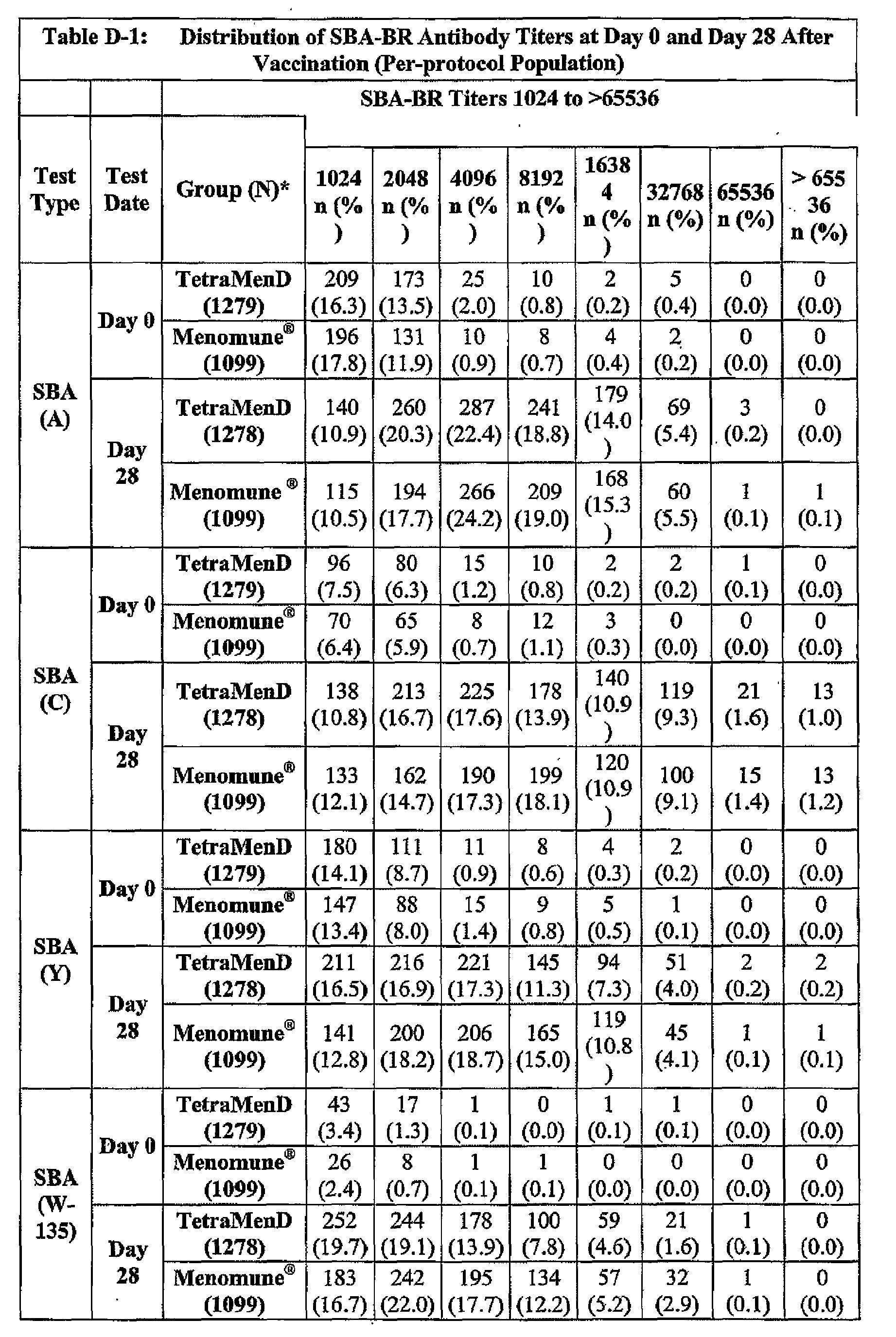

**Table D-2 provides a summary of the Geometric Mean Titer (GMT) by Subject Age and Serogroup for TetraMenD.**

| **Table D-2 Summary of GMT by Subject Age and Serogroup for TetraMenD** | | | | | | |
|---|---|---|---|---|---|---|
| Age (in Year) | Blood Day | No. of Subjects | Serogroup A GMT | Serogroup C GMT | Serogroup W GMT | Serogroup Y-135 GMT |
| 18 | Day 0 | 127 | 238.47 | 45.63 | 42.04 | 111.67 |
| | Day 28 | 122 | 5170.42 | 2690.10 | 1613.23 | 2556.00 |
| 19 | Day 0 | 132 | 224.51 | 40.96 | 37.66 | 244.18 |
| | Day 28 | 127 | 4421.24 | 2425.55 | 1786.78 | 2492.65 |
| 20 | Day 0 | 107 | 193.76 | 69.62 | 41.73 | 108.16 |
| | Day 28 | 103 | 5080.24 | 3193.18 | 1766.17 | 1966.95 |
| 21 | Day 0 | 106 | 239.80 | 57.27 | 32.42 | 122.27 |
| | Day 28 | 105 | 3911.03 | 2447.58 | 1521.66. | 1725.00 |
| 22 | Day 0 | 84 | 371.11 | 47.95 | 34.18 | 175.14 |
| | Day 28 | 82 | 4649.72 | 3151.78 | 1729.45 | 3151.78 |
| 23 | Day 0 | 81 | 219.45 | 54.40 | 50.80 | 180.25 |
| | Day 28 | 80 | 4664.48 | 3788.73 | 1620.81 | 1961.17 |
| 24 | Day 0 | 66 | 223.33 | 71.84 | 42.49 | 124.03 |
| | Day 28 | 64 | 3922.34 | 3057.48 | 1479.87 | 1562.21 |
| 25 | Day 0 | 62 | 289.50 | 48.94 | 29.26 | 148.02 |
| | Day 28 | 59 | 4771.87 | 3685.02 | 2121.47 | 1563.07 |
| 26 | Day 0 | 29 | 131.10 | 37.83 | 17.19 | 73.87 |
| | Day 28 | 26 | 4936.36 | 4320.32 | 1170.01 | 2673.69 |
| 27 | Day 0 | 20 | 315.17 | 45.25 | 28.84 | 445.72 |
| | Day 28 | 20 | 5042.77 | 5595.30 | 803.41 | 2702.35 |
| 28 | Day 0 | 33 | 593.10 | 72.60 | 55.25 | 157.92 |
| | Day 28 | 33 | 6640.01 | 4948.33 | 1558.63 | 2091.47 |
| 29 | Day 0 | 26 | 270.02 | 29.54 | 28.76 | 51.71 |
| | Day 28 | 26 | 4936.36 | 2534.86 | 1201.62 | 2278.46 |
| 30 | Day 0 | 19 | 229.46 | 68.84 | 25.71 | 99.15 |
| | Day 28 | 19 | 5287.69 | 5687.92 | 1645.39 | 1529.61 |
| 31 | Day 0 | 17 | 138.88 | 52.20 | 27.18 | 226.53 |
| | Day 28 | 17 | 2314.48 | 3340.58 | 1111.00 | 1966.18 |
| 32 | Day 0 | 24 | 362.04 | 58.69 | 64.00 | 50.80 |
| | Day 28 | 24 | 4732.32 | 3545.24 | 1448.15 | 1933.05 |
| 33 | Day 0 | 22 | 329.39 | 109.34 | 68.16 | 164.69 |
| | Day 28 | 22 | 2989.02 | 3499.00 | 1317.54 | 1749.50 |
| 34 | Day 0 | 16 | 534.67 | 51.54 | 41.50 | 112.40 |
| | Day 28 | 15 | 3734.42 | 4096.00 | 741.00 | 1415.08 |
| 35 | Day 0 | 17 | 369.50 | 156.95 | 19.62 | 48.11 |
| | Day 28 | 16 | 4096.00 | 6888.62 | 824.57 | 1649.14 |
| 36 | Day 0 | 17 | 192.44 | 69.44 | 20.43 | 156.95 |
| | Day 28 | 17 | 3078.98 | 7864.70 | 1362.24 | 2410.80 |
| 37 | Day 0 | 20 | 238.86 | 78.79 | 20.39 | 73.52 |
| | Day 28 | 19 | 1835.69 | 4406.03 | 637.28 | 1474.81 |
| 38 | Day 0 | 24 | 203.19 | 90.51 | 15.54 | 78.34 |
| | Day 28 | 24 | 3158.45 | 4216.02 | 558.34 | 1824.56 |
| 39 | Day 0 | 18 | 376.25 | 61.58 | 33.26 | 143.68 |
| | Day 28 | 18 | 4778.10 | 4096.00 | 1824.56 | 1970.63 |
| 40 | Day 0 | 25 | 249.00 | 71.51 | 25.63 | 86.82 |
| | Day 28 | 25 | 3983.99 | 3769.09 | 916.51 | 1112.82 |
| 41 | Day 0 | 26 | 242.71 | 60.68 | 23.24 | 131.46 |
| | Day 28 | 26 | 3681.69 | 2403.25 | 1201.62 | 1336.84 |
| 42 | Day 0 | 24 | 128.00 | 71.84 | 25.40 | 32.94 |
| | Day 28 | 24 | 2435.50 | 2169.78 | 542.45 | 574.70 |
| 43 | Day 0 | 23 | 144.40 | 51.83 | 33.99 | 72.70 |
| | Day 28 | 23 | 2453.92 | 1815.42 | 1120.89 | 1515.12 |
| 44 | Day 0 | 27 | 198.04 | 101.59 | 47.03 | 99.02 |
| | Day 28 | 27 | 2647.42 | 3335.54 | 1194.53 | 998.05 |
| 45 | Day 0 | 23 | 158.06 | 53.41 | 31.05 | 79.03 |
| | Day 28 | 23 | 2241.79 | 2686.11 | 453.85 | 1561.48 |
| 46 | Day 0 | 28 | 204.87 | 55.17 | 21.53 | 68.93 |
| | Day 28 | 28 | 2205.89 | 1680.05 | 927.46 | 1217.75 |
| 47 | Day 0 | 20 | 187.40 | 81.57 | 32.00 | 87.43 |
| | Day 28 | 20 | 2352.53 | 4544.80 | 652.58 | 1351.18 |
| 48 | Day 0 | 32 | 94.52 | 38.05 | 25.22 | 139.58 |
| | Day 28 | 32 | 3158.45 | 2435.50 | 939.01 | 1299.51 |
| 49 | Day 0 | 19 | 114.73 | 33.19 | 24.79 | 137.69 |
| | Day 28 | 19 | 2048.00 | 4096.00 | 951.95 | 1586.44 |
| 50 | Day 0 | 16 | 145.76 | 94.52 | 34.90 | 98.70 |
| | Day 28 | 16 | 3922.34 | 2048.00 | 693.38 | 1024.00 |
| 51 | Day 0 | 15 | 73.52 | 29.18 | 27.86 | 67.03 |
| | Day 28 | 15 | 1702.38 | 2702.35 | 280.79 | 370.50 |
| 52 | Day 0 | 12 | 135.61 | 90.51 | 11.99 | 95.89 |
| | Day 28 | 12 | 2169.78 | 3251.00 | 542.45 | 542.45 |
| 53 | Day 0 | 11 | 272.65 | 128.00 | 49.74 | 105.95 |
| | Day 28 | 11 | 1922.93 | 1922.93 | 423.81 | 350.81 |
| 54 | Day 0 | 10 | 84.45 | 90.51 | 9.19 | 238.86 |
| | Day 28 | 10 | 2702.35 | 3104.19 | 222.86 | 1176.27 |
| 55 | Day 0 | 6 | 71.84 | 22.63 | 57.02 | 50.80 |
| | Day 28 | 6 | 812.75 | 3649.12 | 512.00 | 724.08 |

Table D-3 shows the numbers and percentages of participants with a ≥4-fold rise in SBA-BR titer from baseline to Day 28 for the serogroups A, C, Y, and W-135. The numbers and percentages for the serogroups A, 1028/1278 (80.4%); C, 1131/1278 (88.5%); Y, 941/1278 (73.6%); and W-135,1142/1278 (89.4%) in the TetraMenD group are comparable to those in the Menomune^{®} group, with serogroups A, 929/1099 (84.5%); C, 985/1099 (89.6%); Y, 872/1099 (79.3%); and W-135; 1036/1099 (94.3%).

**Table D-3: Number and Percentage of participants with a ≥ 4-Fold Rise from Baseline in SBA-BR Titer by Serogroups ***

| **≥ 4-fold rise in SBA-BR titer for Serogroups** | **TetraMenD** | | **Menomune^{®}** | | **% Difference (P_{Menomune} ® -P_{TetraMenD)}** | **Upper one-Sided 97.5% Confidence limit of the Difference** |
|---|---|---|---|---|---|---|
| | **n/N^{†}** | **P_{TetraMenD} ^{‡}** | **n/N** | **P_{Menomune}®** | | |
| **A** | 1028/1278 | 80.4 | 929/1099 | 84.5 | 4.1 | 7.1 |
| **C** | 1131/1278 | 88.5 | 985/1099 | 89.6 | 1.1 | 3.6 |
| **Y** | 941/1278 | 73.6 | 872/1099 | 79.3 | 5.7 | 9.1 |
| **W-135** | 1142/1278 | 89.4 | 1036/1099 | 94.3 | 4.9 | 7.1 |

| | | | | | | |
|---|---|---|---|---|---|---|
| * Testing the null hypothesis Ho: P_{Menomune}® - P_{TetraMenD} ≥0.10 versus Hₐ: P_{Menomune}® - P_{TetraMenD} < 0. 10 ^{†} n/N: n = number of participants with a ≥4-fold rise from baseline titer / N = total number of participants in the per-protocol population ^{‡}P_{TetraMenD}: percentages of participants with a ≥4-fold rise from baseline in SBA-BR post-vaccination titer from the TetraMenD group. ^{§} P_{Menomune}®: percentages of participants with a ≥4-fold rise from baseline in SBA-BR post-vaccination titer from the Menomune^{®} group. | | | | | | |

### Frequency of SBA-BR Antibody Titers 2≥ 32

The proportion of participants with SBA-BR antibody titers ≥ 32 at Day 28 after vaccination is summarized in Table D-4.

**Table D-4: Percentage and Number of Participants with an SBA Antibody Titter ≥ 32 at Day 28 Post-Vaccination (Per-protocol Population)**

| | **TetraMenD** | | **Menomune^{®}** | |
|---|---|---|---|---|
| | **% (n/N)^{†}** | **95% Cl for the percentage** | **%* _{*} (n/N)^{†}** | **95% Cl for the percentage** |
| **Serogroup A** | 100.0 | (99.77%, | 99.9 | (99.49%, |
| | (1278/1278) | 100.00%) | (1098/1099) | 100.00%) |
| **Serogroup C** | 99.5 | (98.98%, | 98.9 | (98.10%, |
| | (1272/1278) | 99.83%) | (1087/1099) | 99.43%) |
| **Serogroup Y** | 97.8 | (96.85%, | 98.9 | (98.10%, |
| | (1250/1278) | 98.54%) | (1087/1099) | 99.43%) |
| **Serogroup W-135** | 98.7 | (97.88%, | 99.2 | (98.45%, |
| | (1261/1278) | 99.22%) | (1090/1099) | 99.62%) |

| | | | | |
|---|---|---|---|---|
| *%: = n/N ^{†} n: number of participants with a titer ≥ 32 at Day 28 post-vaccination / N: total number of participants with a valid blood sample at Day 28 in this group. | | | | |

### Frequency of SBA-BR Antibody Titers ≥ 128

The proportion of participants with SBA-BR antibody titers ≥ 128 at Day 28 after vaccination is summarized in Table D-5.

**Table D-5: Percentage and Number of Participants with an SBA Antibody Titer > 128 at Day 28 Post-Vaccination (Per-protocol Population)**

| | **TetraMenD** | | **Menomune^{®}** | |
|---|---|---|---|---|
| | **%^{*} (n/N)_{†}** | **95% CI for the percentage** | **%^{*} (n/N)** | **95% Cl for the percentage** |
| **Serogroup A** | 99.8 | (99.44%, | 99.9 | (99.49%, |
| | (1276/1278) | 99.98%) | (1098/1099) | 100.00%) |
| **Serogroup C** | 98.7 | (97.97%, | 98.5 | (97.53%, |
| | (1262/1278) | 99.28%) | (108211099) | 99.1fl%) |
| **Serogroup Y** | 96.9 | (95.85%; | 98.5 | (97.53%, |
| | (12391I27S) | 97.82%) | (108211 Og9) | 99.10%) |
| **Serogroups W-135** | 97.1 | (96.03%, | 98.5 | (97.53%, |
| | (1241/1278) | 97.95 %) | (108211099) | 99.I0°/a) |

| | | | | |
|---|---|---|---|---|
| ^{.} %: n/N. † n: number of participants with a titer t 128 at Day 28 post-vaccination. ‡ N: total number of participants with a valid blood sample at Day 28 in this group. | | | | |

**Table D-6 Analysis of Treatment Effect on GMTs Adjusted by Baseline Covariate: Response of Titer Difference from Day 0 to Day 28 (per-protocol Population)^{*}**

| **Serogroup** | | **Baseline, GMT** | **Estimate of Baseline GMT Effect** | **Difference of Treatment Effect (Menomune^{®}**- TetraMenD) | **Anti-Log of Treatment Effect* (Menomune^{®}**- TetraMenD) | **95 % CI for Anti-Log of Treatment effect (Menomune^{®}** -TetraMenD) |
|---|---|---|---|---|---|---|
| **SBA Serogroups A** | | | | | | |
| | TeaMenD | 223.6 | -0.850 | 0.096 | 1.069 | (0.973, 1.175) |
| | Menomune ® | 203.9 | | | | |
| **SBA Serogroup C** | | | | | | |
| | TetraMenD | 57.2 | -0.772 | 0.130 | 1.094 | (0.965, 1.240) |
| | Menomune ® | 51.8 | | | | |
| **SBA Serogroup Y** | | | | . | | |
| | TetraMenD | 122.9 | -0.743 | 0.469 | 1.384 | (1.225, 1.563) |
| | Menomune ® | 127.4 | | | | |
| **SBA Serogroup W-135** | | | | | | |
| | TetraMenD | 33.2 | -0.766 | 0.576 | 1.491 | (1.334, 1.666) |
| | Menomune ® | 31.0 | | | | |

^{*} Anti-Log of treatment effect is calculated as 2 to the treatment effect (Menomune-Teta34eflD) power. '

### Proportion of Participants with at least a 4-fold rise in SBA-BR Antibody Titers

Table D-7 shows the proportion of participants with at 4-fold rise from baseline in Day 28 SBA antibody titers.

**Table D-7: Number and Percentage of Participants with a ? 4-Fold Rise in Day 28 SBA Antibody Titers From Baseline**

| **Test Type** | **TetraMenD** | | | Menomune^{®} | | |
|---|---|---|---|---|---|---|
| | **%^{*}** | (**n^{†}**/**N^{‡}**) | **(95% CI )** | %**^{*}** | (**n^{†}**/**N^{‡}**) | **(95% CI )** |
| **SBA (A) .** | 80.4 | (1028/1278) | (78.16%, 2.58%) | 84.5 | (929/1099) | (82.26%, 86.62%} |
| **SBA (C)** | 88.5 | (1131/1278) | (86.62%, 90.20%) | 89.6 . | {985l1099) - | (87.67%, 91.37%) |
| **SBA {Y)** | 73.6 | (941/1278) | (71.12%, 76.03%) | 79.3 | (872/1099) | (76.83%, 81.70%) |
| **SBA (W-135)** | 89.4 | (1142/1278) | (87.54%, 91.00%) | 94.3 - | (1036/1099) | (92.72%, 95.57%) |

| | | | | | | |
|---|---|---|---|---|---|---|
| _{. %:} n/N. ^{†}n: number of participants with ≥ 4-fold rise from baseline titer. ^{‡} N: number of participants with blood draws within each serogroup. | | | | | | |

### Proportion of Participants with Undetectable Titers (< 8) at Day 0 Achieving a ≥ 4-Fold Rise in Day 28 SBA-BR Antibody Titers

Table D-8 shows the proportion of participants with undetectable titers (< 8) at Day 0 Achieving a ≥ 4-Fold Rise in Day 28 SBA-BR Antibody Titers. In both treatment groups and for all vaccine serogroups, most participants with an undetectable (< 8) SBA titer at baseline achieved a ≥ 4-fold rise in Day 28 SBA titers. The proportions of participants' with an SBA titer < 8 at Day 0 who had a ≥ 4-fold rise from baseline to Day 28 ranged from 90.7% to 100.0% in the TetraMenD group and from 96.9% to 99.3% in the Menomune^{e} group.

**Table D-8: Proportion of Participants with Undetectable Titers (<8) at Day 0 Achieving a ≥4-Fold Rise in Day 28 SBA-BR Antibody Titers**

| **Test Type** | **TetraMenD** | | | **Menomune^{®}** | | |
|---|---|---|---|---|---|---|
| | **%^{*}** | **(n^{†}/N^{‡})** | **(95% Cl)** | **%^{*}** | **(n^{†}/N^{‡})** | **(95% Cl)** |
| **SBA (A)** | 100.0 | (156/156) | (98.10%, 100.00%) | 99.3 | (143/144) | (96.19%, 99.98%) |
| **SBA (C)** | 99.4 | (341/343) | (97.91%, 99.93%) | 97.7 | (297/304) | (95.31%, 99.07%) |
| **SBA (Y)** | 90.7 | (253/279) | (86.64%, 93.82%) | 96.9 | (221/228) | (93.78%, 98.76%) |
| **SBA (W-135)** | 96.5 | (359/372) | (94.10%, 98.13%) | 99.1 | (325/328) | (97.35%, 99.81%) |

| | | | | | | |
|---|---|---|---|---|---|---|
| *%:n/N. ^{†} n; number of participants with titers < 1:8 at Day 0 and titers≥ 1:32 at Day 28 within each serogroup ^{‡}N: number of participants with titers < 1:8 at Day 0 within each serogroup. | | | | | | |

Table D-9 shows the SBA GMTs at baseline and on Day 28 after vaccination and the fold rises in SBA GMTs.

**Table D-9: Summary of Geometric Mean of Antibody Titers (GMT) and Fold Rise of GMT by Serogroup (Per-protocol Population)**

| **Test Type** | **Param eter*** | **Bleed** | **TetraMenD** | | | **Menomune^{®}** | | |
|---|---|---|---|---|---|---|---|---|
| | | | **N^{†}** | **GMT** | **(95% Cl)^{‡}** | **N^{†}** | **GMT** | **(95% Cl)^{‡}** |
| **Serogroup A SBA** | Titer | Day 0 | 1279 | 223.6 | (199.86, 250.08) | 1099 | 203.9 | (180.53, 230.23) |
| | | Day 28 | 1278 | 3896.6 | (3646.33, 4164.11) | 1099 | 4108. 9 | (3827.43, 4411.15) |
| | Fold Rise | Day 28 | 1278 | 16.0 | (14.39, 17.84) | 1099 | 18.4 | (16.39, 20.67) |
| **Serogroup C SBA** | Titer | Day 0 | 1279 | 57.2 | (50.50, 64.73) | 1099 | 51.8 | (45.47, 59.11) |
| | | Day 28 | 1278 | 3235.2 | (2958.46, 3537.76) | 1099 | 3463. 4 | (3143.05, 3816.34) |
| | Fold Rise | Day 28 | 1278 | 47.1 | (41.74, 53.05) | 1099 | 55.1 | (48.53, 62.67) |
| **Serogroup Y SBA** | Titer | Day 0 | 1279 | 122.9 | (108.89, 138.72) | 1099 | 127.4 | (111.97, 145.03) |
| | | Day 28 | 1278 | 1751.8 | (1598.14, 1920.30) | 1099 | 2446. 7 | (2235.36, 2677.93) |
| | Fold Rise | Day 28 | 1278 | 12.3 | (10.97, 13.68) | 1099 | 16.6 | (14.68, 18.83) |
| **Serogroup W-135 SBA SBA** | Titer | Day 0 | 1279 | 33.2 | (29.95, 36.73) | 1099 | 31.0 | (27.90, 34.46) |
| | | Day 28 | 1278 | 1270.7 | (1171.59, 1378.22) | 1099 | 1865. 5 | (1717.28, 2026.48) |
| | Fold Rise | Day 28 | 1278 | 31.4 | (28.35, 34.70) | 1099 | 48.9 | (44.07, 54.30) |

| | | | | | | | | |
|---|---|---|---|---|---|---|---|---|
| * Titer or fold-rise, where fold rise = titer at Day 28 / Titer at Day 0 ^{†} N: number of participants with blood draws within each serogroup. Note: One Participant did not have a second blood sample done ^{‡} 95 % Cl for the GMT is calculated based on an approximation to the normal distribution. | | | | | | | | |

Twenty-eight to 56 days after receiving the study vaccination, TetraMenD, the majority of participants experience a ≥4-fold rise in the SBA-BR antibody titer for each of then serogroups contained in the vaccine. The percentages of TetraMenD recipients obtaining a 4-fold rise in antibody titer are 80.4%, 88.5%, 73.6 %, and 89.4% for serogroups A, C, Y, and W-135, respectively. Higher pre-vaccination antibody levels are observed for serogroup Y than for C or W-135. This may be related to the fact that serogroup.Y is currently the most common serogroup associated with invasive meningococcal disease in this age group in the U.S. and that natural exposure to this serogroup may be more common. Higher circulating antibody levels reflect recent natural exposure and may reduce the proportion of vaccine recipients exhibiting 4-fold or higher antibody responses. This appears to be the case for serogroup Y responses when compared to other serogroups. The 4-fold rise for serogroup Y is 73.6% compared with 88.5% for serogroup C and 89.4% for serogroup W-135. High pre-vaccination antibody levels are also observed for serogroup A. This may be the result of intermittent exposure over a prolonged period of time to several naturally occurring cross-reacting antigens.

To further evaluate the impact of pre-existing titers and to investigate the rate of seroconversion (as defined by the proportion of vaccine recipients who achieve a 4-fold rise in antibody titer when the pre-vaccination titer for any serogroup is< 1:8),a separate analysis is performed on participants who had pre-vaccination antibody titers of < 1:8 to any one of the 4 serogroup contained in the vaccine. A titer of < 1:8 by the SBA assay using baby rabbit as the complement source is considered to represent an undetectable level of circulating antibody. When participants are evaluated using this criterion, it is observed that there is a 100% seroconversion rate for serogroup A, 99.4% for serogroup C, 96.5% for serogroup W-135, and 90.7% for serogroup Y after vaccination with TetraMenD.

As previously discussed in another Study herein, based on observations in military recruits, Goldschneider proposed that a minimum titer of ≥ 1:4 using an SBA assay with a human complement source correlated with protection from invasive disease against Serogroup C. Baby rabbit complement is suggested as an alternative source, but meningococci appear to be more sensitive to the baby rabbit complement than human complement, resulting in higher measured antibody titers. Several authors have suggested that titers ≥ 1:128 using the baby rabbit complement assay are predictive of protection while titers of < 1:8 are predictive of susceptibility at least for serogroup C. Although this level may be appropriate when evaluating polysaccharide vaccines, it may not be applicable for conjugate vaccines. Borrow suggested that, in subjects receiving a monovalent C conjugate vaccine who demonstrated post vaccination SBA titers between 8 and 64, the demonstration of a memory response using a reduced dose (10µg) of a meningococcal polysaccharide vaccine given several months later showed that these individuals are also protected, having achieved an antibody level ≥1:128. When this criterion is applied to all the serogroups contained in the vaccine, the percentages of participants receiving TetraMenD who achieve a post-vaccination SBA-BR titer ≥ 1:128 are 99.8%, 98.7%, 96.9%, and 97.1% for serogroups A, C, Y, and W-135, respectively.

### Example 13 Study E Td Booster Study in Children Aged 10 to 18

This study compares the tetanus and diphtheria toxoid (Td) booster response in the group receiving the experimental tetravalent Meningococcal Diphtheria Conjugate vaccine, TetraMenD, concomitantly with Td to the response in the group receiving Td with placebo, as measured by the proportion of participants who have an acceptable response in their respective tetanus and diphtheria titers. An acceptable response is defined as, 28 days following vaccination, at least a 4-fold rise from baseline in participants with a predefined low pre-vaccination titer and at least a 2-fold rise from baseline in participants with a predefined high pre-vaccination titer.

To compare the antibody response for serogroups A, C, Y, and W-135 in TetraMenD when administrated concomitantly with Td to the response when TetraMenD is administrated 28 days following Td vaccine, as measured by the proportion of participants with at least a 4-fold rise in titer to each serogroup.

This is a randomized, modified double-blind, active-control multi-center trial, with a total of 1024 participants randomized to one of two treatment groups: A and B.

| | **Day 0** | **Day 28** | **Day 56** |
|---|---|---|---|
| | V1 | V2 | V3 |
| **Group A** | BS-1 Td + TetraMenD | BS-2 Placebo | BS-3 |
| **Group B** | BS-1 Td + Placebo | BS-2 TetraMenD | BS-3 |

The age range of 11 to 17 years is chosen to capture those individuals who would normally receive Td vaccine as part of the routine childhood immunization schedule. In addition, this age range has been identified as high risk for development of invasive meningococcal disease and would most likely be candidates for vaccination with the meningococcal conjugate vaccine once licensed. In order to properly evaluate safety, a modified double-blind design using a placebo control is utilized. For the first visit, the vaccination nurse is unblinded and administered the vaccines in each arm according to protocol; TetraMenD (IM) or placebo in the right arm and Td in the left. For the second visit, each treatment group received the vaccine in the left arm. The evaluation nurse is blinded monitored local and systemic reactions and adverse events.

The age range of 11 to 17 years is chosen to capture those individuals who would normally receive Td vaccine as part of the routine childhood immunization schedule. In addition, this age range has been identified as high risk for development of invasive meningococcal disease and would most likely be candidates for vaccination with the meningococcal conjugate vaccine once licensed.

Blood specimens (at least 5 mL whole blood) for serologic testing are drawn on Day 0 prior to vaccination (baseline) and at Day 28 post-vaccination 1. There is a third blood draw for participants 28 days after visit 2. At each of these time points, sera are assayed for meningococcal serogroups A, C, Y, and W-135, anti-diphtheria antibody and anti-tetanus antibody.

To evaluate antibody function in recipients of TetraMenD, all available specimens are assayed for SBA using baby rabbit complement (SBA-BR) against each vaccine serogroup. One immunologic endpoint is the proportion of participants in each treatment group with a ≥4-fold rise in SBA-BR titerAnti-diphtheria antibody levels are measured by the ability of the test sera to protect Vero cells from a diphtheria toxin challenge. Anti-tetanus antibody levels are measured by an indirect Enzyme Linked Immunosorbent Assay (ELISA).

This study compares the antibody responses to TetraMenD for serogroups A, C, Y, and W-135 as measured by the GMTs in participants from an earlier study, Study C, who receive one dose of TetraMenD to the responses in participants who receive TetraMenD. administered concomitantly with Td and 28 days following Td vaccination.

Serum specimens for serologic analysis are obtained at baseline (Day 0) prior to vaccination and at Day 28 (window: +28 days) and 6 months after vaccination. Antibody titers to tetanus toxoid and diphtheria toxoid (Td) vaccine are measured pre- and 28 days post vaccination.

SBA-BR antibody titers for *N. meningitidis* serogroup A, C, Y, and W-135 are measured for all available serum specimens pre- and 28 days post vaccination. Overall, the safety profile of Group A and Group B are comparable. The results of this Study are summarized in the following Tables.

**Table E-1 summarizes GMT levels bv Subject Age and Serogroup.**

| **1) Table E-1 Summary of GMT by Subject Age and Serogroup** | | | | | | | |
|---|---|---|---|---|---|---|---|
| **Age** | **Dose TetramenD** | **No. of Subjects** | **Blood Day** | **GMT** | **C GMT** | **W GMT** | **Y GMT** |
| 10 | 1 µg | 1 | 0 | 2048.00 | 512.00 | 512.00 | 256.00 |
| | 4 µg | 1 | 28 | 8192.00 | 1024.00 | 4096.00 | 1024.00 |
| | 10 µg | 1 | 56 | 32768.00 | 4096.00 | 16384.00 | 8192.00 |
| 11 | 1 µg | 273 | 0 | 186.38 | 63.35 | 23.06 | 140.97 |
| | 4 µg | 267 | 28 | 2331.86 | 513.33 | 339.73 | 775.64 |
| | 10 µg | 265 | 56 | 10668.96 | 2005.59 | 2285.81. | 2203.62 |
| 12 | 1 µg | 236 | 0 | 185.87 | 56.24 | 27.23 | 138.56 |
| | 4 µg | 229 | 28 | 1672.07 | 516.67 | 280.33 | 752.00 |
| | 10 µg | 226 | 56 | 10030.01 | 2522.93 | 2164.24 . | 2231.65 |
| 13 | 1 µg | 172 | 0 | 219.65 | 60.25 | 20.46 | 128.00 |
| | 4 µg | 170 | 28 | 2562.84 | 664.66 | 372.52 | 898.74 |
| | 10 µg | 168 | 56 | 10493.02 | 2700.12 | 2346.72 | 2289.34 |
| 14 | 1 µg | 128 | 0 | 326.64 | 65.05 | 30.81 | 107.63 |
| | 4 µg | 126 | 28 | 1896.19 | 597.26 | 388.88 | 752.50 |
| | 10 µg | 126 | 56 | 9044.70 | 2375.94 | 2248.77 | 1885.79 |
| 15 | 1 µg | 101 | 0 | 223.17 | 83.07 | 47.00 | 114.69 |
| | 4 µg | 95 | 28 | 2352.53 | 716.20 | 504.58 | 776.05 |
| | 10 µg | 94 | 56 | 9424.03 | 4575.06 | 2356.01 | 1988.48 |
| 16 | 1 µg | 71 | 0 | 393.36 | 46.83 | 24.11 | 70.56 |
| | 4 µg | 70 | 28 | 2399.59 | 783.77 | 450.16 | 512.00 |
| | 10 µg | 69 | 56 | 8527.88 | 3154.48 | 1908.93 | 1833.75 |
| 17 | 1 µg | 35 | 0 | 358.47 | 107.10 | 28.41 | 79.58 |
| | 4 µg | 35 | 28 | 2173.36 | 403.70 | 156.70 | 411.78 |
| | 10 µg | 34 | 56 | 7398.11 | 3340.58 | 141.8.93 | 1390.30 |

**Table E-2 shows the numbers and proportions of participants with at least a 4-fold or 2-fold rise in tetanus and diphtheria antibody on Day 28.**

| **Table E-2 Numbers and Proportions of Participants with at least a 4-fold or 2-fold rise in Tetanus and Diphtheria Antibody on Day 28** | | | | |
|---|---|---|---|---|
| **Antigen Response** | **Td+TetraM enD, Placebo n/N (%=Pa)** | **Td+Placebo, TetraMenD n/N (%=Pb)** | **Difference (Pb-Pa)** | **95% Cl for the % Difference** |
| **Tetanus** | | | | |
| **2-Fold (Pre-titer >5.3 IU/mL)** | 0/24 | 2/23 | | |
| | (0.00) | (8.70) | | |
| **4-Fold (Pre-titer 55.3 IU/mL)** | 399/439 | 417/448 | | |
| | (90.89) | (93.08) | | |
| **Total Responders** | 399/463 | 4191471 | 2.78 | (-1.45,7.01) |
| | (86.18) | (88.96) | | |
| **Diphtheria** | | | | |
| **2-Fold(Pre-titer >1.28 IU/mL)** | 44/47 | 42/49 | | |
| | (93.62) | (85.71) | | |
| **4-Fold(Pre-titer ≤ 1.28 IU/mL)** | 419/419 | 416/425 | | |
| | (100.00) | (97.88) | | |
| **Total Responders** | 463/466 | 458/474 | -2.73 | (-4.51,-0.95) |
| | (99.36) | (96.62) | | |

### Tetanus and Diphtheria Antibody Titers and SBA Antibody Titers for Serogroups A, C, Y, and W-135

Table E-2 shows the numbers and proportions of participants with at least a 4-fold or 2-fold rise in tetanus and diphtheria antibody on Day 28. The differences in the proportions are: 2.78 and -2.73 for tetanus and diptheria, respectively.

| **Table E-2: Total Number and Proportion of Participants with at least 4-Fold or 2-Fold Rise Response in Tetanus and Diphtheria Antibody on Day 28 Following the Tetanus and Diphtheria Vaccination, Primary Hypothesis 1(per-Protocol Population)** | | | | |
|---|---|---|---|---|
| **Antigen Response** | **Td+TetraMenD,Placebo n/N (%=Pa)** | **Td+Placebo,TetraMenD n/N (%=Pb)** | **Difference (Pb-Pa)** | **95% Cl for the % Difference** |
| **Tetanus** | | | | |
| **2-Fold(Pre-titer> >5.3 IU/mL)** | 0/24 0.00) | 2/23 (8.70) | | |
| **4-Fold (Pre-titer≤5.3 IU/ML)** | 399/439 (90.89) | 417/448 (93.08) | | |
| **Total Responders** | 399/463 (86.18) | 419/471 (88.96) | 2.78 | (-1.45,7.01) |

| **Table E-2: Total Number and Proportion of Participants with at least 4-Fold or 2-Fold Rise Response in Tetanus and Diphtheria Antibody on Day 28 Following the Tetanus and Diphtheria Vaccination, Primary Hypothesis 1(Per-Protocol Population)** | | | | |
|---|---|---|---|---|
| **Antigen Response** | **Td+TetraMenD,Placebo n/N (%=Pa)** | **Td+Placebo,TetraMenD n/N (%=Pb)** | **Difference (Pb-Pa)** | **95% Cl for the % Difference** |
| **Diphtheria** | | | | |
| **2-Fold(Pre-titer >1.28 IU/mL)** | 44/47 (93.62) | 42/49(85.71) | | |
| **4-Fold(Pre-titer ≤ 1.28 IU/mL)** | 419/419 (100.00) | 416/425 (97.88) | | |
| **Total Responders** | 463/466 (99.36) | 458/474 (96.62) | -2.73 | (-4.51,-0.95) |

**Table E-3 shows the numbers and proportions of participants with at least a 4-fold rise in antibody titer to serogroups A, C, Y, and W-135 on Day 28.**

| **Table E-3: Number and Proportion of Participants with a ≥4-Fold Rise in SBA-BR Titer on Day 28 Following the TetraMenD Vaccination, Primary Hypothesis 2 (per-Protocol Population)** | | | | |
|---|---|---|---|---|
| **Serogroup** | **Td+TetramenD,Placebo n/N (%=Pa)** | **Td+Placebo,TetraMenD n/N (%=Pb)** | **Difference (Pb-Pa)** | **95% Cl for the % Difference** |
| **Serogroup A** | 419/466 (89.91) | 433/478 (90.59) | 0.67 | (-3.11,4.46) |
| **Serogroup C** | 424/466 (90.99) | 394/478 (82.43) | -8.56 | (-12.85,-4.27) |
| **Serogroup Y** | 399/466 (85.62) | 311/478(65.06) | -20.56 | (-25.89,-15.23) |
| **Serogroup W-135** | 448/466 (96.14) | 419/478 (87.66) | -8.48 | (-11.91, 5.05) |

**Table E-4 shows the number of participants with high diphtheria and tetanus titers at baseline and the number and proportion of participants with a 2-fold rise on Day 28.**

| **Table E-4: Number (%) of Participants with High Diphtheria and Tetanus Pre-Titers at Baseline and Number and Proportion of Participants with 2-Fold Rise on Day 28 Per-Protocol Population** | | | | | | | | |
|---|---|---|---|---|---|---|---|---|
| | **Td+TetramenD,Placebo** | | | | **Td+Placebo,TetraMenD** | | | |
| | **Baseline Titer** | | **≥ 2-Fold Rise** | | **Baseline Titer** | | **≥ 2-Fold Rise** | |
| | **n/N** | **%** | **n/N** | **%** | **n/N** | **%** | **n/N** | **%** |
| **Tetanus > 5.3 IU/ml** | 24/468 | 5.13 | 0/24 | 0.00 | 23/472 | 4.87 | 2/23 | 8.70 |
| **Diphtheria** > **1.28 IU/ml** | 47/469 | 10.02 | 44/47 | 93.62 | 49/476 | 10.29 | 42/49 | 85.71 |

**Table E-5 shows the number of participants with low diphtheria and tetanus titers at baseline and the number and proportion of participants with a 4-fold rise on Day 28.**

| **Table E-5: Number (%) of Participants with Low Diphtheria and Tetanus Pre-Titers at Baseline and Number and Proportion of Participants with 4-Fold Rise on Day 28- Per Protocol Population** | | | | | | | | |
|---|---|---|---|---|---|---|---|---|
| | **Td+TetramenD,Placebo** | | | | **Td+Placebo,TetraMenD** | | | |
| | **Baseline Titer** | | ≥**4-fold Rise** | | **Baseline Titer** | | ≥**4-Fold Rise** | |
| | **n/N** | **%** | **/N** | **%** | **n/N** | **%** | **n/N** | **%** |
| **Tetanus ≤5.3 lU/ml** | 444/468 | 94.87 | 399/439 | 90.89 | 449/472 | 95.13 | 417/448 | 93.08 |
| **Diphtheria ≤1.28 IU/Ml** | 422/469 | 89.98 | 419/419 | 100.00 | 427/476 | 89.71 | 416/425 | 97.88 |

**Table E-6 shows the number and proportion of participants with a titer ≥1.0 IU/ml in tetanus and diphtheria antibody on Day 28 following tetanus and diphtheria vaccination given concomitantly with TetraMenD or Placebo.**

| **Table E-6:Number and Proportion of Participants with Titer ≥1.0 IU/ml in Tetanus and Diphtheria Antibody on Day 28 Following Tetanus and Diphtheria Vaccination, (Per-Protocol Population)** | | | | |
|---|---|---|---|---|
| | **Td+TetraMenD,Placebo n/N (%=Pa)** | **Td+Placebo,TetraMenD n/N (%=Pb)** | **Difference (Pb-Pa)** | **95% Cl for the % Difference** |
| **Tetanus > 1.0 IU/ml** | 461/465 (99.14) | 470/477 (98.53) | -0.61 | (-1.97,0.76) |
| **Diphtheria ≥ 1.0 IU/ml** | 467/467(100.00) | 474/476 (99.58) | -0.42 | (-1.00,0.16) |

**Table E-7 shows the geometric mean antibody titers(GMTs) for tetanus and diphtheria on Day 28 following tetanus and diphtheria vaccination (given concomitantly with TetraMenD or Placebo).**

| **Table E-7: Comparison of Geometric Mean Antibody Titers(GMTs) for Tetanus and Diphtheria on Day 28 Following Tetanus and Diphtheria Vaccination, (Per-Protocol Population) [1]** | | | | | | |
|---|---|---|---|---|---|---|
| | **Td+TetramenD,Placebo** | | **Td+Placebo,TetramenD** | | | |
| | **GMTa** | **(95% Cl)** | **GMTb** | **(95% Cl)** | **GMT Ratio GMTb/GMTa** | **95% Cl for GMT Ratio** |
| **Tetanus** | 11.46 | (10.79,12.18) | 13.56 | (12.73,14.44) | 1.18 | (1.08,1.29) |
| **Diphtheria** | 304.69 | (221.69,418.78) | 10.60 | (9.23,12.18) | 0.03 | (0.02,0.05) |

**Table E-8 shows the geometric mean antibody titers(GMTs) for SBA-BR for serogroups A, C, Y, and W 135 on Day 28 post TetraMenD vaccination. The GMT.ratios are 0.92, 0.42, 4.39, and 0.32 for serogroups A, C, Y, and W-135, respectively..**

| **Table E-8: Comparison of Geometric Mean Antibody Titers(GMTs) for SBA-BR on Day 28 Following the TetraMenD Vaccination, (Per-Protocol Population)[11** | | | | | | |
|---|---|---|---|---|---|---|
| | **Td+TetraMenD,Placebo** | | **Td+Placebo,TetraMenD** | | | |
| | **GMTa** | **(95%CI)** | **GMTb** | **(95% CI)** | **GMT Ratio GMTb/GMT a** | **95% CI for GMT Ratio** |
| **Serogroup A** | 11321.8 | (10173.2,12600.0) | 10391.4 | (9523.1,11338.8) | 0.92 | (0.8,1.1) |
| **Serogroup C** | 5042.0 | (4389.4,5791.7) | 2136.0 | (1810.8,2519.4) | 0.42 | (0.3,0.5) |
| **Serogroup Y** | 3387.3 | (2978.2,3852.5) | 1331.3 | (1170.2,1514.6) | 0.39 | (0.3,0.5) |
| **Serogroup W-135** | 4175.8 | (3702.1,4710.1) | 1339.1 | (1161.8,1543.4) | 0.32 | (0.3,0.4) |

**Table E-9 shows the geometric mean antibody titers(GMTs) for SBA-BR for serogroups A, C, Y, and W 135 on Day 28 post TetraMenD vaccination in the Td + TetraMenD, Placebo group and the corresponding results from study MTA02. The GMT ratios are 0.48, 0.38, 0.34, and (0.39 for serogroup A, C, Y, and W-135, respectively.**

| **Table E-9: Comparison of Geometric Mean Antibody Titers(GMTs) for SBA-BR on Day 28 Following the TetraMenD Vaccination in Group Td+TetraMenD,Placebo to the Corresponding Results from Study C, (Per-Protocol Population)** | | | | | | |
|---|---|---|---|---|---|---|
| | **Td+TetraMenD,Platebo** | | **Study C** | | | |
| | **GMTa** | **95%CI)** | **GMT Study C** | **(95% CI)** | **GMT Ratio GMTmta02/ -GMTa** | **95% CI for GMT Ratio** |
| **Serogroup A** | 11321.8 | (10173.2,12600.0) | 5483.2 | (4920.1,6110.7) | 0.48 | (0.4,0.6) |
| **Serogroup C** | 5042.0 | (4389.4,5791.7) | 1924.4 | (1662.1,2228.0) | 0.38 | (0.3,0.5) |
| **Serogroup Y,** | 3387.3 | (2978.2,3852.5) | 1322.3 | (11G1.9,1504.5) | 0.39 | (0.3,0.5) |
| **Serogroup W-135** | 4175.8 | (3702.1,4710.1) | 1407.2 | (1232.1,1607.3) | 0.34 | (0.3,0.4) |

**Table E-10 shows the geometric mean antibody titers (GMTs) for SBA-BR for serogroups A, C, Y, and W-135 on Day 28 post TetraMenD vaccination in the Td + Placebo, TetraMenD group and the corresponding results from study MTA02. The GMT ratios are 0.53, 0.90, 0.99, and 1.05 for serogroups A, C, Y, and W-135, respectively.**

| **Table E-10: Comparison of Geometric Mean Antibody Titers (GMTs) for SBA-BR on Day 28 Following the TetraMenD Vaccination in Group B to the Corresponding Results from Study C, Observational Hypothesis(Per-Protocol Population)[1]** | | | | | | |
|---|---|---|---|---|---|---|
| | **Td+Placebo,TetraMenD** | | **Study C** | | | |
| | **GMTb** | **(95%CI)** | **GMT Study C** | **(95%CI)** | **GMT Ratio GMTmta02/ GMTb** | **95% CI for GMT Ratio** |
| **Serogroup A** | 10391.4 | (9523.1,11338.8) | 5483.2 | (4920.1,6110.7) | 0.53 | (0.5,0.6) |
| **Serogroup C** | 2136.0 | (1810.8,2519.4) | 1924.4 | (1662.1,2228.0) | 0.90 | (0.7,1.1) |
| **Serogroup Y** | 1331.3 | (1170.2,1514.6) | 1322.3 | (1161.9,1504.8) | 0.99 | (0.8,1.2) |
| **Serogroup W-135** | 1339.1 | (1161.8,1543.4) | 1407.2 | (1232.1,1607.3) | 1.05 | (0.9,1.3) |

Table E-11 show the distribution of SBA-BR antibody titers on Day 0 and Day 28 after the TetraMenD Vaccination by serogroup for the Per-Protocol Population (SBA-BR Titers <8 to 1024). Table E-12 shows the distribution of SBA-BR antibody titers on Day 0 and Day 28 after the TetraMenD Vaccination by serogroup for the Per-Protocol Population (SBA-BR Titers 2048 to 524288)

| **Table E-11: Distribution of SBA-BR Antibody Titers on Day 0 and Day 28 After the TetraMenD Vaccination by Serogroup (Per-Protocol Population) (SBA-BR Titers <8 to 1024)** | | | | | | | | | | | | |
|---|---|---|---|---|---|---|---|---|---|---|---|---|
| | | | | **SBA Titers** | | | | | | | | |
| | | | | **<8** | **8** | **16** | **32** | **64** | **128** | **256** | **512** | **1024** |
| **Test Type** | **Test Date** | **Grou P** | **N** | **n %** | **n %** | **n %** | **n %** | **n %** | **n %** | **n %** | **n %** | **n %** |
| **Sero-group A** | Day 0 | Group | 470 | 90 | 13 | 5 | 2 | 9 | 25 | 60 | 68 | 97 |
| | | A | | 19.1 | 2.8 | 1.1 | 0.4 | 1.9 | 5.3 | 12.8 | 14.5 | 20.6 |
| | | Group | 478 | 53 | 6 | 2 | 0 | 5 | 36 | 74 | 73 | 103 |
| | | B | | 11.1 | 1.3 | 0.4 | 0.0 | 1.0 | 7.5 | 15.5 | 15.3 | 21.5 |
| | Day 28 | Group | 470 | 0 | 0 | 0 | 0 | 0 | 3 | 3 | 7 | 10 |
| | | A | | 0.0 | 0.0 | 0.0 | 0.0 | 0.0 | 0.6 | 0.6 | 1.5 | 2.1 |
| | | Group | 478 | 0 | 0 | 0 | 0 | 0 | 0 | 1 | 4 | 6 |
| | | B | | 0.0 | 0.0 | 0.0 | 0.0 | 0.0 | 0.0 | 0.2 | 0.8 | 1.3 |
| **Sero-group C** | Day 0 | Group | 470 | 151 | 27 | 15 | 27 | 18 | 31 | 38 | 52 | 64 |
| | | A | | 32.1 | 5.7 | 3.2 | 5.7 | 3.8 | 6.6 | 8.1 | 11.1 | 13.6 |
| | | Group | 478 | 153 | 31 | 20 | 13 | 12 | 55 | 49 | 42 | 45 |
| | | B | | 32.0 | 6.5 | 4.2 | 2.7 | 2.5 | 11.5 | 10.3 | 8.8 | 9.4 |
| | Day 28 | Group | 470 | 1 | 0 | 0 | 2 | 1 | 5 | 11 | 26 | 39 |
| | | A | | 0.2 | 0.0 | 0.0 | 0.4 | 0.2 | 1.1 | 2.3 | 5.5 | 8.3 |
| | | Group | 478 | 2 | 2 | 2 | 9 | 4 | 20 | 35 | 52 | 67 |
| | | B | | 0.4 | 0.4 | 0.4 | 1.9 | 0.8 | 4.2 | 7.3 | 10.9 | 14.0 |
| **Serogroup Y** | Day 0 | Group | 470 | 101 | 5 | 6 | 22 | 30 | 53 | 81 | 67 | 52 |
| | | A | | 21.5 | 1.1 | 1.3 | 4.7 | 6.4 | 11.3 | 17.2 | 14.3 | 11.1 |
| | | Group | 478 | 93 | 1 | 2 | 8 | 20 | 84 | 69 | 71 | 69 |
| | | B | | 19.5 | 0.2 | 0.4 | 1.7 | 4.2 | 17.6 | 14.4 | 14.9 | 14.4 |
| | Day 28 | Group | 470 | 1 | 1 | 1 | 2 | 3 | 7 | 12 | 23 | 65 |
| | | A | | 0.2 | 0.2 | 0.2 | 0.4 | 0.6 | 1.5 | 2.6 | 4.9 | 13.8 |
| | | Group | 478 | 4 | 3 | 1 | 2 | 5 | 20 | 38 | 69 | 85 |
| | | B | | 0.8 | 0.6 | 0.2 | 0.4 | 1.0 | 4.2 | 7.9 | 14.4 | 17.8 |
| **Serogroup W-135** | Day 0 | Group | 470 | 205 | 30 | 17 | 33 | 37 | 41 | 39 | 31 | 17 |
| | | A | | 43.6 | 6.4 | 3.6 | 7.0 | 7.9 | 8.7 | 8.3 | 6.6 | 3.6 |
| | | Group | 478 | 213 | 29 | 9 | 14 | 26 | 69 | 48 | 32 | 24 |
| | | B | | 44.6 | 6.1 | 1.9 | 2.9 | 5.4 | 14.4 | 10.0 | 6.7 | 5.0 |
| | Day 28 | Group | 470 | 1 | 0 | 0 | 0 | 1 | 4 | 7 | 24 | 62 |
| | | A | | 0.2 | 0.0 | 0.0 | 0.0 | 0.2 | 0.9 | 1.5 | 5.1 | 13.2 |
| | | Group | 478 | 7 | 3 | 1 | 2 | 1 | 21 | 42 | 72 | 87 |
| | | B | | 1.5 | 0.6 | 0.2 | 0.4 | 0.2 | 4.4 | 8.8 | 15.1 | 18.2 |
| Group A: Vaccination 1 = Td + TetraMenD Vaccination 2 = Placebo Group B: Vaccination 1= Td + Placebo Vaccination 2 = TetraMenD | | | | | | | | | | | | |

| **Table E-12: Distribution of SBA-BR Antibody Titers on Day 0 and Day 28 After the TetraMenD Vaccination by Serogroup (Per-Protocol Population) (SBA-BR Titers 2048 to 524288)** | | | | | | | | | | | | |
|---|---|---|---|---|---|---|---|---|---|---|---|---|
| | | | | **SBA Titers** | | | | | | | | |
| | | | | **2048** | **4096** | **8192** | **16384** | **32768** | **65536** | **131072** | **524288** | **Missing** |
| **Test Type** | **Test Date** | **Group** | **N** | **n %** | **n %** | **n %** | **n %** | **n %** | **n %** | **n %** | **n %** | **n %** |
| **Serogroup A** | **Day 0** | A | 470 | 70 | 16 | 10 | 3 | 1 | 0 | 0 | 0 | 1 |
| | | | | 14.9 | 3.4 | 2.1 | 0.6 | 0.2 | 0.0 | 0.0 | 0.0 | 0.2 |
| | | B | 478 | 75 | 29 | 13 | 4 | 5 | 0 | 0 | 0 | 0 |
| | | | | 15.7 | 6.1 | 2.7 | 0.8 | 1.0 | 0.0 | 0.0 | 0.0 | 0.0 |
| | **Day 28** | A | 470 | 29 | 60 | 109 | 102 | 117 | 17 | 10 | 0 | 3 |
| | | | | 6.2 | 12.8 | 23.2 | 21.7 | 24.9 | 3.6 | 2.1 | 0.0 | 0.6 |
| | | B | 478 | 38 | 75 | 112 | 147 | 83 | 7 | 5 | 0 | 0 |
| | | | | 7.9 | 15.7 | 23.4 | 30.8 | 17.4 | 1.5 | 1.0 | 0.0 | 0.0 |
| **Serogroup C** | **Day 0** | A | 470 | 36 | 4 | 2 | 3 | 1 | 0 | 0 | 0 | 1 |
| | | | | 7.7 | 0.9 | 0.4 | 0.6 | 0.2 | 0.0 | 0.0 | 0.0 | 0.2 |
| | | B | 478 | 32 | 14 | 8 | 2 | 2 | 0 | 0 | 0 | 0 |
| | | | | 6.7 | 2.9 | 1.7 | 0.4 | 0.4 | 0.0 | 0.0 | 0.0 | 0.0 |
| | **Day 28** | A | 470 | 80 | 79 | 77 | 74 | 51 | 10 | 10 | 1 | 3 |
| | | | | 17.0 | 16.8 | 16.4 | 15.7 | 10.9 | 2.1 | 2.1 | 0.2 | 0.6 |
| | | B | 478 | 79 | 62 | 59 | 36 | 35 | 5 | 9 | 0 | 0 |
| | | | | 16.5 | 13.0 | 12.3 | 7.5 | 7.3 | 1.0 | 1.9 | 0.0 | 0.0 |
| **Serogroup Y** | **Day 0** | A | 470 | 37 | 9 | 4 | 2 | 0 | 0 | 0 | 0 | 1 |
| | | | | 7.9 | 1.9 | 0.9 | 0.4 | 0.0 | 0.0 | 0.0 | 0.0 | 0.2 |
| | | B | 478 | 35 | 15 | 6 | 5 | 0 | 0 | 0 | 0 | 0 |
| | | | | 7.3 | 3.1 | 1.3 | 1.0 | 0.0 | 0.0 | 0.0 | 0.0 | 0.0 |
| | **Day 28** | A | 470 | 74 | 114 | 77 | 56 | 28 | 1 | 2 | 0 | 3 |
| | | | | 15.7 | 24.3 | 16.4 | 11.9 | 6.0 | 0.2 | 0.4 | 0.0 | 0.6 |
| | | B | 478 | 113 | 73 | 47 | 15 | 3 | 0 | 0 | 0 | 0 |
| | | | | 23.6 | 15.3 | 9.8 | 3.1 | 0.6 | 0.0 | 0.0 | 0.0 | 0.0. |
| **Serogroup W 135** | **Day 0** | A | 470 | 15 | 3 | 1 | 0 | 0 | 0 | 0 | 0 | 1 |
| | | | | 3.2 | 0.6 | 0.2 | 0.0 | 0.0 | 0.0 | 0.0 | 0.0 | 0.2 |
| | | B | 478 | 11 | 1 | 2 | 0 | 0 | 0 | 0 | 0 | 0 |
| | | | | 2.3 | 0.2 | 0.4 | 0.0 | 0.0 | 0.0 | 0.0 | 0.0 | 0.0 |
| | **Day 28** | A | 470 | 85 | 90 | 77 | 74 | 37 | 3 | 2 | 0 | 3 |
| | | | | 18.1 | 19.1 | 16.4 | 15.7 | 7.9 | 0.6 | 0.4 | 0.0 | 0.6 |
| | | B | 478 | 109 | 60 | 39 | 19 | 12 | 2 | 1 | 0 | 0 |
| | | | | 22.8 | 12.6 | 8.2 | 4.0 | 2.5 | 0.4 | 0.2 | 0.0 | 0.0 |
| Group A: Vaccination 1 = Td + TetraMenD Vaccination 2 = Placebo Group B: Vaccination 1 = Td + Placebo Vaccination 2 = TetraMenD | | | | | | | | | | | | |

| **Table E-13: Summary of Antibody Titers: Distribution of Tetanus and Diphtheria Titers (Per-Protocol Population)[1]** | | | | | |
|---|---|---|---|---|---|
| | | **Td+TetraMenD, Placebo** | | **Td+Placebo, TetraMenD** | |
| **Antibody Titer** | **Test Date** | **n/N** | **(%)** | **n/N** | **(%)** |
| **Tetanus** | | | | | |
| **< 0.1 IU/ml** | Day 0 | 51468 | (1.1) | 31472 | (0.6) |
| | Day 28 | 0/465 | (0.0) | 0/477 | (0.0) |
| | | | | | |
| **≥0.1 - < 1.0 IU/ml** | Day 0 | 281/468 | (60.0) | 274/472 | (58.1) |
| | Day 28 | 4/465 | (0.9) | 7/477 | (1.5) |
| | | | | | |
| **≥1.0 IU/ml** | Day 0 | 182/468 | (38.9) | 195/472 | (41.3) |
| | Day 28 | 461/465 | (99.1) | 470/477 | (98.5) |
| | | | | | |
| **Diphtheria** | | | | | |
| **< 0.1 IU/ml** | Day 0 | 87/469 | (18.6) | 103/476 | (21.6) |
| | Day 28 | 0/467 | (0.0) | 0/476 | (0.0) |
| | | | | | |
| **≥0.1- < 1.0 IU/ml** | Day 0 | 2551469 | (54.4) | 267/476 | (56.1) |
| | Day 28 | 0/467 | (0.0) | 21476 | (0.4) |
| | | | | | |
| **≥1.0 IU/ml** | Day 0 | 127/469 | (27.1) | 106/476 | (22.3) |
| | Day 28 | 467/467 | (100.0) | 474/476 | (99.6) |

Related studies were conducted to access the safety and immunogenicity of MCV-4 vaccine concomitantly administered with licensed Td vaccine in healthy 10 -17 year old adolescents. Briefly, in a multicenter randomized trial healthy 10-17 year olds (mean age 12,9 years) received TetraMenD (MCV-4) + Td either concomitantly (n = 509), or at separate visits one month apart (n = 512). Safety assessments for the two vaccines, given separately and concomitantly, were collected on days 8 and 28 post-vaccination. Immune responses were assessed prior to and 4 weeks post-vaccination by antibody titers to diphtheria and tetanus, and serum bactericidal activity (SBA) to the meningococcal serogroups. The safety profile for subjects given Td alone was similar to that in subjects given Td + TetraMenD. Concomitant Administration of Td + TetraMenD did not interfere with the immune response to either tetanus or diphtheria toxoids. SBA responses to the four serogroups are summarized in Table E-14 shown below.

**Table E-14: SBA response to the four serogroups**

| **Serogroup** | **SBA (GMT)** | | | |
|---|---|---|---|---|
| | **Concomitant Administration** | | **Separate Administration** | |
| | **Pre (N=468)** | **Post (N=466)** | **Pre (N=477)** | **Post (N=478)** |
| A | 232 | 11313 | 228 | 10391 |
| c | 66 | 5059 | 57 | 2136 |
| Y | 124 | 3391 | 115 | 1331 |
| W-135 | 26 | 4195 | 27 | 1339 |

This study shows that the co-administration of Td and TetraMenD was safe and well tolerated in the test subjects. Concomitant administration of TetraMenD + Td does not adversely affect immune responses to tetanus and diphtheria toxoids. The immune responses to serogroups C, Y, and W 135 polysaccharide were enhanced when MCV-4 was co-administered with Td. The enhanced immune response observed in this study was surprising and unexpected.

### Example 14 Comparison of Serum Bactericidal Assay with Baby Rabbit

### Complement and with Human Complement for N. Meningococcal Serogroups C, W-135 and Y

A subset of serum samples from Study A, Stage III, is used in this study to compare the results obtained with SBA-BR titers with SBA-HC titers for Serogroups C, W-135 and Y. Subjects enrolled into this trial are at least 2 years of age but not yet 11 years of age and each is randomly assigned to one of the two vaccine groups. Approximately 5 mL of whole blood is collected from each subject at baseline (prior to vaccination) and on Day 28 post-vaccination. Blood specimens from subjects are centrifuged within 4 hours of collection. The serum is taken off the clot, transferred into labeled cryotubes and stored in a temperature-monitored freezer at -20°C or colder. All samples that are used in the analysis in this report are from paired sera obtained from the first subjects enrolled in the clinical study and that are of sufficient serum volume to complete all planned testing. All samples are from 2 year old and 3 year old subjects with the exception of a single 4 year old. There are 2 subjects in the intent-to-treat category. One of these is from the TetraMenD vaccine group (Day 28 post-vaccination sample is collected on Day 24) and one is from the Menomune® vaccine group (Day 28 post-vaccination is collected on Day 9).

Baby rabbit complement (Pel-Freeze®, Clinical Systems LLC, Brown Deer, WI, product code 31038) is pre-screened for suitability in each of the serogroups specific assays. The criteria for suitability included an agreement with SBA-BR test results for a defined set of serum samples (within a 2-fold dilution) using a previously qualified lot of rabbit complement. Criteria for meeting predetermined titers for a reference serum and control samples are also used. Aliquots of 2.5 ml of the rabbit complement are stored at -70°C or colder until ready for use. Aliquots are thawed once and used or discarded.

Serum from subjects enrolled is screened for anti-meningoeoccal polysaccharide IgG and IgM levels by ELISA and tested in the SBA-BR for functional antibodies to identify potential sources of complement for use in the SBA-H. Criteria established for selection of a human source of complement are the following; (1) lack of detectable antibody when assayed in the SBA-BR assay, (2) lack of intrinsic bactericidal activity when used as the complement source in the assay, (3) acceptable performance when used as a complement source with a panel of negative control, using sera with previous negative test results determined at an independent outside lab by Dr. Ray Borrow, and (4) acceptable reproducibility performance with a panel of 24 samples. Exogenous complement sources used in each of the serogroup specific assays are from different subjects. No complement sources are found to work for more than one serogroup. Also, the three complement sources used in the SBA assays are from a single donor per serogroup.

### Serogroup C

Serum from several subjects with acceptably low ELISA values (less than 0.5 µg/ml for both IgG and IgM) demonstrated bactericidal activity.

### Serogroup Y

The complement source for the serogroup Y SBA-H is selected from the subjects enrolled in the collection protocol. Serum from the source of complement displayed low-level serogroup Y IgG and IgM antibodies by ELISA and is negative in the SBA-BR assay. Serum from the source showed no intrinsic bactericidal activity when used in the SBA.

### Serogroup W-135

The complement source for the serogroup W-135 SBA-H is selected from the subjects enrolled in the collection protocol. Serum from the source of complement displayed low-level serogroup W-135 IgG and IgM antibodies by ELISA and is negative in the SBA-BR assay. Serum from the source showed no intrinsic bactericidal activity when used in-the SBA.

### Serum Bactericidal Assays

Briefly, Meningococcal serogroups C, Y, and W-135 strains are obtained from the Centers for Disease Control, Atlanta, GA (CDC). Target strains of bacteria are prepared for use in the assays from freshly thawed working seed lot vials of serogroups C, Y and W-135. Each vial is used to streak a Thayer Martin plate that is incubated overnight at 37°C ± 0.5°C in 5% CO₂. The following day, isolated colonies are harvested with a sterile swab and used to inoculate the entire surface of fresh Thayer Martin plates that had been warmed to ambient temperature. Plates are incubated for 4 h at 37°C ± 0.5°C in 5% CO₂ to obtain a light veil of confluent bacterial growth that is harvested with sterile swabs and suspended in Dulbecco's PBS +0.1% Dextrose Buffer to a prescribed optical density (absorbance at 600 nm). A working solution with a prescribed concentration of bacteria is prepared in Dulbecco's PBS + 0.1% Dextrose Buffer, maintained at ambient temperature and used within 30 minutes of preparation.

Test samples are heat-treated at 56°C for 30 minutes to inactivate endogenous complement. To all wells of a 96-well microtiter plate, Dulbecco's PBS + 0.1% Dextrose Buffer is added, then test serum samples are dispensed in 2-fold serial dilutions across the plate leaving the final two columns of wells for complement and serum control wells. Columns on every plate included a complement column ([column 11] - serum/+complement) and a serum control column ([column 121+serum/-complement).

Freshly thawed complement is mixed with the working concentration of bacteria and the mixture is dispensed into all but the serum control wells of the microtiter plates. Bacteria without added complement are dispensed into the serum control wells. The plates are covered and placed on a plate-shaker for 1 minute then removed to a 37°C ± 0.5°C CO₂ incubator. Incubation times are 90 minutes for the serogroup A assay plates and 60 minutes for the serogroups C, Y, and W-135 assay plates. After incubation, 100 µl af agarose overlay medium at 50°C ± 1 °C is carefully added to all wells avoiding air bubble formation. After a 10 minute period at ambient temperature with the microtiter plate lids ajar to avoid moisture formation, the plates are covered and removed to a dry (no added humidity) 5% CO₂ incubator at 37°C ± 0.5°C for 20 ± 4 h. After this incubation, the number of bacterial colonies per well is counted. The average number of colonies per well for the complement controls wells is calculated and divided in half to obtain the 50% survival at To.

The bactericidal titer of each unknown serum is expressed as the final reciprocal serum dilution yielding ≥50% killing compared with the 50% survival value at To. The starting dilution for samples in the SBA-BR is a 1:8 dilution. For the SBA-H, the starting dilution is lowered to a 1:4 dilution as described in the original assays.

A comparison of the SBA-BR procedure for the serogroup A assay described herein with the Standardized SBA procedure (CDC) and with the SBA procedure as performed at the Manchester Public Health Laboratory Services, Meningococcal Reference Unit, Manchester, UK (PHLS) is provided as Table 14-1.

**Table 14-1: Serum Bactericidal Assay Methods Comparison**

| | **AvP-US** | **CDC** | **PHLS** |
|---|---|---|---|
| Frozen stock | Greaves Soln w 10% Glycerol | Greaves Soln w 10% Glycerol | Frozen in Glycerol (15%) broth |
| Bacterioidal buffer | Dulbecco's with 0.1% Glucose | Dulbecco's with 0.1% Glucose | Geys with 0.5% BSA |
| Over night growth Media | Thayer Martin (MR0232) | Brain Heart Infusion w/1% Horse Serum | Blood agar w/ 5% Horse Blood |
| Over night Growth Conditions | 37°C w/ 5% C02 | 37ºC w/5% C02 | 37°C w/ 5% CO₂ |
| Complement | Baby Rabbit (Pel-Freez) | Baby Rabbit (Pel-Freez) | Baby Rabbit (Pel-Freez) |
| A Strain | F8238 | F8238 | F8238 |
| Assay day growth Media | Thayer Martin (MR0232) | Brain Heart Infusion wl 1% Horse Serum | Blood agar |
| Assay day growth conditions | 4 hours 37°C w/ 5% CO2 | 4 hours 37°C w/ 5% C02 | 4 hours 37°C w/ 5% CO₂ |
| **To targeted (per ml)** | **4000 CFU/ml** | **4000 CFU/ml** | **80,000 CFU/ml** |
| Initial starting dilution of sera | 1:4 | 1:4 | 1:2 |
| Serum Treatment | 56ºC for 30 minutes | 56ºC for 30 minutes | 56°C for 30 minutes |
| Total volume at incubation step | 50 µl | 50 µl | -40 µl |
| Serum mixture as % total (Vol) | 50% (25µl) | -50% (25µl) | 50%(20µl) |
| Cell suspension % (volume) | 25% (12.5µl) | 25% (12.5µl) | 25%(10µl) |
| Complement % (volume) | 25%(12.5µl) | 25% (12.5µl) | 25%(10µl) |
| **CFU well In r'xn mixture (theor.)** | 50 | 50 | 800 |
| Final starting dilution | 1:8 | 1:8 | 1:4 |
| Serum Incubation conditions | 37°C wl 5% CO2 for 90 minutes | 37°C for 90 minutes | 37ºC w/o CO2 for 90 minutes |
| **Overnight Incubation method** | 100 µl TSB Agar overlay added (in 96 well plates) - 37°C w/ 5% CO2 | 100 µl TSB Noble Agar overlay added -*37°C wl 5*% C02 (in 96 well plates) | 10 ul on Agar Plates (Tilt Method) 37°C |
| To conditions | 37°C for 90 minutes (i.e. Complement Control Average) | 37°C for 90 minutes (i.e. Complement Control Average) | Plated prior to 90 minute incubation Overnight at 37°C w/5% CO2 |
| Endpoint Titer | 50% Killing | 50% Killing | 50% Killing |

A reference serum is obtained from Dr. George Carlone, CDC (CDC donar-R21654-3430107) as lyophilized powder in vials, which are stored at 2°C to 8°C until used. When needed, vials are each rehydrated with 0.5 ml sterile water and stored at -80°C to -40°C as 100 µl working aliquots. The titer of the reference serum when reconstituted under these conditions is 1:256 + 1 two-fold dilution in the standardized SBA-BR for serogroups A, C, Y, and W-135. Reference serum samples are run twice on different plates of the daily set of plates.

Group-specific rabbit antisera for serogroups A, C, Y, and W-135 are purchased from Difco as lyophilized powder in vials, that are stored at 2° to 8°C until used. When needed, each vial is rehydrated with 1 ml sterile water and stored at-80°C to -40°C as 50 µl aliquots for use as quality control samples in the SBA.

The results of the Serum Bactericidal Assay using baby rabbit complement (SBA-BR) provided herein for the determination of complement-mediated anti-polysaccharide bactericidal activity to *Neisseria meningitidis* serogroups C, Y, and W-135 in clinical serum samples is fully validated for precision, dilutability (linearity), specificity and limit of detection. The SBA-H assay (for Serogroup C) is repeated on five consecutive days with an identical set of serum samples to establish the precision of the assay.

### Calculation of sensitivity and specificity of the SBA-BR

Titers obtained in the SBA-BR are classified as true positive (TP) (and false positive [FP]) and true negative (TN) (and false negative [FN]) using the SBA-H benchmark titers of 1:4 and 1:8. Sensitivity is calculated as TP/(TP+ FN) and specificity is calculated as TN/(TN + FP). The results of these calculations are expressed as percentages.

### SBA Titer Distribution Comparison of SBA-BR versus SBA-H

The pre- and twenty-eight day post-immunization SBA titers are shown in Tables 1 and 4 for serogroup C, Tables 2 and 5 for serogroup Y, and Tables 3 and 6 for serogroup W-135. Summarized in the following subsections is an analysis of the pre- and post-immunization SBA. titers comparing the results obtained for the two sources of complement (BR versus H).

### Serogroup C SBA Titer Distribution

of the 10 1 pre-immunizatian serum samples, 63 are negative as defined by having a SBA-H titer of < 1:4 and a SBA-BR titer < 1:8. Twenty-seven of the pre-immunization samples are negative by SBA-H (< 1:4) but are positive by SBA-BR (>= 1:8). The false positive rate using a SBA-BR cut off titer of < 1:8 is 30%. The false positive rate decreases at higher SBA-BR cut off titers to less than 20% at a cut off titer of 1:128, and to less than 10% at a cut off titer of 1:512. Seven of the samples that are positive by SBA-H (>=1:4) are negative by SBA-BR (< 1:8).

In the post-immunization sera, 48 samples are negative by SBA-H, and only 11 are negative by SBA-BR. Of the 11 samples that are negative by SBA-BR, 3 are positive by SBA-H. Seventeen of 51 post-immunization samples (32%) in the conjugate group are negative by SBA-H, but positive by SBA-BR (>=1:8). For the polysaccharide group, 23 of 50 post-immunization samples (46%) are negative by SBA-H, but positive by SBA-BR titer (>= 1:8). In terms of positive responses in the post-immunization sera, 90 of 101 (89%) of samples are positive by SBA-BR (>= 1:8) but only 53 of 101 (52%) are positive by SBA-H (>= 1:4). There is a notable difference in the positive response rates when comparing the SBA titers (BR versus H) obtained for the two vaccine groups. For the 51 post-immunization samples in the conjugate group, 33 of 51 (65%) are positive by SBA-H (>= 1:4) and by SBA-BR (>= 1:8). Agreement between the SBA titers (BR versus H) improves at a SBA-BR threshold titer of >=1:64 and higher. Of the 50 post-immunization samples in the polysaccharide group, 17 of 50 (34%) are positive by SBA-H (>=1:4) and by SBA-BR (>=1:8). Agreement between the SBA titers (BR versus H) improves at SBA-BR threshold titer >=1:512 and higher.

### Serogroup Y SBA Titer Distribution

Unlike the serogroup C pre-immunization sera, only 9 of the serogroup Y pre-immunization samples are negative as defined by having an SBA-H titer < 1:4 and a SBA-BR titer < 1:8. Fifty-two of 61 pre-immunization samples are negative by SBA-H (< 1:4) but positive by SBA-BR (>= 1:8). The false positive rate using a SBA-BR cut off titer of < 1:8 is 85%. The false positive rate decreases at higher SBA-BR cut off titers to less than 15% at a cut off titer of 1:256, and less than 2% at 1:512. Two samples are positive by SBA-H (>= 1:4) but negative by SBA-BR (< 1:8).

There are no post-immunization serum samples that had a 5BA-H titer < 1:4 and a SBA-BR titer that is < 1:8. Nineteen samples that are negative by SBA-H (< 1:4) are positive buy SBA=BR (>= 1:8). As noted for serogroup C, there is a difference in the proportion of false negative results. In the conjugate group, 5 of 48 samples (9%) are negative by SBA-H (< 1:4), but positive by SBA-BR. In the polysaccharide group, 14 of 52 samples (27%) are negative by SBA-H (< 1:4), but positive by SBA-BR.

There is good agreement between the two SBA titers (BR versus H) for positive responses in the post-immunization sera for serogroup Y. For the total set of 100 samples, all 100 post-immunization samples had SBA-BR titers >= 1:8, and 81 of 100 had SBA-H titers of >= 1:4. As noted for the SBA responses for serogroup C, there is better correlation between the SBA titers (BR versus H) in the conjugate group compared to the SBA titers (BR versus H) obtained for the polysaccharide group. Of the 48 post-immunization samples in the conjugate group, 43 (90%) are positive by SBA-H (>= J.:4) and by SBA-BR (>=1:8). Only 1 of 48 samples had a SBA-BR titer less than 1:32, and that sample is positive by SBA-H (>=1:4). The agreement between the SBA titers (BR versus H) is not as good in the polysaccharide group. Only 38 of 52 (73%) had post-immunization SBA-H titers >=1:4 and a SBA-BR titer >=1:8. Agreement between the SBA titers (BR versus H) in the post-immunization sera for the polysaccharide group improves at a SBA-BR titers of>= 1:128.

### Serogroups W-135 SBA Titer Distribution

For serogroupW-135, 54 of 100 (54%) are negative where both the SBA-H titer is < 1:4 and the SBA-BR titer is < 1:8. Of the pre-immunization samples, 27 of 81 are negative by SBA-H (< 1:4) but positive by SBA-BR (>= 1:8). The false positive rate using a SBA-BR cut off titer of < 1:8 is 33%. The false positive rate decreases as higher SBA-BR cut off titers to less than 15% at a cut off titer of 1:128, and to less than 5% at a cut off titer of 1:256. Eleven samples are positive by SBA-H (>= 1:4) but are negative by SBA-BR (< 1:8).

Three post-immunization samples are negative by SBA-H (< 1:4) and negative by SBA-BR (<1:8). Thirty-nine post-immunization samples are negative by SBA-H (< 1:4) but are positive by SBA-BR titer (>= 1:8). In the conjugate group, 11 of 47 samples (23%) are negative by SBA-H but positive by SBA-BR. In the polysaccharide group, 28 of 53 samples (53%) are negative by SBA-H but positive by SBA-BR.

The agreement between the post-immunization SBA-BR and SBA-H titers is comparable to serogroup C, but not as good compared to serogroup Y. As with both serogroup C and serogroup Y, there is a notable difference in the agreement between the two SBA titers (BR versus H) when comparing the two vaccine groups. The agreement between the two SBA titers (BR versus H) is better for the conjugate group compared to the polysaccharide group. In the post-immunization SBA titers for the conjugate group, 36 of 47 (77%) had an SBA-H titer of>= 1:4 and all are positive by SBA-BR (>= 1:8). All samples from the conjugate group had post vaccination SBA-BR titers >= 1:32.For the post-immunization titers for the polysaccharide group, the correlation between the two titers is not as good, only 22 of 53 (42%) had an SBA-H titer >= 1:4 and 50 of 53 (94%) had a SBA-BR titer >= 1:8.

**Table I. Comparison of the SBA-BR titer in sera positive and negative by SBA-H for serogroup C**

| **1/SBA-BR titer** | **No. of sera with indicated SBA-H titer:** | | | | | | | |
|---|---|---|---|---|---|---|---|---|
| | **Pre-immunization** | | **28-day post-immunization** | | | | | |
| | | | **TetraMenD** | | **Menomune^{®}** | | **Combined** | |
| | **<4** | **>=4** | **<4** | **>=4** | **<4** | **>=4** | **<4** | **>= 4** |
| <8 | 63 | 7 | 1 | 0 | 7 | 3 | 8 | 3 |
| 8 | 4 | 0 | 1 | 0 | 1 | 0 | 2 | 0 |
| 16 | 1 | 1 | 1 | 0 | 1 | 1 | 2 | 1 |
| 32 | 2 | 0 | 5 | 3 | 1 | 1 | 6 | 4 |
| 64 | 4 | 0 | 4 | 6 | 5 | 2 | 9 | 8 |
| 128 | 3 | 2 | 3 | 9 | 8 | 2 | 11 | 11 |
| 256 | 11 | 1 | 1 | 3 | 4 | 3 | 5 | 6 |
| 512 | 1 | 0 | 0 | 5 | 1 | 3 | 1 | 8 |
| 1024 | 0 | 0 | 2 | 6 | 1 | 4 | 3 | 10 |
| 2048 | 1 | 0 | 0 | 1 | 1 | 0 | 1 | 1 |
| 4096 | 0 | 0 | 0 | 0 | 0 | 1 | 0 | 1 |
| **Total** | **90** | **11** | **18** | **33** | **30** | **20** | **48** | **53** |

**Table 2. Comparison of the SBA-BR titer in sera positive and negative by SBA-H for serogroup Y**

| **1/SBA-BR titer** | **No. of sera with indicated SBA-H titer:** | | | | | | | |
|---|---|---|---|---|---|---|---|---|
| | **Pre-immunization** | | **28-day post-immunization** | | | | | |
| | | | **TetraMenD** | | **Menomune^{®}** | | **Combined** | |
| | **<4** | **>=4** | **<4** | **>=4** | **<4** | **>= 4** | **<4** | **>= 4** |
| <8 | 9 | 2 | 0 | 0 | 0 | 0 | 0 | 0 |
| 8 | 1 | 1 | 0 | 1 | 0 | 0 | 0 | 1 |
| 16 | 4 | 2 | 0 | 0 | 0 | 0" | 0 | 0 |
| 32 | 10 | 6 | 1 | 1 | 3 | 2 | 4 | 3 |
| 64 | 14 | 6 | 1 | 3 | 4 | 2 | 5 | 5 |
| 128 | 15 | 12 | 1 | 9 | 4 | 6 | 5 | 15 |
| 256 | 7 | 9 | 1 | 13 | 2 | 16 | 3 | 29 |
| 512 | 1 | 0 | 1 | 7 | 1 | 6 | 2 | 13 |
| 1024 | 0 | 0 | 0 | 5 | 0 | 4 | 0 | 9 |
| 2048 | 0 | 1 | 0 | 3 | 0 | 1 | 0 | 4 |
| 4096 | 0 | 0 | 0 | 1 | 0 | 1 | 0 | 2 |
| **Total** | **61** | **39** | **5** | **43** | **14** | **38** | **19** | **81** |

**Table 3. Comparison of the SBA-BR titer in sera positive and negative by SBA-H for serogroup W-135**

| **1/SBA-BR titer** | **No. of sera with indicated SBA-H titer:** | | | | | | | |
|---|---|---|---|---|---|---|---|---|
| | **Pre-immunization** | | **28-day post-immunization** | | | | | |
| | | | **TetraMenD** | | **Menomune^{®}** | | **Combined** | |
| | **<4** | **>=4** | **<4** | **>=4** | **<4** | **>= 4** | **<4** | **>= 4** |
| <8 | 9 | 2 | 0 | 0 | 0 | 0 | 0 | 0 |
| <8 | 54 | 11 | 0 | 0 | 3 | 0 | 3 | 0 |
| 8 | 1 | 3 | 0 | 0 | 0 | 0 | 0 | 0 |
| 16 | 3 | 0 | 0 | 0 | 1 | 0 | 1 | 0 |
| 32 | 2 | 0 | 0 | 1 | 0 | 0 | 0 | 1 |
| 64 | 11 | 2 | 1 | 2 | 3 | 3 | 4 | 5 |
| 128 | 7 | 1 | 2 | 2 | 7 | 2 | 9 | 4 |
| 256 | 3 | 2 | 4 | 3 | 6 | 5 | 10 | 8 |
| 512 | 0 | 0 | 4 | 13 | 5 | 4 | 9 | 17 |
| 1024 | 0 | 0 | 0 | 3 | 5 | 3 | 5 | 6 |
| 2048 | 0 | 0 | 0 | 11 | 1 | 5 | 1 | 16 |
| 4096 | 0 | 0 | 0 | 1 | 0 | 0 | 0 | 1 |
| **Total** | **81** | **19** | **11** | **36** | **31** | **22** | **42** | **58** |

**Table 4. Summary of distribution of serogroup C titers measured by the SBA-BR and the SBA-H**

| **1/bactericidal titer** | **No. of samples¹ (% of pre- or post-) with indicated titer by:** | | | | | |
|---|---|---|---|---|---|---|
| | **SBA-BR** | | | **SBA-H** | | |
| | **Pre-imm.** | **Post-imm.** | | **Pre-imm.** | **Post-imm.** | |
| | | **Menomune^{®}** | **TetraMenD** | | **Menomune^{®}** | **TetraMenD** |
| <4 | | | | 90 (89.11) | 30 (60.00) | 188(35.29) |
| 4 | | | | 2(1.98) | 1(2.00) | 1(1.96) |
| <8 | 70 (69.31) | 10(20.00) | 1 (1.96) | | | |
| 8 | 4(3.96) | 1 (2.00) | 1(1.96) | 0 | 2 (4.00) | 1(1.96) |
| 16 | 2 (1.98) | 2 (4.00) | 1 (1.96) | 3 (2.97) | 2 (4.00) | 5 (9.80) |
| 32 | 2 (1.98) | 2 (4.00) | 8 (15.69) | 1 (0.99) | 3 (6.00) | 3 (5.88) |
| 64 | 4 (3.96) | 7 (14.00) | 10 (19.61) | 1 (0.99) | 0 | 5 (9.80) |
| 128 | 5(4.95) | 10 (20.00) | 12 (23.53) | 3 (2.97) | 3 (6.00) | 5 (9.80) |
| 256 | 12 (11.88) | 7 (14.00) | 4 (7.84) | 1 (0.99) | 1 (2.00) | 5 (9.80) |
| 512 | 1 (0.99) | 4 (8.00) | 5 (9.8) | 0 | 5 (10.00) | 2 (3.92) |
| 1024 | 0 | 5 (10.00) | 8 (15.69) | 0 | 1 (2.00) | 5 (9.80) |
| 2048 | 1 (0.99) | 1 (2.00) | 1 (1.96) | 0 | 0 | 1 (1.96) |
| 4096 | 0 | 1 (2.00) | 0 | 0 | 1 (2.00) | 0 |
| 8192 | 0 | 0 | 0 | 0 | 1 (2.00) | 0 |

| | | | | | | |
|---|---|---|---|---|---|---|
| ¹Total number of samples is 101. | | | | | | |

**Table 5. Summary of distribution of serogroup Y titers measured by the SBA-BR and the SBA-H**

| **1/bactericidal titer** | **No. of samples¹ (% of pre- or post-) with indicated titer by:** | | | | | |
|---|---|---|---|---|---|---|
| | **SBA-BR** | | | **SBA-H** | | |
| | | **Post-imm.** | | | **Post-imm.** | |
| | **Pre-imm.** | **Menomune^{®}** | **TetraMenD** | **Pre-imm.** | **Menomune^{®}** | **TetraMenD** |
| <4 | | | | 61 (61.00) | 14 (26.92) | 5 (10.42) |
| <8 | 11 (11.00) | 0 | 0 | | | |
| 8 | 2 (2.00) | 0 | 1 (2.08) | 3 (3.00) | 1 (1.92) | 4 (8.33) |
| 16 | 6 (6.00) | 0 | 0 | 11 (11.00) | 12 (23.08) | 2(4.17) |
| 32 | 16 (16.00) | 5 (9.62) | 2 (4.17) | 12 (12.00) | 11(21.15) | 11(22.92) |
| 64 | 20 (20.00) | 6(11.54) | 4(8.33) | 5 (5.00) | 9(17.31) | 5 (10.42) |
| 128 | 27 (27.00) | 10 (19.23) | 10 (20.83) | 6 (6.00). | 2 (3.85) | 8 (16.67) |
| 256 | 16 (16.00) | 18 (34.62) | 14 (29.17) | 1 (1.00) | 1(1.92) | 6 (12.50) |
| 512 | 1 (1.00) | 7 (13.46) | 8 (16.67) | 0 | 1 (1.92) | 4 (8.33) |
| 1024 | 0 | 4 (7.69) | 5 (10.42) | 0 | 1 (1.92) | 2 (4.17) |
| 2048 | 1 (1.00) | 1 (1.92) | 3 (6.25) | 0 | 0 | 0 |
| 4096 | 0 | 1 (1.92) | 1 (2.08) | 0 | 0 | 0 |

| | | | | | | |
|---|---|---|---|---|---|---|
| Total number of samples is 100. | | | | | | |

**Table 6. Summary of distribution of serogroup W-135 titers measured by the SBA-BR and the SBA-H**

| **1/bactericidal titer** | **No. of samples¹ (% of pre- or post-) with indicated titer by:** | | | | | |
|---|---|---|---|---|---|---|
| | **SBA-BR** | | | **SBA-H** | | |
| | **Pre-imm.** | **Post-imm.** | | **Pre-imm.** | **Post-imm.** | |
| | | **Menomune^{®}** | **TetraMenD** | | **Menomune^{®}** | **TetraMenD** |
| <4 | | | | 81 (81.00) | 31 (58.49) | 11 (23.40) |
| 4 | | | | 4 (4.00) | 5 (9.43) | 6 (12.77) |
| <8 | 65 (65.00) | 3 (5.66) | 0 | | | |
| 8 | 4 (4.00) | 0 | 0 | 10 (10.00) | 7 (13.21) | 2 (4.26) |
| 16 | 3 (3.00) | 1 (1.89) | 0 | 2 (2.00) | 5 (9.43) | 11 (23.40) |
| 32 | 2 (2.00) | 0 | 1 (2.13) | 3 (3.00) | 1 (1.89) | 5 (10.64) |
| 64 | 13 (13.00) | 6 (11.32) | 3 (6.38) | 0 | 3 (5.66) | 2 (4.26) |
| 128 | 8 (8.00) | 9 (16.98) | 4 (8.51) | 0 | 0 | 5 (10.64) |
| 256 | 5(5.00) | 11 (20.75) | 7 (14.89) | 0 | 0 | 3 (6.38) |
| 512 | 0 | 9 (16.98) | 17 (36.17) | 0 | 1 (1.89) | 1 (2.13) |
| 1024 | 0 | 8 (15.09) | 3 (6.38) | 0 | 0 | 1 (2.13) |
| 2048 | 0 | 6 (11.32) | 11 (23.40) | 0 | 0 | 0 |
| 4096 | 0 | 0 | 1 (2.13) | 0 | 0 | 0 |

| | | | | | | |
|---|---|---|---|---|---|---|
| ¹Total number of samples is 100. | | | | | | |

### Sensitivity and Specificity Comparison of the SBA-BR and SBA-H Titers

SBA-BR titers are compared to the SBA-H protective titers of 1:4 and 1:8 in performing sensitivity and specificity assessments between the two sets of titers. Both pre- and post-immunization sera are used in this analysis. Using the SBA-H benchmark titers of 1:4 and 1:8, specificity and sensitivity are calculated for all three serogroups and are summarized in Tables 7, 9 and 10. The analysis of sensitivity and specificity are discussed in turn for each serogroup.

For serogroup C, the sensitivity is greater than 80% for SBA-BR threshold titers of 1:8, 1:16, and 1:32 relative to both a 1:4 and 1:8 SBA-H titer. However, the specificity at these SBA-BR titers is less than 60%. Specificity increased above 60% at a SBA-BR titer of 1:64, and above 70% at a SBA-BR titer of 1:128. For these latter two SBA-BR titers, the specificity begins to drop off. At a SBA-BR threshold titer of 1:64, the sensitivity is between 75 and 78%, but at a SBA-BR titer 1:128, sensitivity falls to between 62 and 65%. Specificity continues to improve at SBA-BR titers > 1:64 ranging from 73% up to 83% for 1:128 and 1:256, respectively. However, sensitivity falls from 43% to less than 20%. The SBA-BR titer for serogroup C with the best balance between sensitivity and specificity falls in a range of SBA-BR titers between 1:32 and 1:128. The sensitivity and specificity results for serogroup C are found to be quite comparable to the results obtained by Santos GF, et al., 2001. Clin. Diagn. Lab. Immunol. 8:616-623, obtained by different set of serum samples and reagents (Table 8). With respect to Santos's results, the best balance of sensitivity and specificity is observed between the SBA-BR titers of 1:64 and 1:128 versus both the SBA-H titers of 1:4 and 1:8.

**Table 7. Sensitivity and Specificity of the SBA-BR at Protective Titers in the SBA-H for Serogroup C**

| | **SBA-H titer of >=1:4** | | **SBA-H titer of >=1:8** | |
|---|---|---|---|---|
| **1/SBA-BR titer** | **SBA-H titer of >= 1:4** | | **SBA-H titer of >= 1:8** | |
| **1/SBA-BR titer** | **Sensivity (%)** | **Specificity (%)** | **Sensitivity (%)** | **Specificity (%)** |
| 8 | 84 | 51 | 88 | 53 |
| 16 | 84 | 56 | 88 | 56 |
| 32 | 81 | 58 | 85 | 58 |
| 64 | 75 | 64 | 78 | 64 |
| 128 | 62 | 73 | 65 | 73 |
| 256 | 42 | 83 | 43 | 83 |
| 512 | 31 | 95 | 33 | 95 |
| 1024 | 19 | 96 | 20 | 96 |

**Table 8. Sensitivity and Specificity of the SBA-BR at Protective Titers in the SBA-H for Serogroups C as reported by Santos et al. 2001. Clin. Diagn. Lab. Immunol. 8:616-623.**

| **1/SBA BR titer** | **SBA-H titer of >= 1:4** | | **SBA-H titer of >= 1:8** | |
|---|---|---|---|---|
| | **Sensitivity %** | **Specificity %** | **Sensitivity %** | **Specificity %** |
| 32 | 85 | 61 | 91 | 58 |
| 64 | 78 | 73 | 86 | 68 |
| 128 | 69 | 83 | 78 | 81 |
| 256 | 54 | 87 | 63 | 88 |
| 512 | 41 | 92 | 49 | 94 |

For serogroup Y, sensitivity is highest for SBA-BR threshold titers ranging from 1:8 to 1:64, but, as expected, drops off at higher SBA-BR threshold titers. Specificity results for serogroup Y start out much lower compared to the results for serogroup C, and do not reach a level of greater than 50% until a threshold SBA-BR titer of 1:128. The SBA-BR titer that is best balanced for sensitivity and specificity for serogroup Y falls in a range between 1:64 and 1:256. At 1:256, the sensitivity drops off to approximately 55%, but specificity increases from the mid-30% region to approximately 82-83%.

**Table 9. Sensitivity and Specificity of the SBA-BR at Protective Titers in the SBA-H for Serogroup Y**

| | **SBA-H titer off >= 1:4** | | **SBA-H titer of >= 1:8** | |
|---|---|---|---|---|
| | **Sensitivity (%)** | **Specificity (%)** | **Sensitivity (%)** | **Specificity (%)** |
| 8 | 98 | 11 | 98 | 11 |
| 16 | 97 | 12 | 97 | 12 |
| 32 | 95 | 17 | 95 | 17 |
| 64 | 87 | 35 | 87 | 34 |
| 128 | 78 | 59 | 78 | 57 |
| 256 | 56 | 84 | 55 | 82 |
| 512 | 56 | 84 | 55 | 82 |
| 1024 | 13 | 100 | 14 | 100 |

For serogroup W135, the values for sensitivity follow more closely to the values obtained for serogroup C, but the overall pattern is the same for all three serogroups. Sensitivity starts out high at a SBA-BR threshold titer of 1:8 and drops off at titers >= 1:128. Likewise, specificity starts out low at a SBA-BR titer of 1:8 begins to level out at a titer of 1:256. As observed for serogroup Y, the SBA-BR with the best balance between sensitivity and specificity for serogroup W135 falls in a range between 1:64 and 1:256.

**Table 10. Sensitivity and Specificity of the SBA-BR at Protective Titers in the SBA-H for Serogroup W-135**

| **1/SBA-BR titer** | **SBA-H titer of >= 1:4** | | **SBA-H titer of >= 1:8** | |
|---|---|---|---|---|
| | **Sensitivity (%)** | **Specificity (%)** | **Sensitivity (%)** | **Specificity (%)** |
| 8 | 86 | 46 | 87 | 43 |
| 16 | 82 | 48 | 82 | 44 |
| 32 | 82 | 50 | 82 | 47 |
| 64 | 80 | 52 | 81 | 49 |
| 128 | 71 | 64 | 73 | 61 |
| 256 | 65 | 77 | 64 | 72 |
| 512 | 52 | 88 | 50 | 83 |
| 1024 | 30 | 95 | 34 | 94 |

Summarized in Table 11 are the proportion of four-fold rise in SBA titers using both baby rabbit complement or human complement for serogroups C, Y, and W135 relative to the post-immunization SBA-BR titer. This analysis is performed separately on the conjugate group, TetraMenD, and the polysaccharide group, Menomune^{®}, and both sets of analysis are included in Table 11. There are some observable differences in the four-four rise patterns comparing the bactericidal responses induced to the three serogroups and for the two vaccine groups. The response patterns are discussed in turn for each serogroup and for both vaccine groups.

**Table 11. Stratified Comparative Ratios to a 4-Fold Rise in Meningococcal Polysaccharide Titers Determined by SBA-BR and SBA-H¹**

| | **Serogroup C** | | | | **Serogroup Y** | | | | **Serogroup W-135** | | | |
|---|---|---|---|---|---|---|---|---|---|---|---|---|
| | **TetraMenD** | | **Menomune^{®}** | | **TetraMenD** | | **Menomune^{®}** | | **TetraMenD** | | **Menomune®** | |
| **1/ SBA-** | **SBA-BR** | **SBA-H** | **SBA-BR** | **SBA-H** | **SBA- BR** | **SBA-H** | **SBA- BR** | **SBA-H** | **SBA-BR** | **SBA-H** | **SBA-BR** | **SBA-H** |
| **BR titer** | | | | | | | | | | | | |
| <8 | 0/1 | 0/1 | 0/10 | 1/10 | na | na | na | na | na | na | 0/3 | 0/3 |
| | (0%) | (0%) | (0%) | (0%) | | | | | | | (0%) | (0%) |
| 8 | 0/1 | 0/1 | 0/1 | 0/1 | 0/1 | 1/1 | na | na | na | na | na | na |
| | (0%) | (0%) | (0%) | (0%) | (0%) | (100%) | | | | | | |
| 16 | 0/1 | 0/1 | 0/2 | 1/2 | Na | na | na | na | na | na | 0/1 | 0/1 |
| | (0%) | (0%) | (0%) | (50%) | | | | | | | (0%) | (0%) |
| 32 | 7/8 | 3/8 | 2/2 | 1/2 | 1/2 | 0/2 | 2/5 | 1/5 | 0/1 | 1/1 | na | na |
| | (87%) | (37%) | (100%) | (50%) | (50%) | (0%) | (40%) | (20%) | (0%) | (100%) | | |
| 64 | 9/10 | 6/10 | 6/7 | 2/7 | 1/4 | 0/4 | 2/6 | 0/6 | 3/3 | 0/3 | 6/6 | 0/6 |
| | (90%) | (60%) | (86%) | (29%) | (25%) | (0%) | (33%) | (0%) | (100%) | (0%) | (100%) | (0%) |
| 128 | 11/12 | 7/12 | 8/10 | 2/10 | 5/10 | 6/10 | 4/10 | 2/10 | 2/4 | 1/4 | 8/9 | 2/9 |
| | (92%) | (58%) | (80%) | (20%) | (50%) | (60%) | (40%) | (20%) | (50%) | (25%) | (89%) | (22%) |
| >=256 | 11/18 | 14/18 | 12/18 | 7/18 | 28/31 | 23/31 | 17/31 | 18/31 | 36/39 | 23/39 | 32/34 | 6/34 |
| | (61%) | (78%) | (67%) | (39%) | (90%) | (74%) | (55%) | (58%) | (92%) | (59%) | (94%) | (18%) |
| **Total** | **38/51** | **30/51** | **28/50** | **14/50** | **35/48** | **30/48** | **25/52** | **21/52** | **41/47** | **25/47** | **46/53** | **8/53** |

| | | | | | | | | | | | | |
|---|---|---|---|---|---|---|---|---|---|---|---|---|
| The table shows as a ratio (and percentage) the 4-fold rise in SBA titer as determined in each assay (from paired samples pre-immunization and 28-day post-immunization) stratified by SBA-BR titer, vaccine and serogroup. | | | | | | | | | | | | |

For serogroup C, there is close agreement in the four-fold rise between SBA-BR versus SBA-H for the conjugate group. Within this vaccine group, there appears to be a trend that at low post-immunization titers, e.g. 1:32 ― 1:128, the SBA-H four-fold rise is lagging behind the four-fold rise in SBA-BR. But, at post-immunization SBA-BR titers >= 1:256, the number of subjects achieving a four-fold rise by SBA-H appears to be greater than the number of subjects achieving a four-fold rise by SBA-BR. This difference in the fold rises comparing the two complement sources is small, and may be due to higher pre-immunization SBA-BR titers that alter the percentage of achieving a four-fold rise by SBA-BR. For the polysaccharide group, the agreement between the four-fold rise between SBA-BR versus SBA-H is not as close as it is for the conjugate group. Also, there is no notable trend that the four-fold rise by SBA-H becomes more sensitive at higher post-immunization SBA-BR titers than is observed for the conjugate group.

For serogroup Y, the agreement between the four-fold rise by SBA-H and SBA-BR is very close for both vaccine groups. There are very few post-immunization SBA-BR titers less than 1:32 for either vaccine group compared to the post-immunization SBA-BR titers for serogroup C. For this reason, the proportion of subjects achieving a four-fold rise in both SBA-BR and SBA-H for serogroup Y occurs at a higher post-immunization SBA-BR titer compared to serogroup C. For serogroup Y, the proportion of four-fold rise increases to >= 50% at a post-immunization SBA-BR titer of 1:128 for the conjugate group, whereas for serogroup C the threshold SBA-BR titer is 1:32 for the conjugate group.

The agreement between four-fold rise by SBA-H and SBA-BR for serogroup W135 is not as close compared to the other two serogroups. As observed for serogroup Y, there are a limited number of subjects with post-immunization SBA-BR titers less than 1:32 for either vaccine group. The agreement between the four-fold rise in SBA titers (BR versus H) occurs at SBA-BR titers >= 1:256. As noted for serogroup C, there is a difference in the proportion of four-fold rises (BR versus H) between the two vaccine groups that is less evident for serogroup Y. The agreement between the four-fold rise in the serogroup W135 SBA titers (BR versus H) is poorest in the polysaccharide group compared to the four-fold rise in SBA titers (BR versus H) by the polysaccharide vaccine for the two other serogroups.

The Serum Bactericidal Assay with baby rabbit complement (SBA-BR) is compared to the corresponding SBA using human complement (SBA-H) for measuring titers in serum samples from 2 to 3 year old subjects vaccinated with either the licensed quadrivalent meningococcal polysaccharide vaccine (Menomune^{®}) or an experimental quadrivalent meningococcal polysaccharide conjugate vaccine (TetraMenD). Human complement sources for serogroups C, Y, and W135 are identified and used to support this comparison in the SBA. The SBA results from this comparative study are analyzed by two approaches. In one approach the SBA-BR and SBA-H data obtained by measuring the pre- and post-immunization titers for both vaccine groups are pooled for analysis. In the second approach, the pre- and post-immunization titers from the two vaccine groups are analyzed separately. One of the goals of this study is to describe the SBA-BR serum titer that best correlates to a negative SBA-H serum titer. A second goal is to determine the titer using baby rabbit complement that best correlated with a positive titer using human complement in the assay as a correlate of protection for serogroup C and to extrapolate to the protective bactericidal titers for serogroups Y and W135. Other laboratories have published results attempting to establish a protective threshold correlate for the SBA-BR for serogroup C. The results of this study will be compared to those published results. Lastly, the four-fold rise in SBA titers measured in pre- and post-immunization sera are compared for the two complement sources.

A correlation of a SBA titer to protection against disease has only been established for serogroup C. The SBA correlate of protection for serogroup C is determined using human complement in the SBA assay. For the other serogroups an assumption will be made that the SBA-H correlate of protection for serogroup C (SBA titer of 1:4) applies for the other serogroups. Defining a SBA-BR titer(s) that correlates to the SBA titer of 1:4 for serogroup C may differ between serogroups. In terms of defining a SBA-BR titer that best correlates to a negative SBA-H serum titer, a SBA-BR titer of <1:8 is compared to SBA-H titer of <1:4 in the pre- and post-immunization sera. The SBA-BR titer of <1:8 is used based in part on the results from the WHO/CDC study for comparing serogroup C SBA titers (BR versus H) and on the recent finding that a SBA-BR titer of < 1:4 is linked to susceptibility to serogroup C disease in a University Outbreak in the United Kingdom (Jones, G.R., et al., 2000. J. Infect. Dis. 181:1172-1175).

Based upon the SBA titers (BR versus H) generated in this study, the false positive rate for serogroup C using a SBA-BR cut off titer of < 1:8 is 30%. Using higher SBA-BR cut off titers improves the false positive rate as follows: at >=1:16 the false positive rate decreases to 26%, at >=1:32 the false positive rate decreases to 24%, at >=1:64, the false positive rate decreases to 22%, at >=1:128 the false positive rate decreases to 18%, at >=1:256 the false positive rate decreases to 14%, and at >=1:512 the false positive rate decreases to 2%. Increasing the SBA-BR cut off titer does serve to improve the accuracy of defining a negative titer that corresponds to a SBA-H titer of < 1:4, however the sensitivity in discriminating between a positive response and a negative response is much lower when using higher SBA-BR cut off titers. The data in this report showed that sensitivity of the SBA-BR is highest (81-84%) at cut off titers of 1:8, 1:16, or 1:32. At SBA-BR titers greater than 1:32, the sensitivity drops below 80%. However, specificity of the assay at cut off titers of 1:8, 1:16, and 1:32 is at a minimum, ranging from 51 to 58%. A balance is made between sensitivity, specificity and false positive rates in selecting a cut off titer that correlates to a negative titer in the human complement assay. The assignment of 1:32 as the cut off titer for the SBA-BR would result in unnecessarily rejecting true positive responders. The results of this study indicate that a SBA-BR titer of >=1:16 may be a more appropriate cut off titer. It is minimally 2-dilutions above the titer deemed protective based upon both the WHO/CDC study analysis (< 1:8) and the U.K. University Outbreak analysis (< 1:4). Identification of the protective cut off titer in the assays for serogroups W135 and Y are derived by assuming that bactericidal antibody protection is analogous with serogroup C disease and the bactericidal titers that correspond to a negative titer in the human complement assay. For serogroup Y, the false positive rate using a SBA-BR cut off titer of < 1:8 is quite high at 85% compared to 30% for serogroup C. However, as with serogroup C, increasing the SBA-BR cut off titer lowers the false positive rate. At a SBA-BR cut off titer of 1:16 the false positive rate decreases to 84%, at 1:32 the false positive rate is 75%, at 1:64 the false positive rate is 61 %, at 1:128 the false positive rate is 38%, at 1:256 the false positive rate is 13%, and at 1:512 the false positive rate is 2%. Even though the false positive rate for serogroup Y starts out much higher compared to serogroup C, at cut off titers of >= 1:128, the false positive rates for the two serogroup assays become quite comparable. Such high cut off titers, hoarer, may overstate the threshold titers for a positive response. Based upon the sensitivity and specificity analysis, sensitivity is maximized at SBA-BR cut off titers of 1:8 to 1:32, where sensitivity ranged from 95 to 98%. However, as with serogroup C, specificity at these SBA-BR titers is correspondingly low, ranging from 11 to 18%. At the next highest SBA-BR titer, 1:64, sensitivity decreases from 95% to 88%, but the specificity sharply increases to 35%. A cut off titer of < 1:64 in the serogroup Y assay appears to best correspond to a negative titer in the human complement assay.

For serogroup W135, the false positive rate at a SBA-BR cut off titer of <1:8 is 33%, which is similar to serogroup C. As noted for both serogroups C and Y at higher SBA-BR cut off titers, the false positive rate decreases to lower levels. At a SBA-BR cut off titer of 1:16 the false positive rate decreases to 32%, at 1:32 the false positive rate decreases to 28%, at 1 :64 the false positive rate decreases to 26%, at 1:128 the false positive rate decreases to 12%, at 1:256 the false positive rate decreases to 4%, and at 1:512 the false positive rate is minimized at 0%. Sensitivity is highest at SBA-BR titers ranging from 1:8 to 1:64 (86% to 81%). Specificity, as expected, is lowest (46% to 52%) at this range of SBA-BR titers. Even though the false positive rates for serogroup W135 start out much lower compared to serogroup Y, the cut off titer that best corresponds to a negative titer in the human complement assay is < 1:64.

Having identified titers for the three serogroups that correspond to negative titers in the human complement assay, SBA-BR titers above these levels are analyzed for a threshold titer for consideration of a positive response. For serogroup C the threshold titer for a positive response is >= 1:16, for serogroup Y the threshold titer for a positive response is >= 1:64, and for serogroup W135 the threshold titer for a positive response is >=1:64. It appears that a threshold SBA-BR titers of >= 1:128 for serogroup C provides good assurance that a protective titer is achieved, relative to either a SBA-H titer of 1:4 or 1:8 and correlates of efficacy SBA-H titer of 1:4 for serogroup C. This threshold titer compares well with the analysis made on the WHO/CDC data set for serogroup C, and to the data set of Santos. As noted in both of these studies, titers >= 1:128 are highly predictive of protection, but SBA-BR titers less than 1:128 may also be protective. For the WHO/CDC and the Santos data set, SBA-BR titers of 1:8, 1:16, 1:32, 1:64 are referred to as the equivocal titers. Since SBA-BR titers less than 1:16 for the data set presented herein for serogroup C are regarded as negative, the equivocal titers for this analysis is 1:16 to 1:64, which is a subset of the other two studies. In all of these analyses, SBA-BR titers are being compared to SBA-H titers (1:4 or 1:8) that correlate to natural protection data that is collected in the 1960's.

More recently, efforts have been made to correlate the United Kingdom efficacy data for the monovalent C conjugates directly to a SBA-BR titer (Miller E, et al., 2002, Vaccine 20:S58-S67). In this analysis both SBA-BR titers of >= 1:8 and >= 1:128 are found to correlate well with the efficacy data that has been collected thus far for 15 to 17 year old subjects vaccinated with one dose of the monovalent C conjugates. However, when Miller and colleagues performed the same analysis for the toddler age group (12 to 30 months of age) they found very good agreement between SBA-BR titers of >= 1:8 with efficacy, but at SBA-BR titers of >=1:128 the agreement is not as close. Miller presented additional data at the 13^{th} International Pathogenic *Neisseria* Conference, September 1-6, 2002 in Oslo, Norway where the predicted efficacy of subjects achieving a SBA-BR titers >=1:64, one-month post vaccination, are outside the 95% confidence interval of the observed efficacy for this age group. These data help support the notion that SBA-BR titers in the equivocal region of 1:8 to 1:64 may lend assurance of protection. Based upon the analysis for this study, SBA-BR titers of 1:16 to 1:64 may likewise lend assurance of protection against serogroup C.

The serogroup Y assay at a SBA-BR titer of 1:64 has a specificity of only 35%, but as the cut off titer is increased to 1:128 and 1:256, specificity increases to 59% and 84%, respectively. A threshold titer of>= 1:256 provides good assurance of a protective titer in the human complement assay of 1:4 and 1:8. However, sensitivity and specificity are better balanced at a threshold titer of 1:128 for serogroup Y. The range of SBA-BR titers of 1:64 to 1:128 for serogroup Y represent the equivocal range of titers compared to the corresponding range of SBA-BR titers of 1:16 to 1:64 for serogroup C that have been correlated to the SBA-H protective titer.

The serogroup W-135 assay at a SBA-BR titer of 1:64, has a specificity of 52%, but as the titer is increased to 1:128 and 1:256, specificity increases to 64 and 77%, respectively. As with serogroup Y, a threshold titer of >= 1:256 provides good assurance of of a protective titer in the human complement assay of 1:4 and 1:8. Like serogroup Y, sensitivity and specificity are better balanced at a threshold titer of 1:128 for serogroup W135. The range of SBA-BR titers of 1:64 to 1:128 for serogroup W135 represent the equivocal range compared to the range of SBA-BR titers of 1:16 to 1:64 for serogroup C that are correlated to the SBA-H protective titer in this study.

The four-fold rise in bactericidal titers are calculated for each serogroup and separately analyzed by vaccine group using both assays. In general, there is an indication that a higher four-fold rise in SBA titer (BR and H) is detected at higher post-immunization SBA-BR titers for all three serogroups. There are differences in the four-fold rises in SBA titers (BR vs. H) both by serogroup and by vaccine group. For serogroup C, four-fold rises in the assay with human complement appeared lower compared to titers in the assay with baby rabbit complement at low post-immunization SBA-BR titers. However at higher post-immunization SBA-BR titers, the four-fold rise in titer appeared higher in the assay with human complement. This pattern seems to suggest that at low post-immunization SBA-BR titers, the assay is less sensitive with human complement than it is with baby rabbit complement, but at high post-immunization SBA-BR titers, the opposite is true, that is, the assay with human complement becomes more sensitive. This pattern is not apparent when assaying samples from the polysaccharide vaccine group. In those samples the four-fold rise in titer appeared lower when using human complement in the assay. Although no explanation for this observation between sample type is apparent, it does suggest that the assay performed with human complement lacks sensitivity with serum samples containing low titers of bactericidal activity.

For serogroup Y, there is good agreement in the four-fold rises in SBA titers in comparing the two complement sources. The four-fold rises in SBA titers (BR and H) are slightly higher for the conjugate group compared to the polysaccharide group, but the difference is not as large as noted for serogroup C.

For serogroup W135, the agreement between four-fold rise in SBA titers using human complement or baby rabbit complement in the assay for either vaccine group is not as good as compared to the results with the other two serogroup assays. The four-fold rise by SBA-BR titer is very good for both vaccine groups, but the proportion of four-fold rise titers by SBA-H is lower compared to the other two serogroups. The four-fold rise by SBA-H in the polysaccharide groups is quite low, and comparatively lower in the four-fold rise in SBA-H titers for the other two serogroups.

Four-fold rise in SBA titer using BR complement has been the benchmark for registration of the meningococcal polysaccharide vaccines. More recently, an effort has been made to link four-fold rise in SBA-BR titers to clinical efficacy from the post-registration surveillance data on the monovalent C conjugates in the United Kingdom (Borrow R, et al., 2001, Infect. Immun. 69:1568-1573). Based on this analysis the efficacy of the monovalent C conjugates in toddlers, ranging in age from 12 to 30 months, has been estimated at 88% (69 to 95%) within 16 months of the first dose. For this age group, the proportion of subjects achieving a four-fold rise in SBA-BR titer ranges from 89 to 100% following one dose of the monovalent C conjugate vaccines.

The SBA-BR provided herein provides bactericidal titer values that are comparable to the values obtained using human complement as the source of complement in the assay for serogroup C. Therefore, these SBA-BR titers are relevant to the original studies that established the surrogate for protective immunity to serogroup C meningococcal disease and support the extrapolation of the clinical results provided herein to protection, and the SBA-BR titers for serogroup C at are comparable to those reported from other laboratories. Performance of the SBA using human complement in the determination of the serogroup specific response to serogroup Y and serogroup W-135 capsular polysaccharides, by analogy to the serogroup C model, support the relevance of the SBA-BR for determining bactericidal titers to serogroups Y and W-135.

### Relevant Paragraphs

1. A polysaccharide-protein conjugate, wherein a conjugate comprises a capsular polysaccharide of *N. meningitidis* serogroup A, C, W-135 or Y, conjugated to one or more a carrier protein(s), and the composition comprises 0.5 to 15 µg/ml of each capsular polysaccharide to a average size of less than 100,000 daltons.
2. The conjugate according to paragraph 1, wherein the capsular polysaccharide is derivatized to average size of 5,000 to 75,000 daltons.
3. The conjugate according to paragraph 2, wherein the capsular polysaccharide is derivatized to average size of 7,000 to 50,000 daltons.
4. The conjugate according to paragraph 3, wherein the capsular polysaccharide is derivatized to average size of 8,000 to 35,000 daltons.
5. The conjugate according to paragraph 4, wherein the capsular polysaccharide is derivatized to average size of 12,000 to 25,000 daltons.
6. The conjugate according to paragraph 5, wherein the capsular polysaccharide is derivatized to average size of 15,000 to 22,000 daltons.
7. The conjugate according to paragraph 1, wherein the average ratio of derivatized polysaccharide to carrier protein is about 1:1 to about 1:20 (w/w).
8. The composition according to paragraph 7, wherein the average ratio of derivatized polysaccharide to carrier protein is about 1:2 to about 1:10 w/w).
9. The composition according to paragraph 8, wherein the average ratio of derivatized polysaccharide to carrier protein is about 1:2 to about 1:6 (w/w).
10. The composition according to paragraph 9, wherein the average ratio of derivatized polysaccharide to carrier protein is about 1 :(4±1) (w/w).
11. The composition according to paragraph 10, wherein the average ratio of derivatized polysaccharide to carrier protein is about 1:(4±0.5) (w/w).
12. The composition according to paragraph 11, wherein the average ratio of derivatized polysaccharide to carrier protein is about 1:(4±0.25) (w/w).
13. The conjugate according to paragraph 1, wherein the carrier protein comprises a bacterial toxin or toxoid, or a bacterial outer membrane protein,
14. The conjugate according to paragraph 1, wherein the carrier protein comprises a diphtheria toxin, diphtheria toxoid, CRM₁₉₇, tetanus toxoid, pertussis toxoid, E. coli LT, E. coli ST, exotoxin A, outer membrane complex c (OMPC), porin, transferrin binding protein, pneumolysis, pneumococcal surface protein A (PspA), pneumococcal adhesin protein (PsaA), ovalbumin, keyhole limpit hemocyanin (KLH), bovine serum albumin (BSA) or purified protein derivative of tuberculin (PPD).
15. The conjugate according to paragraph 14, wherein the carrier protein comprises a diphtheria toxin, diphtheria toxoid, CRM₁₉₇, tetanus toxoid, exotoxin A, or outer membrane complex c (OMPC).
16. The conjugate according to paragraph 15, wherein the carrier protein comprises a diphtheria toxin, diphtheria toxoid, or CRM₁₉₇.
17. The conjugate according to paragraph 16, wherein the carrier protein comprises a diphtheria toxin, or diphtheria toxoid.
18. The conjugate according to paragraph 17, wherein the capsular polysaccharide is derivatized to average size of 8,000 to 35,000 daltons.
19. The conjugate according to paragraph 17, wherein the average ratio of derivatized polysaccharide to carrier protein is about 1:2 to about 1:10 (w/w).
20. The conjugate according to paragraph 19, wherein the average ratio of derivatized polysaccharide to carrier protein is about 1:(4±1) (w/w).
21. A composition comprising a polysaccharide-protein conjugate, wherein a conjugate comprises two or more capsular polysaccharides of *N. meningitidis* of serogroup A, C, W-135 or Y, conjugated to one or more a carrier protein(s), and the composition comprises 0.5 to 15 µg/ml of each capsular polysaccharide to a average size of less than 100,000 daltons.
22. The composition according to paragraph 21, wherein the capsular polysaccharide is derivatized to average size of 5,000 to 75,000 daltons.
23. The composition according to paragraph 22, wherein the capsular polysaccharide is derivatized to average size of 7,000 to 50,000 daltons.
24. The composition according to paragraph 23, wherein the capsular polysaccharide is derivatized to average size of 8,000 to 35,000 daltons.
25. The composition according to paragraph 24, wherein the capsular polysaccharide is derivatized to average size of 12,000 to 25,000 daltons.
26. The composition according to paragraph 25, wherein the capsular polysaccharide is derivatized to average size of 15,000 to 22,000 daltons.
27. The composition according to paragraph 21, wherein the average ratio of each derivatized polysaccharide to carrier protein is about 1:1 to about 1:20 (w/w).
28. The composition according to paragraph 27, wherein the average ratio of each derivatized polysaccharide to carrier protein is about 1:2 to about 1:10 (w/w).
29. The composition according to paragraph 28, wherein the average ratio of each derivatized polysaccharide to carrier protein is about 1:2 to about 1:6 (w/w).
30. The composition according to paragraph 29, wherein the average ratio of each derivatized polysaccharide to carrier protein is about 1:(4±1) (w/w).
31. The composition according to paragraph 30, wherein the average ratio of each derivatized polysaccharide to carrier protein is about 1:(4±0.5) (w/w).
32. The composition according to paragraph 31, wherein the average ratio of each derivatized polysaccharide to carrier protein is about 1:(4±0.25) (w/w).
33. The composition according to paragraph 31, wherein the composition is a liquid.
34. The composition according to paragraph 33, comprising about 0.5 to about 15 ug ofN. meningococcal derivatized polysaccharide to serogroup A, C, W-135 or Y per milliliter of liquid.
35. The composition according to paragraph 33, comprising about 0.5 to about 15 ug ofN. meningococcal derivatized polysaccharide to serogroup W-135 or Y per milliliter of liquid.
36. The composition according to paragraph 31, wherein the carrier protein in diphtheria toxin or toxoid.
37. The composition according to paragraph 36, further comprising an adjuvant.
38. The composition according to paragraph 37, wherein the adjuvant comprises aluminum hydroxide, aluminum phosphate or combination thereof.
39. The composition according to paragraph 31, wherein the composition comprises sodium phosphate, sodium chloride or combination thereof
40. A method of immunizing a human patient against N. meningococcal disease by administration to the human patient a composition according to paragraph 21 to 39.
41. The method according to paragraph 40, wherein the composition comprises an antigen to diphtheria, tetanus, pertussis FHA, pertussis PT, or PRP.
42. The method according to paragraph 40, wherein the composition according to paragraph 21 is a first composition, and a second composition comprising an antigen to diphtheria, tetanus, pertussis FHA, pertussis PT, or PRP is administered to the human patient within six months of administration of the first composition.
43. The method according to paragraph 42, wherein the second composition comprising an antigen to diphtheria, tetanus, pertussis FHA, pertussis PT, or PRP is administered to the human patient within three months of administration of the first composition.
44. The method according to paragraph 43, wherein the second composition comprising an antigen to diphtheria, tetanus, pertussis FHA, pertussis PT, or PRP is administered to the human patient concomitantly with the administration of the first composition.
45. The method according to paragraph 40, wherein the human patient is under the age of 60.
46. The method according to paragraph 45, wherein the human patient is between the ages of 35 and 60.
47. The method according to paragraph 45, wherein the human patient is between the ages of 35 and 60.
48. The method according to paragraph 45, wherein the human patient is between the ages of 18 and 35.
49. The method according to paragraph 45, wherein the human patient is between the ages of 18 and 25.
50. The method according to paragraph 45, wherein the human patient is between the ages of 15 and 18.
51. The method according to paragraph 45 wherein the human patient is between the ages of 10 and 15.
52. The method according to paragraph 45, wherein the human patient is under the age of 11.
53. The method according to paragraph 45, wherein the human patient is between the ages of 2 and 10.
54. The method according to paragraph 45, wherein the human patient under the age of 2.
55. The method according to paragraph 45, wherein the human patient is between the ages of 6 weeks and one year.
56. The composition according to paragraph 21, wherein the capsular polysaccharide is selected from the group consisting of A and W-135;Y and W-135, C and Y, C and W-135; A, C and Y, A, C and W-135, (7) C, Y and W-135, A, Y and W-135 and A, C, Y and W-135
57. A method of inducing an immunological response against meningococcal A in a human patient, wherein the method comprises administering to the human patient a vaccine composition comprising a polysaccharide-protein conjugate, wherein the conjugate comprises a 0.5 to 15 µg/ml of capsular polysaccharide *of N. meningitidis* of serogroup A derivatized to an average size of less than 100,000 daltons.
58. A method of inducing an immunological response against meningococcal C in a human patient, wherein the method comprises administering to the human patient a vaccine composition comprising a polysaccharide-protein conjugate, wherein the conjugate comprises a 0.5 to 15 µg/m1 of capsular polysaccharide *of N. meningitidis* of serogroup C derivatized to an average size of less than 100,000 daltons.
59. A method of inducing an immunological response against meningococcal Y in a human patient, wherein the method comprises administering to the human patient a vaccine composition comprising a polysaccharide-protein conjugate, wherein the conjugate comprises a 0.5 to 15 µg/ml of capsular polysaccharide *of N. meningitidis* of serogroup Y derivatized to an average size of less than 100,000 daltons.
60. A method of inducing an immunological response against meningococcal. W-135 in a human patient, wherein the method comprises administering to the human patient a vaccine composition comprising a polysaccharide-protein conjugate, wherein the conjugate comprises a 0.5 to 15 µg/ml of capsular polysaccharide *of N. meningitidis of* serogroup W-135 derivatized to an average size of less than 100,000 daltons.
61. The method according to any of paragraph 57 to 60, wherein the vaccine composition does not comprise an adjuvant.
62. A method of immunizing a human patient against *N. meningitidis* by administration of a vaccine composition comprising a conjugate according to paragraph 1 to 20, whereby the human patient has a fourfold or-greater increase in serum GMT or IgG titer within 28 days of vaccination compared with pre-administration serum GMT or IgG titer.
63. A method of immunizing a human patient against *N. meningitidis by* administration of a vaccine composition comprising a conjugate according to paragraph 1 to 20, whereby the human patient has a serum SBA-BR titer of 1:32 or higher within 20 to 40 days of vaccination compared with pre-administration SBA-BR titer.
64. A method of immunizing a human patient against *N. meningitidis* by administration of a vaccine composition comprising a conjugate according to paragraph 1 to 20, whereby the human patient has a serum SBA-BR titer of 1:64 or higher within 20 to 40 days of vaccination compared with pre-administration SBA-BR titer.
65. A method of immunizing a human patient against *N. meningitidis* by administration of a vaccine composition comprising a conjugate according to paragraph 1 to 20, whereby the human patient has a serum SBA-BR titer of 1: 128 or higher within 20 to 40 days of vaccination compared with pre-administration SBA-BR titer.

## Claims

1. A polysaccharide-protein conjugate, wherein a conjugate comprises a capsular polysaccharide of *N. meningitidis* serogroup A, C, W-135 or Y, conjugated to one or more a carrier protein(s), and the composition comprises 0.5 to 15 µg/ml of each capsular polysaccharide to a average size of less than 100,000 daltons.

2. The conjugate according to claim 1, wherein the capsular polysaccharide is derivatized to average size of 5,000 to 75,000 daltons.

3. The conjugate according to claim 2, wherein the capsular polysaccharide is derivatized to average size of 15,000 to 22,000 daltons.

4. The conjugate according to claim 1, wherein the average ratio of derivatized polysaccharide to carrier protein is about 1:1 to about 1:20 (w/w).

5. The composition according to claim 4, wherein the average ratio of derivatized polysaccharide to carrier protein is about 1:2 to about 1:6 (w/w).

6. The composition according to claim 5, wherein the average ratio of derivatized polysaccharide to carrier protein is about 1:(40±0.25) (w/w).

7. The conjugate according to claim 1, wherein the carrier protein comprises a bacterial toxin or toxoid, or a bacterial outer membrane protein.

8. The conjugate according to claim 1, wherein the carrier protein comprises a diphtheria toxin, diphtheria toxoid, CRM₁₉₇, tetanus toxoid, pertussis toxoid, E. coli LT, E. coli ST, exotoxin A, outer membrane complex c (OMPC), porin, transferrin binding protein, pneumolysis, pneumococcal surface protein A (PspA), pneumococcal adhesin protein (PsaA), ovalbumin, keyhole limpit hemocyanin (KLH), bovine serum albumin (BSA) or purified protein derivative of tuberculin (PPD).

9. The conjugate according to claim 8, wherein the carrier protein comprises a diphtheria toxin, diphtheria toxoid, or CRM₁₉₇.

10. A composition suitable for use in producing an immunological response against *N. meningitidis* which contains a conjugate as claimed in any of claims 1 to 9 which contains capsular conjugates of serogroups A, C, W-135 and Y of *N. meningitidis.*

11. A composition as claimed in any of claims 1 to 10 for use in inducing an immunological response against *N. meningitidis* serogroups A, C, W-135 and/or Y in a patient.

12. A composition as claimed in claim 11 wherein the patient is under the age of 11, for example between 2 and 10 or under the age of 2.

13. A composition as claimed in claim 11 wherein the patient is between the ages of 6 weeks and 1 year.

14. A composition as claimed in any of claims 1 to 9 or composition for use as claimed in any of claims 10 to 13 which does not comprise an adjuvant.
